# EUROPEAN PATENT APPLICATION

(11) **EP 4 286 009 A2**
(43) Date of publication of application: **06.12.2023**
(21) Application number: 23205199.5
(22) Date of filing: 23.04.2018
(51) Int. Cl.: A61P 35/00

(54) **ONCOLYTIC VACCINIA VIRUS AND CHECKPOINT INHIBITOR COMBINATION THERAPY**

(30) Priority: 21.04.2017 US 201762488623 P; 25.08.2017 US 201762550486 P
(62) Divisional of application: 18723290.5
(71) Applicant: Sillajen, Inc., Busan 46508 (KR)
(72) Inventor: KIM, Chan, Busan 46508 Buk-gu, (KR); JEON, Hongjae, Busan 46508 Buk-gu, (KR); MOON, Eun, Sang, Busan 46508 Buk-gu, (KR); CHI, Sungkuon, Busan 46508 Buk-gu, (KR); CHOI, Jiwon, Sarah, Busan 46508 Buk-gu, (KR); JUNG, Joon-goo, Busan 46508 Buk-gu, (KR); BAE, Jungu, Busan 46508 Buk-gu, (KR)
(74) Representative: Mathys & Squire

(57) **Abstract**

A pharmaceutical combination comprising (i) a replicative oncolytic vaccinia virus and (ii) an immune checkpoint protein inhibitor is provided as well as a kit comprising the pharmaceutical combination and methods for treating and/or preventing cancer.

## Description

### CROSS-REFERENCE TO RELATED APPLICATIONS

This application claims the benefit under 35 U.S.C. § 119(e) of U.S. Provisional Application No. 62/488,623, filed on April 21, 2017 and U.S. Provisional Application No. 62/550,486 filed on August 25, 2017 which are hereby incorporated by reference in their entireties.

### FIELD OF THE INVENTION

The present invention relates generally to virology and medicine In certain aspects, the invention relates to a therapeutic combination comprising a replicative oncolytic vaccinia virus and an immunomodulator.

### BACKGROUND

Normal tissue homeostasis is a highly regulated process of cell proliferation and cell death. An imbalance of either cell proliferation or cell death can develop into a cancerous state. For example, cervical, kidney, lung, pancreatic, colorectal, and brain cancer are just a few examples of the many cancers that can result. In fact, the occurrence of cancer is so high that over 500,000 deaths per year are attributed to cancer in the United States alone.

Replication-selective oncolytic viruses hold promise for the treatment of cancer. These viruses can cause tumor cell death through direct replication-dependent and/or viral gene expression-dependent oncolytic effects. However, immune suppression by tumors and premature clearance of the virus often result in only weak tumor-specific immune responses, limiting the potential of these viruses as a cancer therapeutic.

Similarly, immune checkpoint inhibitors have shown some promise in treating certain cancers, yet only a limited percentage of patients achieve objective clinical response. There remains a need for improved cancer therapies.

### SUMMARY OF THE INVENTION

The present inventors have discovered that concurrent administration of an immune checkpoint inhibitor and an intratumorally administered replicative oncolytic vaccinia virus to a clinically relevant cancer model, in which the agents are administered simultaneously for a first and preferably for multiple consecutive administrations, results in synergistic antitumor effects. Accordingly, in several embodiments, the present application provides a combination therapy for use in the treatment and/or prevention of cancer and/or the establishment of metastases in a mammal comprising concurrently administering to the mammal (i) a replicative oncolytic vaccinia virus and (ii) an immune checkpoint inhibitor, wherein the oncolytic vaccinia virus is intratumorally administered to the mammal. In certain aspects, concurrent administration of the pharmaceutical combination partners to a mammal provides an enhanced and even synergistic anti-tumor immunity compared to either treatment alone.

In some embodiments, the replicative oncolytic vaccinia virus is administered intratumorally, intravenously, intra-arterially, or intraperitoneally. In some embodiments, the replicative oncolytic vaccinia virus is administered intratumorally. In some embodiments, the replicative oncolytic vaccinia virus is administered in an amount effective to induce expression of an immune checkpoint protein in the tumor. In some embodiments, the tumor does not express the immune checkpoint protein or expresses the immune checkpoint protein at a relatively low level prior to administering the replicative oncolytic vaccinia virus. In some embodiments, the immune checkpoint inhibitor is an antibody or fragment thereof that specifically binds to the immune checkpoint protein, preferably a monoclonal antibody, humanized antibody, fully human antibody, fusion protein or combination thereof.

In some embodiments, the immune checkpoint inhibitor of the combination inhibits an immune checkpoint protein selected from the group consisting of cytotoxic T-lymphocyte antigen-4 (CTLA4 or CTLA-4), programmed cell death protein 1 (PD-1), B7-H3, B7-H4, T-cell membrane protein 3 (TIM3), galectin 9 (GAL9), lymphocyte activation gene 3 (LAG3), V-domain immunoglobulin (Ig)-containing suppressor of T-cell activation (VISTA), Killer-Cell Immunoglobulin-Like Receptor (KIR), B and T lymphocyte attenuator (BTLA), T-cell immunoreceptor with Ig and ITIM domains (TIGIT), indoleamine 2,3-dioxygenase (IDO) or a combination thereof. In an additional aspect, the checkpoint inhibitor interacts with a ligand of a checkpoint protein including without limitation, CTLA-4, PD-1, B7-H3, B7-H4, TIM3, GAL9, LAG3, VISTA, KIR, BTLA, TIGIT or a combination thereof. In preferred embodiments, the immune checkpoint protein inhibitor is an antibody (*e*.*g*. monoclonal antibody, chimeric antibody, human antibody or humanized antibody), an antibody fragment, or a fusion protein that specifically binds to an immune checkpoint protein or ligand thereof.

In some preferred embodiments, the immune checkpoint inhibitor of the combination is an antibody or antigen-binding fragment thereof, that specifically binds to (and inhibits) PD-1, PD-L1, PD-L2, TIGIT, TIM3, LAG3, or CTLA4. As a non-limiting example, a method of treating and/or preventing cancer in a mammal is provided comprising concurrently administering to the subject effective amounts of (i) a replicative oncolytic vaccinia virus by intratumoral injection and (ii) a CTLA4 and/or PD-1 inhibitor.

In some embodiments, the immune checkpoint inhibitor is a monoclonal antibody that selectively binds to PD-1 or PD-L1, preferably selected from the group consisting of: BMS-936559, atezolizumab, durvalumab, avelumab, nivolumab, pembrolizumab, and lambrolizumab. In some embodiments, the immune checkpoint inhibitor is a monoclonal antibody that selectively binds to CTLA4, preferably selected from the group consisting of ipilimumab and tremelimumab. In some embodiments, multiple checkpoint inhibitors are concurrently administered to the subject with the oncolytic vaccinia virus. In some embodiments, the subject is concurrently administered: (a) a CTLA4 inhibitor, a PD-1 inhibitor and a replicative oncolytic vaccinia virus; (b) a CTLA4 inhibitor, an IDO inhibitor and a replicative oncolytic vaccinia virus; (c) a PD-1 inhibitor, an IDO inhibitor and a replicative oncolytic vaccinia virus; or (d) a PD-1 inhibitor, a CTLA4 inhibitor, an IDO inhibitor and a replicative oncolytic vaccinia virus; (e) a LAG3 inhibitor, a PD-1 inhibitor and a replicative oncolytic vaccinia virus; or (f) a TIGIT inhibitor, a PD-1 inhibitor and a replicative oncolytic vaccinia virus. In some embodiments, the replicative oncolytic vaccinia virus is a Wyeth Strain, Western Reserve Strain, Lister strain or Copenhagen strain. In some embodiments, the vaccinia virus comprises one or more genetic modifications to increase selectivity of the virus for cancer cells, preferably the virus is engineered to lack functional thymidine kinase and/or to lack functional vaccinia growth factor. In some embodiments, the vaccinia virus comprises a functional 14L and/or F4L gene.

In some embodiments, the vaccinia virus is a Wyeth strain, Western Reserve strain, Lister strain or Copenhagen strain with one or more genetic modifications to increase selectivity of the vaccinia virus for cancer cells such as inactivation of thymidine kinase (TK) gene and/or vaccinia virus growth factor (VGF) gene. In related embodiments, the vaccinia virus is engineered to express a cytokine such as, without limitation, GM-CSF, IL-2, IL-4, IL-5 IL-7, IL-12, IL-15, IL-18, IL-21, IL-24, IFN-γ, and/or TNF-α, preferably selected from IFN-γ, TNF-a, IL-2, GM-CSF and IL-12. In other related embodiments, the replicative oncolytic vaccinia virus is engineered to express a tumor antigen such as, without limitation, BAGE, GAGE-1, GAGE-2, CEA, AIM2, CDK4, BMI1, COX-2 , MUM-1, MUC-1, TRP-1 TRP-2, GP100, EGFRvIII, EZH2, LICAM, Livin, Livinβ, MRP-3, Nestin, OLIG2 , SOX2, human papillomavirus-E6, human papillomavirus-E7, ART1, ART4, SART1, SART2, SART3, B-cyclin, β-catenin, Gli1, Cav-1, cathepsin B, CD74, E-cadherin, EphA2/Eck, Fra-1/Fosl 1, Ganglioside/GD2, GnT-V, β1,6-N, Her2/neu, Ki67, Ku70/80, IL-13Ra2, MAGE-1, MAGE-3, NY-ESO-1, MART-1, PROX1, PSCA, SOX10, SOX11, Survivin, caspase-8, UPAR, CA-125, PSA, p185HER2, CD5, IL-2R, Fap-α, tenascin, melanoma-associated antigen p97, WT-1, regulator of G-protein signaling 5 (RGS5), Surivin (BIRC5=baculoviral inhibitor of apoptosis repeat-containg 5), Insulin-like growth factor-binding protein 3 (IGF-BP3), thymidylate synthetase (TYMS), hypoxia-inducible protein 2, hypoxial inducible lipid droplet associated (HIG2), matrix metallopeptidase 7 (MMP7), prune homolog 2 (PRUNE2), RecQ protein-like (DNA helicase Q1-like) (RECQL), leptin receptor (LEPR), ERBB receptor feedback inhibitor 1 (ERRFI1), lysosomal protein transmembrane 4 alpha (LAPTM4A); RAB1B, RAS oncogene family (RAB1B), CD24, homo sapiens thymosin beta 4, X-linked (TMSB4X), homo sapiens S100 calcium binding protein A6 (S100A6), homo sapiens adenosine A2 receptor (ADORA2B), chromosome 16 open reading frame 61 (C16orf61), ROD1 regulator of differentiation 1 (ROD1), NAD-dependent deacetylase sirtuin-2 (SIR2L), tubulin alpha 1c (TUBA1C), ATPase inhibitory factor 1 (ATPIF1), stromal antigen 2 (STAG2), nuclear casein kinase, and cyclin-dependent substrate 1 (NUCKS1). In some embodiments, the tumor antigen is a renal cell carcinoma tumor antigen. In some embodiments, the renal cell carcinoma tumor antigen is selected from the group consisting of regulator of G-protein signaling 5 (RGS5), Surivin (BIRC5=baculoviral inhibitor of apoptosis repeat-containg 5), Insulin-like growth factor-binding protein 3 (IGF-BP3), thymidylate synthetase (TYMS), hypoxia-inducible protein 2, hypoxial inducible lipid droplet associated (HIG2), matrix metallopeptidase 7 (MMP7), prune homolog 2 (PRUNE2), RecQ protein-like (DNA helicase Q1-like) (RECQL), leptin receptor (LEPR), ERBB receptor feedback inhibitor 1 (ERRFI1), lysosomal protein transmembrane 4 alpha (LAPTM4A); RAB1B, RAS oncogene family (RAB1B), CD24, homo sapiens thymosin beta 4, X-linked (TMSB4X), homo sapiens S100 calcium binding protein A6 (S100A6), homo sapiens adenosine A2 receptor (ADORA2B), chromosome 16 open reading frame 61 (C16orf61), ROD1 regulator of differentiation 1 (ROD1), NAD-dependent deacetylase sirtuin-2 (SIR2L), tubulin alpha 1c (TUBA1C), ATPase inhibitory factor 1 (ATPIF1), stromal antigen 2 (STAG2), and nuclear casein kinase and cyclin-dependent substrate 1 (NUCKS1).

In some embodiments, the the vaccinia virus is administered in an amount from about 10⁷ to about 10¹¹ pfu, preferably about 10⁸-10¹⁰ pfu, more preferably about 10⁹-10¹⁰ pfu. In some embodiments, the the checkpoint inhibitor is administered in an amount from about 2 mg/kg to 15 mg/kg.

In some embodiments, the pharmaceutical combination is administered to a mammal to treat and/or prevent cancer in a mammal. In some embodiments, the cancer is a solid tumor type cancer. In some embodiments, the cancer is selected from the group consisting of selected from the group consisting of melanoma, hepatocellular carcinoma, renal cell carcinoma, bladder cancer, head and neck cancer, pancreatic cancer, breast cancer, ovarian cancer, prostate cancer, mesothelioma, gastrointestinal cancer, leukemia, lung cancer (including non-small cell lung cancer), stomach cancer, esophageal cancer, mesothelioma, colorectal cancer, sarcoma, or thyroid cancer. In other preferred embodiments, the pharmaceutical combination is administered to a mammal to treat a metastasis. In some embodiments, the subject has renal cell carcinoma.

In preferred embodiments, the mammal to be treated with the pharmaceutical combination is a human subject. In a related aspect, the subject in need of treatment is a human with a cancer that is refractory (or resistant) to treatment with one or more chemotherapeutic agents and/or refractory to treatment with one or more antibodies. In specific embodiments, the human has a cancer (*e*.*g*. colorectal cancer) that is refractory (or resistant) to a treatment comprising an immune checkpoint inhibitor and optionally is also refractory to treatment with one or more chemotherapeutic agents. In other embodiments, the human in need of treatment is a human identified as a candidate for therapy with one or more immune checkpoint inhibitors.

In some embodiments, the subject has failed at least one previous chemotherapy or immunotherapy treatment. In some embodiments, the subject has a cancer that is refractory to an immune checkpoint inhibitor therapy, preferably the cancer is resistant to treatment with anti-PD-1 antibodies and/or anti-CTLA-4 antibodies. In some embodiments, the subject is identified as a candidate for an immune checkpoint inhibitor therapy. In some embodiments, the method comprises administering to the subject an additional therapy selected from chemotherapy (alkylating agents, nucleoside analogs, cytoskeleton modifiers, cytostatic agents) and radiotherapy. In some embodiments, the method comprisesadministering to the subject an additional oncolytic virus therapy (*e*.*g*. rhabdovirus, Semliki Forest Virus). In some embodiments, the subject is a human. In some embodiments, a first dose of the replicative oncolytic vaccinia virus and a first dose of the immune checkpoint inhibitor are simultaneously administered to the subject followed by at least one subsequent consecutive simultaneous administration of the virus and checkpoint inhibitor to the subject. In some embodiments, the method comprises at least a first, second and third consecutive simultaneous administration of the replicative oncolytic vaccinia virus and checkpoint inhibitor to the subject. In some embodiments, the method comprises at least a first, second, third and fourth consecutive simultaneous administration of the replicative oncolytic vaccinia virus and checkpoint inhibitor to the subject. In some embodiments, simultaneous administration of the first dose of the replicative oncolytic vaccinia virus and the first dose of the immune checkpoint inhibitor and at least one subsequent consecutive simultaneous administration of the virus and checkpoint inhibitor to the subject is followed by administration of at least one dose of checkpoint inhibitor alone to the subject. In some embodiments, the method comprises an interval of 1-3 weeks between consecutive simultaneous administration of the agents, preferably comprising an interval of about one week, about two weeks or about 3 weeks.

The present application demonstrates that intratumoral administration of replicative oncolytic vaccinia virus (i) attracts host immune cells *(e.g.* tumor infiltrating T-cells) to the tumor and (ii) induces the expression of several checkpoint proteins, including PD-1, PD-L1, CTLA-4, LAG3, TIM3, and TIGIT, in tumor cells, thereby sensitizing the tumor cells to concurrent treatment with inhibitors of the checkpoint protein(s).

Thus, in some aspects, the expression level of one or more of these checkpoint proteins is used as a biomarker to select human cancer patients for treatment with the combination therapy herein described based on their expression level(s). In some embodiments, the expression can be measured using any assay for measuring protein levels. In some embodiments, the protein expression can be measured using an assay such as a FACS or Nanotring assay.

In related embodiments, the human in need of treatment is a human with a tumor that does not express a checkpoint protein *(e.g.* a checkpoint inhibitor refractory subject) or expresses a checkpoint protein at a relatively low level in which case the oncolytic vaccinia virus component of the combination therapy is administered in an amount effective to sensitize the tumor to the immune checkpoint inhibitor of the combination by inducing expression of the checkpoint protein (*e*.*g*. PD-L1). In some embodiments, the human may have a tumor that does not express PD-1, PD-L1, CTLA-4, LAG3, TIM3, and/or TIGIT or expresses one or more of these checkpoint proteins at a relatively low level and the oncolytic vaccinia virus is administered in an amount effective to sensitize the tumor to a PD-1, PD-L1, CTLA-4, LAG3, TIM3, and/or TIGIT inhibitor. In other related embodiments, the level of a checkpoint protein is measured in a tumor prior to administration of the oncolytic vaccinia virus and checkpoint inhibitor combination therapy and the combination therapy is administered to a subject if it is determined that the checkpoint protein is not expressed or is expressed at a relatively low level in the tumor. In other related embodiments, a method for sensitizing a tumor to a checkpoint inhibitor is provided comprising administering to a human with a tumor an amount of an oncolytic vaccinia virus effective to induce expression of the checkpoint protein in the tumor and concurrently administering to the human the checkpoint inhibitor. In some aspects, a tumor that does not express a checkpoint protein or expresses a checkpoint protein at a relatively low level means that less than 50%, less than 25%, less than 15%, less than 10%, less than 5%, less than 1% or less than 0.5% of tumor cells stain positive for the checkpoint protein as evaluated by immunohistochemistry (IHC) of a tumor sample. See *e.g.* Ilie et al., Virchows Arch, 468(5):511-525 (2016). In some aspects, the human has non-small cell lung cancer, gastric cancer, renal cell carcinoma, pancreatic cancer, or colorectal cancer.

In yet other related embodiments, the human in need of treatment is a human with a tumor that is immunologically "cold", by which it is meant that the tumor is essentially or relatively free of immune cells in the tumor microenvironment. Treatment with an oncolytic vaccinia virus attracts immune cells (*e.g.* T-cells) into the tumor and synergizes with concurrently administered checkpoint inhibitors to treat the tumor. A "cold" tumor may be identified by methods known in the art including, but not limited to, single stain or multiplex immunohistochemistry (IHC) for immune markers such as CD3 and CD8 at the tumor center and invasive margin, flow cytometry for phenotyping, genomic analysis of tumor tissue, RNA profiling of tumor tissue, and/or cytokine profiling in serum.

The oncolytic vaccinia virus and the immune checkpoint inhibitor of the combination are administered concurrently (*e*.*g*., simultaneously) and may be administered as part of the same formulation or in different formulations. By simultaneous (or concurrent) administration, it is meant that a first dose of each of the combination partners is administered at or about the same time (within 24 hours of each other, preferably within 12, 11, 10, 9, 8, 7, 6, 5, 4, 3, 2 hours or within 1 hour of each other) and preferably at least one subsequent dose of each of the combination partners is administered at or about the same time. Thus, in one aspect, combination therapy as described herein comprises a first dose of the replicative oncolytic vaccinia virus administered simultaneously with a first dose of the checkpoint inhibitor (*e*.*g*., treatment of a subject with the combination therapy entails at least a first administration wherein the oncolytic vaccinia virus and checkpoint inhibitor are simultaneously administered to the subject) and preferably further comprises at least one, two, three, four or more additional consecutive simultaneous administrations of the oncolytic vaccinia virus and checkpoint inhibitor. Thus, a concurrent treatment regimen with the pharmaceutical combination may comprise at least two, at least three, at least four, at least five, at least six, at least seven, or more consecutive simultaneously administered doses of the agents. In preferred embodiments, the interval between consecutive simultaneously administered doses of the oncolytic vaccinia virus and checkpoint inhibitor ranges from about 1 day to about 3 weeks or any interval there between such as 1 day, 2 days, 3 days, 4 days, 5 days, 6 days, 7 days, 8 days, 9 days, 10 days, 11 days, 12 days, 13 days, 14 days, 15 days, 16 days, 17 days, 18 days, 19 days, 20 days, or 21 days. In some preferred embodiments, the interval between consecutive simultaneously administered doses of the oncolytic vaccinia virus and checkpoint inhibitor is about 1 week or about 2 weeks. Following the at least one initial simultaneously administered doses of the oncolytic virus and immune checkpoint inhibitor, one or more doses of the checkpoint inhibitor alone may be administered to the subject.

In yet other aspects, the present invention provides a commercial package comprising as active agents a combination of an oncolytic vaccinia virus as herein described and an immune checkpoint inhibitor, together with instructions for simultaneous use in the treatment and/or prevention of cancer as herein described. In a preferred aspect, the commercial package comprises as active agents a combination of a Western Reserve, Copenhagen, Wyeth or Lister strain vaccinia virus and a PD-1, PD-L1, TIGIT or CTLA4 inhibitor.

In some embodiments, the present invention provides a method of treating a tumor in a human comprising concurrently administering to the human a combination comprising (a) a replicative oncolytic vaccinia virus and (b) an inhibitor of the immune checkpoint protein. In some embodiments, the replicative oncolytic virus is administered intratumorally. In some embodiments, the replicative oncolytic virus is administered via intravenous administration. In some embodiments, the replicative oncolytic virus is administered via intra-arterial administration. In some embodiments, the replicative oncolytic virus is administered via intraperitoneal administration. In some embodiments, the replicative oncolytic virus is only delivered via intratumoral administration. In some embodiments, the replicative oncolytic virus is administered intratumorally and the checkpoint inhibitor is administered systemically. In some embodiments, the replicative oncolytic virus is administered intravenously and the checkpoint inhibitor is administered systemically. In some embodiments, the replicative oncolytic virus is administered intraperitoneally and the checkpoint inhibitor is administered systemically. In some embodiments, the replicative oncolytic virus is administered intra-arterially and the checkpoint inhibitor is administered systemically. In some embodiments, the replicative oncolytic vaccinia virus is administered in an amount effective to induce expression of an immune checkpoint protein in the tumor. In some embodiments of the method of treatment, the immune checkpoint protein is selected from PD-1, PD-L1, CTLA-4, LAG3, TIM3, and TIGIT. In some embodiments, the present invention provides a method of treating a tumor in a human comprising concurrently administering to the human a combination comprising (a) a replicative oncolytic vaccinia virus in an amount effective to induce expression of an immune checkpoint protein in the tumor and (b) an inhibitor of the immune checkpoint protein. In some embodiments, the replicative oncolytic vaccinia virus is administered intratumorally. In some embodiments, the replicative oncolytic vaccinia virus is administered IV. In some embodiments of the method of treatment, the immune checkpoint protein is selected from PD-1, PD-L1, CTLA-4, LAG3, TIM3, and TIGIT. In some embodiments of the method of treatment, the immune checkpoint protein is CTLA-4. In some embodiments of the method of treatment, the immune checkpoint protein is PD-L1. In some embodiments of the method of treatment, the immune checkpoint protein is LAG3. In some embodiments of the method of treatment, the immune checkpoint protein is TIGIT. In some embodiments of the method of treatment, the immune checkpoint protein is PD-1. In some embodiments of the method of treatment, the immune checkpoint protein is TIM3. In some embodiments of the method of treatment, the tumor is a solid cancer. In some embodiments of the method of treatment, the tumor is a colorectal cancer. In some embodiments of the method of treatment, the tumor is a renal cell carcinoma.

In some embodiments of the method of dual combination treatment, the inhibitor of the immune checkpoint protein is a monoclonal antibody that selectively binds to PD-1 or PD-L1. In some embodiments, the monoclonal antibody that selectively binds to PD-1 or PD-L1 is selected from the group consisting of BMS-936559, atezolizumab, durvalumab, avelumab, nivolumab, pembrolizumab, and lambrolizumab.

In some embodiments of the method of dual combination treatment, the inhibitor of the immune checkpoint protein is a monoclonal antibody that selectively binds to CTLA-4. In some embodiments, monoclonal antibody that selectively binds to CTLA-4 is selected from the group consisting of ipilimumab and tremelimumab.

In some embodiments of the method of dual combination treatment, the tumor does not express the immune checkpoint protein or expresses the immune checkpoint protein at a relatively low level prior to administering the replicative oncolytic vaccinia virus.

In some embodiments of the method of dual combination treatment, the method comprises a step of measuring the expression level of the immune checkpoint protein in the tumor prior to administering the combination.

In some embodiments, the present invention provides a method of treating a tumor in a human comprising concurrently administering to the human a combination comprising (a) a replicative oncolytic vaccinia virus, (b) an inhibitor of PD-1 and/or PD-L1, and (c) an inhibitor of the immune checkpoint protein. In some embodiments, the replicative oncolytic vaccinia virus is administered in an amount effective to induce expression of an immune checkpoint protein. In some embodiments, the replicative oncolytic virus is administered intratumorally. In some embodiments, the replicative oncolytic virus is administered via intravenous administration. In some embodiments, the replicative oncolytic virus is administered via intra-arterial administration. In some embodiments, the replicative oncolytic virus is administered via intraperitoneal administration. In some embodiments, the replicative oncolytic virus is only delivered via intratumoral administration. In some embodiments, the replicative oncolytic virus is administered intratumorally and the checkpoint inhibitor is administered systemically. In some embodiments, the replicative oncolytic virus is administered intravenously and the checkpoint inhibitor is administered systemically. In some embodiments, the replicative oncolytic virus is administered intraperitoneally and the checkpoint inhibitor is administered systemically. In some embodiments, the replicative oncolytic virus is administered intra-arterially and the checkpoint inhibitor is administered systemically. In some embodiments, the present invention provides a method of treating a tumor in a human comprising concurrently administering to the human a combination comprising (a) a replicative oncolytic vaccinia virus in an amount effective to induce expression of an immune checkpoint protein in the tumor, (b) an inhibitor of PD-1 and/or PD-L1, and (c) an inhibitor of the immune checkpoint protein, wherein the replicative oncolytic vaccinia virus is administered intratumorally. In some embodiments of the method of treatment, the immune checkpoint protein is selected from CTLA-4, LAG3, TIM3, and TIGIT. In some embodiments of the method of treatment, the immune checkpoint protein is CTLA-4. In some embodiments of the method of treatment, the immune checkpoint protein is LAG3. In some embodiments of the method of treatment, the immune checkpoint protein is TIGIT. In some embodiments of the method of treatment, the immune checkpoint protein is TIM3. In some embodiments of the method of treatment, the tumor is a solid cancer. In some embodiments of the method of treatment, the tumor is a colorectal cancer. In some embodiments of the method of treatment, the tumor is a renal cell carcinoma.

In some embodiments of the method of triple combination treatment, the inhibitor of the immune checkpoint protein is a monoclonal antibody that selectively binds to PD-1 or PD-L1. In some embodiments, the monoclonal antibody that selectively binds to PD-1 or PD-L1 is selected from the group consisting of BMS-936559, atezolizumab, durvalumab, avelumab, nivolumab, pembrolizumab, and lambrolizumab.

In some embodiments of the method of triple combination treatment, the inhibitor of the immune checkpoint protein is a monoclonal antibody that selectively binds to CTLA-4. In some embodiments, the monoclonal antibody that selectively binds to CTLA-4 is selected from the group consisting of ipilimumab and tremelimumab.

In some embodiments of the method of triple combination treatment, the tumor does not express the immune checkpoint protein or expresses the immune checkpoint protein at a relatively low level prior to administering the replicative oncolytic vaccinia virus.

In some embodiments of the method of triple combination treatment, the method comprises a step of measuring the expression level of the checkpoint protein in the tumor prior to administering the combination.

Other embodiments of the invention are discussed throughout this application. Any embodiment discussed with respect to one aspect of the invention applies to other aspects of the invention as well and vice versa. The embodiments in the Example section are understood to be embodiments of the invention that are applicable to all aspects of the invention.

### BRIEF DESCRIPTION OF THE DRAWINGS

The following drawings form part of the present specification and are included to further demonstrate certain aspects of the present invention. The invention may be better understood by reference to one or more of these drawings in combination with the detailed description of specific embodiments presented herein.

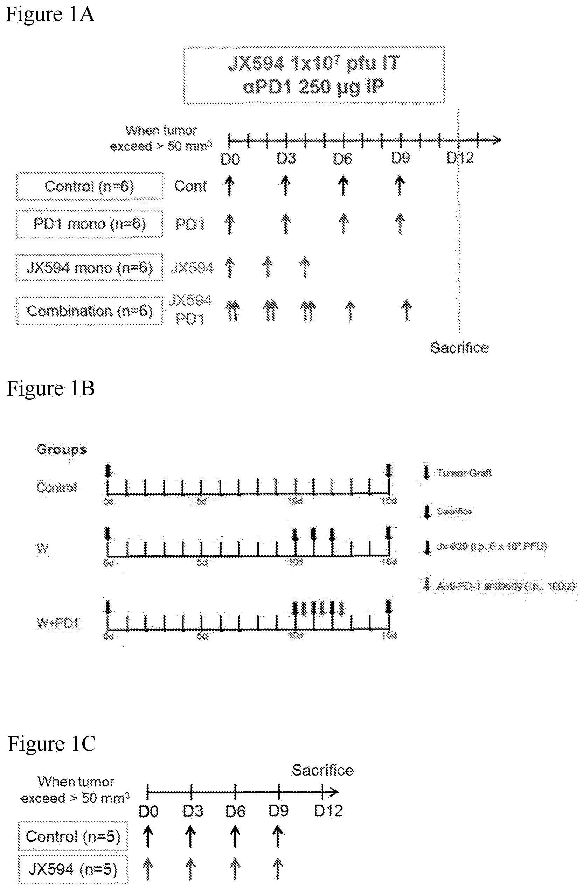

Fig. 1A-1C. Fig. 1A: Chart depicting a concurrent combination treatment regimen with intratumoral (IT) injection of mJX-594 and intraperitoneally administered anti-PD-1 checkpoint inhibitor antibody. 8 week old BALB/c immune competent mice were injected with 5 × 10⁵ Renca (kidney cancer) cells. Once tumor size reached ≥ 50 mm³, the mice were treated (Day 0) with PBS (control, days 0, 3, 6 and 9), anti-PD-1 antibody alone (Days 0, 3, 6, and 9), mJX-594 alone (Days 0, 2 and 4) or anti-PD-1 and mJX-594 delivered concurrently (simultaneous administration of the agents on Days 0, 2 and 4 followed by administration of anti-PD-1 alone on Days 6 and mJX-594 was administered intratumorally (IT) at 1×10⁷ pfu and anti-PD-1 at 10 mg/kg intraperitoneally (IP). Fig. 1B: Eight-week-old female BALB/c mice were injected with RENCA cells (2×10⁶ cells) in 100µl of PBS into the subcapsule of the left kidney. On day 10 post-implantation, mice harboring Renca tumors (50 mm³-100 mm³ as visualized with the IVIS^{®} Spectrum in vivo imaging system) were treated intraperitoneally (i.p) with (i) PBS (control) (ii) vaccinia virus (JX-929) monotherapy (6×10⁷ PFU on days 10, 11 and 12 post-implantation for a total of 3 doses) (iii) anti-PD1 monotherapy (BioXcell, West Lebanon, NH, 100µl) (days 10, 11 and 12 post-implantation for a total of 3 doses) or (iv) concurrent JX929 + anti-PD1 treatment (each administered on days 10, 11 and 12 post-implantation, with JX-929 administered on the morning and ICI in the afternoon of the same day with a 9-hour interval) according to the regimen shown. Fig. 1C: Balb/c mice carrying Renca tumors exceeding 50 mm³ were administered four intratumoral doses of mJX594 (1 × 10⁷ on each of days 0, 3, 6, and 9) or PBS control according to the treatment regimen shown.

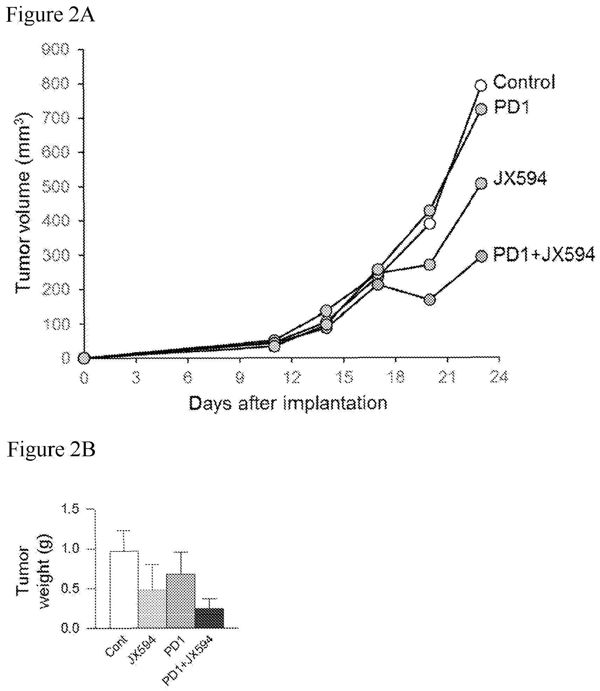

Figs. 2A-2B. Fig. 2A: Graph depicting the effects of the treatment regimens described in Figure 1A on tumor volume. Concurrent combination treatment (PD1+mJX594) significantly suppressed tumor growth (following Day 18 after implantation) compared to all other treatment groups. Fig. 2B: photo and graph depicting tumor weight in each treatment group described in Figure 1A. Concurrent combination treatment (PD1+mJX594) synergized to markedly reduce tumor volume relative to either monotherapy.

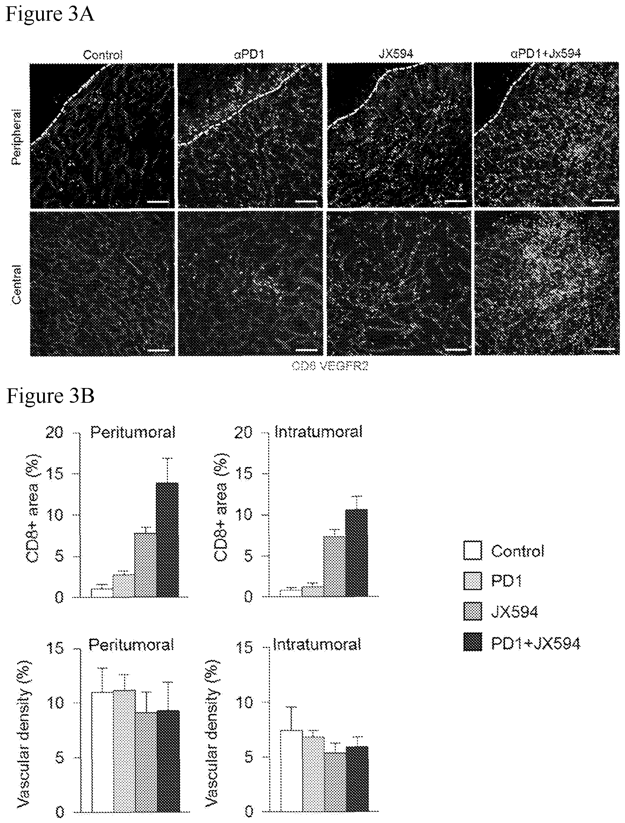

Figs. 3A-3B. Concurrent combination treatment with IT mJX-594 and anti-PD-1 markedly increases intratumoral T-cell infiltration compared to control and single treatment with either agent. Mice were treated according to the administration regimens depicted at Figure 1. Figure 3A: Images demonstrating marked increase in CD8 T-cell infiltration in both peritumoral and intratumoral regions in concurrent combination treatment group compared to control and monotherapy groups. Figure 3B: Graphs demonstrating marked increase in peritumoral and intratumoral CD8 T-cell infiltration in concurrent combination group compared to control and either monotherapy.

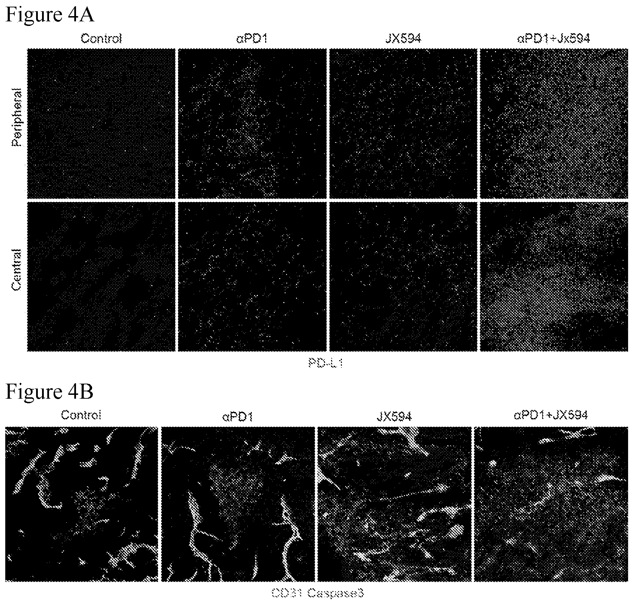

Figs. 4A-4B. Concurrent combination treatment with IT mJX-594 and anti-PD-1 upregulates intratumoral PD-L1 expression. Mice were treated according to the administration regimens depicted at Figure 1. Figure 4A: Images demonstrating a marked increase in PD-L1 expression level in both peripheral and central tumor regions in concurrent combination treatment group compared to control and either monotherapy (PD-L1 staining). Figure 4B: Images demonstrating a marked increase in intratumoral apoptosis in concurrent combination treatment group compared to control and either monotherapy group.

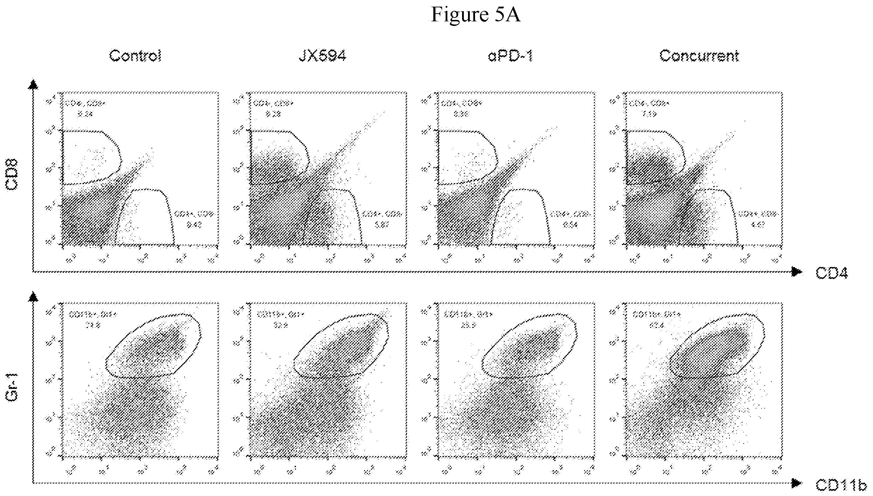

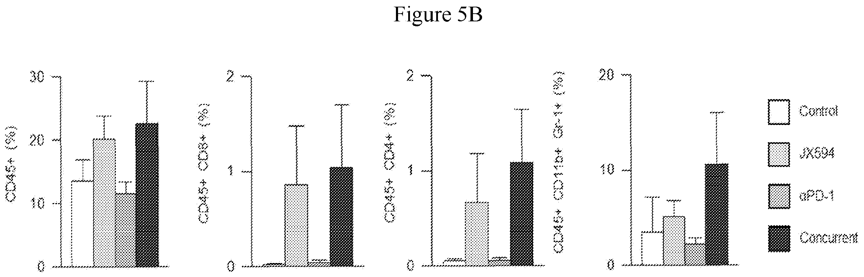

Figs. 5A-5B. CD8 T-cells and CD1 1b+Gr1+ Myeloid-derived suppressor cells (MDSCs) are increased in the concurrent combination therapy group compared to control. Fig. 5A: flow cytometric graphs showing positivity for CD8 and Gr-1 in tumors from each treatment group, with a significant increase in CD8+ T-cells demonstrated for tumors from the concurrent combination group compared to either monotherapy. An increase in MDSCs is also shown relative to either monotherapy. Fig. 5B: Bar graphs depicting the results of flow cytometry.

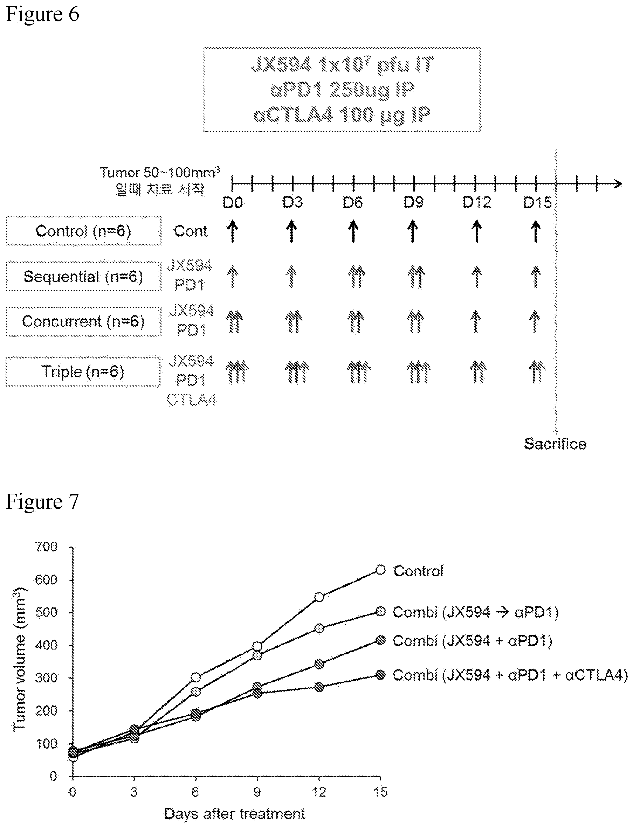

Fig. 6. Chart depicting combination treatment regimen with IT injection of mJX-594 and anti-PD1 (+/- anti-CTLA4) checkpoint inhibitor antibody delivered intraperitoneally. 5 × 10⁵ Renca cells were injected subcutaneously into the right flank of 8 week old BALB/c mice. Treatment was initiated (Day 0) when tumor size reached 50-100 mm³. On Day 0, the mice (carrying Renca tumors) were treated with PBS (control), combination of mJX-594+anti-PD1 delivered sequentially, combination of mJX-594+anti-PD-1 delivered concurrently and triple combination of mJX-594+anti-PD-1+anti-CTLA4 delivered concurrently. mJX-594 was administered at 1×10⁷ pfu IT, anti-PD1 at 10mg/kg IP and anti-CTLA4 at 4 mg/kg IP.
Fig. 7. Graph depicting the effects of the treatment regimens described in Figure 6 on tumor volume. Concurrent combination treatment with αPD1+mJX594 and αPD1+mJX594+αCTLA4 significantly suppressed tumor growth from Day 6 (after treatment) compared to all other treatment groups and both concurrent combination treatment groups markedly delayed tumor growth compared to control and sequential combination treatment group (mJX594〉̶αPD1), in which tumor regression was observed from the 12^{th} day.

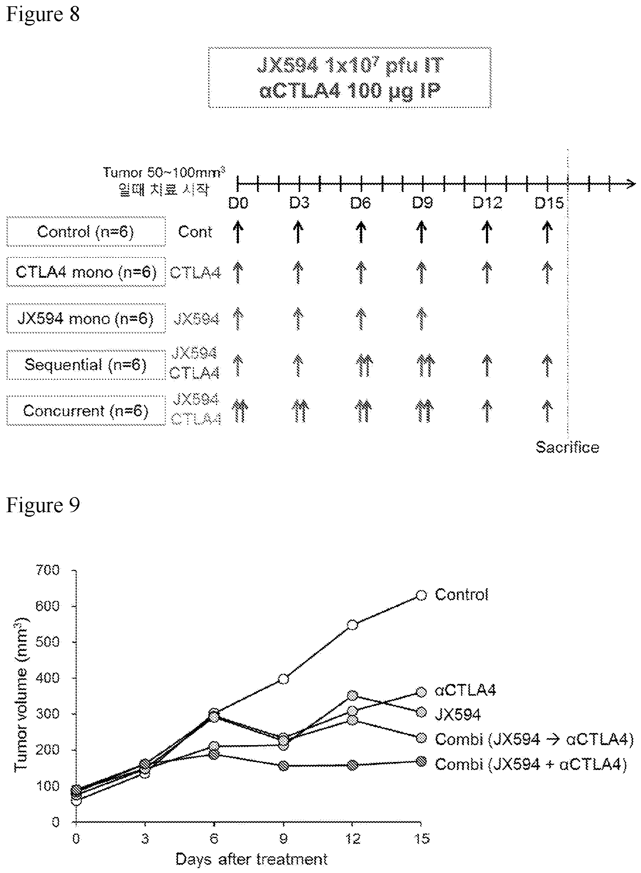

Fig. 8. Chart depicting combination treatment regimen with IT injection of mJX-594 and anti-CTLA4 checkpoint inhibitor antibody delivered intraperitoneally. 5 × 10⁵ Renca cells were injected subcutaneously into the right flank of 8 week old BALB/c mice. Treatment was initiated (Day 0) when tumor size reached 50-100 mm³. On Day 0, the mice (carrying Renca tumors) were treated with PBS (control), anti-CTLA4 alone, mJX-594 alone, combination of mJX-594+CTLA4 delivered sequentially and combination of mJX-594+CTLA4 delivered concurrently. mJX-594 was administered at 1×10⁷ pfu IT and anti-CTLA4 at 4 mg/kg.
Fig. 9. Graph depicting the effects of the treatment regimens described in Figure 8 on tumor volume. Concurrent combination treatment with mJX594+αCTLA4 markedly delayed tumor growth compared to sequential combination treatment with mJX594+aCTLA4 and either monotherapy.

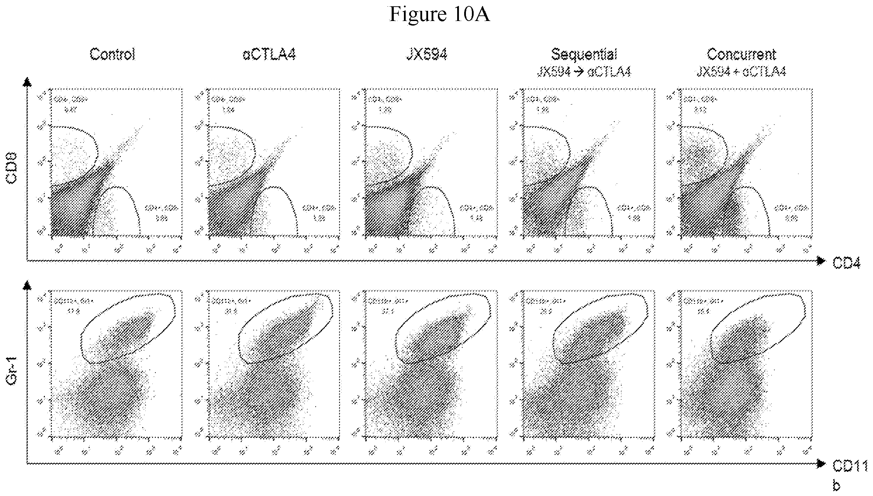

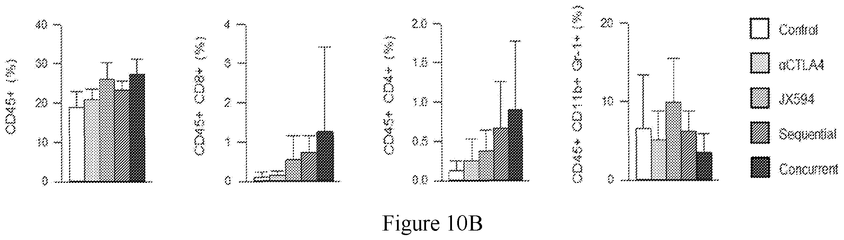

Figs. 10A-10B. Concurrent combination of IT mJX594 and anti-CTLA4 markedly increases CD8+ T-cell tumor infiltration and reduces MDSC level compared to sequential combination and either monotherapy. Fig. 10A: flow cytometric graphs showing positivity for CD8 and Gr-1 in tumors from each treatment group, with a significant increase in CD8+ T-cells and a significant decrease in MDSCs demonstrated for tumors from the concurrent combination group compared to sequential treatment group and either monotherapy. Fig. 10B: Bar graphs depicting the results of flow cytometry.

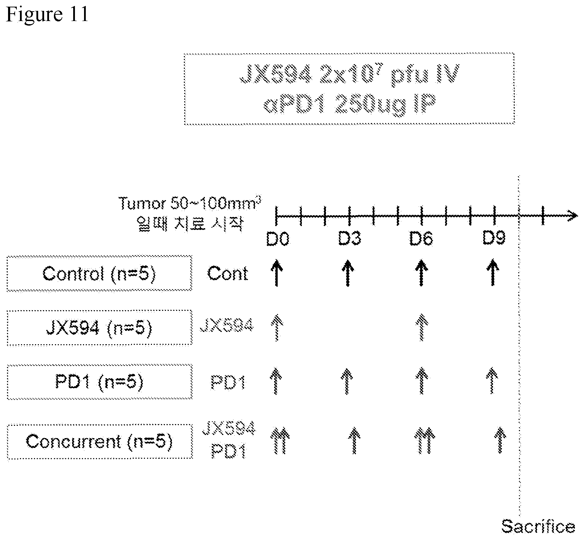

Fig. 11. Chart depicting combination treatment regimen with intravenous (IV) injection of mJX-594 and anti-PD1 checkpoint inhibitor antibody delivered intraperitoneally. 5 × 10⁵ Renca cells were injected subcutaneously into the right flank of 8 week old BALB/c mice. Treatment was initiated (Day 0) when tumor size reached 50-100 mm³. On Day 0, the mice (carrying Renca tumors) were treated with PBS (control), anti-PD1 alone, mJX-594 alone, or mJX-594+anti-PD1 delivered concurrently. mJX-594 was administered at 2 × 10⁷ pfu IV, anti-PD1 at 10 mg/kg IP.

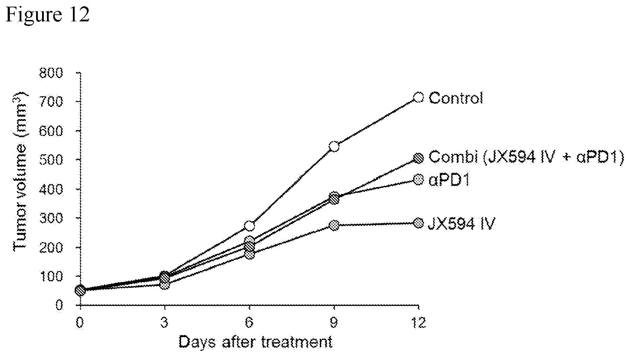

Fig. 12. Graph depicting the effects of the treatment regimens described in Figure 11 on tumor volume. Concurrent combination treatment with mJX594 IV+ αPD1 was inferior to treatment with mJX594 alone and no better than treatment with αPD1 alone.

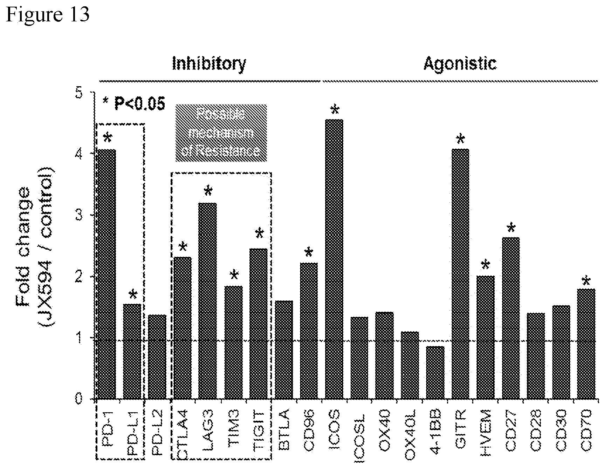

Fig. 13. A chart demonstrating fold-changes (relative to pre-treatment levels) of immune checkpoint proteins in Renca tumor-carrying mice treated intratumorally with four 1 × 10⁷ pfu doses of mJX594mJX594 (Wyeth vaccinia virus engineered to contain a disruption of the viral thymidine kinase gene and insertion of murine GM-CSF) administered every three days.

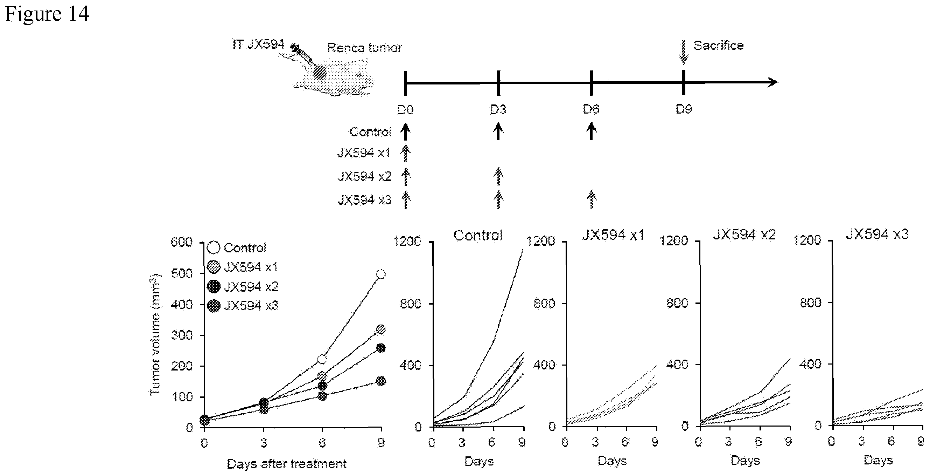

Fig. 14. Provides data regarding the number of mJX594 injections and tumor growth inhibition. To find out optimal immunotherapy with mJX594, various number of doses in Renca kidney cancer were tested. Tumor growth was decreased dependent upon the increasing number of mJX594 doses.

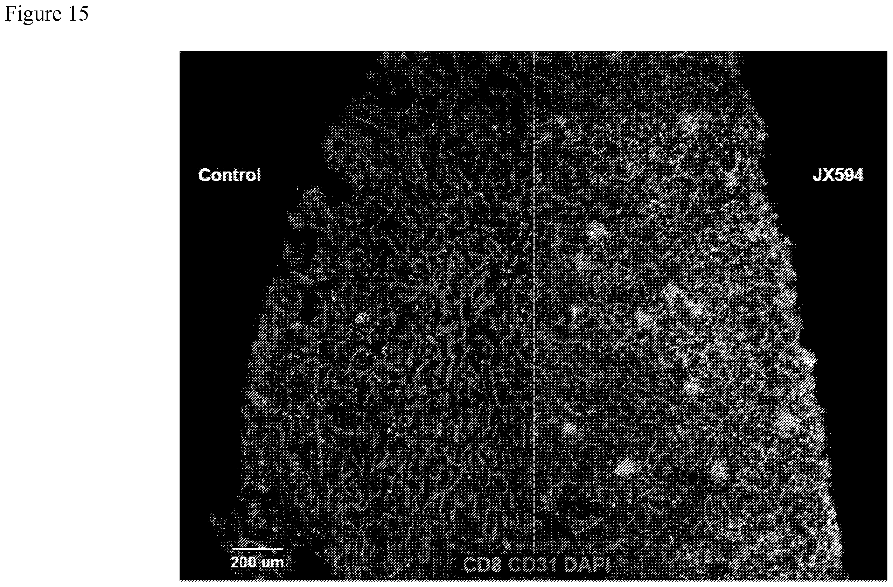

Fig. 15. Images showing the intratumoral recruitment of CD8+ T-cells after mJX594 treatment.

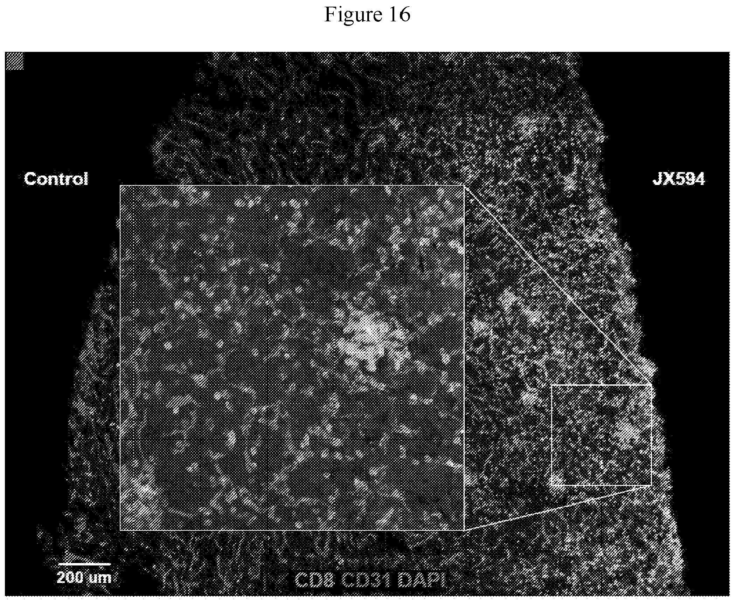

Fig. 16. Images showing the intratumoral recruitment of CD8+ T-cells after mJX594 treatment. In mJX594-treated tumors, aggregates of CD8+ lymphoid cells were observed, which are similar to lymphoid follicles.

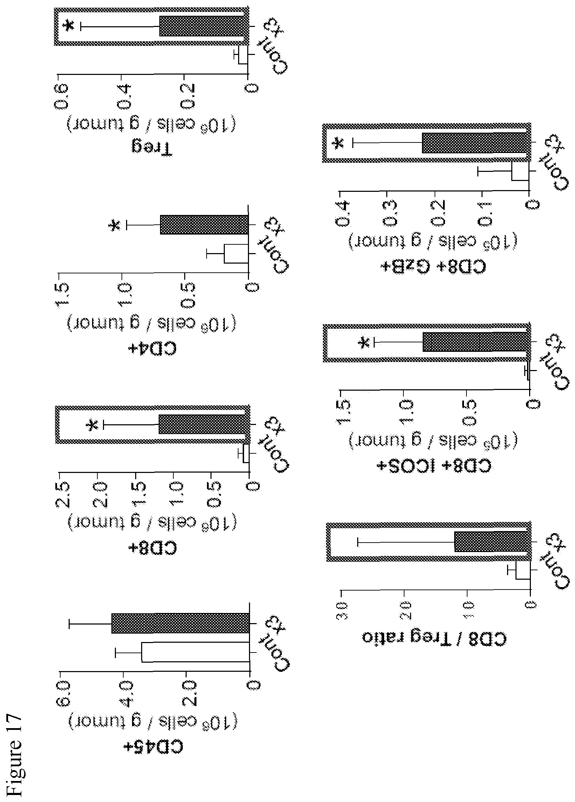

Fig. 17. Data showing that mJX594 increases the number and the effector function of intratumoral CD8+ T-cells. The ratio of CD8+ T-cells to regulatory T-cells were escalated after mJX594 treatment. Expression of ICOS and granzyme B in CD8+ T-cells was increased after mJX594 treatment. Though the number of CD4+Foxp3+CD25+ regulatory T-cell as well as CD8+ and CD4+ T-cells were simultaneously expanded in compartment of lymphoid cell, the ratio of CD8+ effector T-cells to regulatory T-cells was more escalated compared to the control. Additionally, expression of ICOS and granzyme B (GzB), which are co-stimulatory and activation markers for T-cells, was increased in CD8+ T-cells.

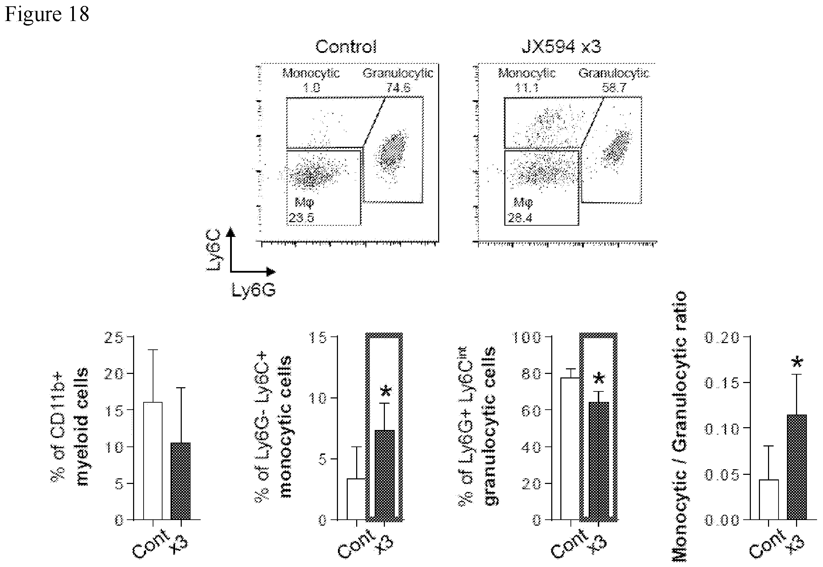

Fig. 18. Data showing that mJX594 treatment repolarized myeloid cells (Ly6G-Ly6C+↑, Ly6G+Ly6Cint ↓). mJX594 increases CD1 1b+Ly6G-Ly6C+ monocytic myeloid cells and reduces CD1 1b+Ly6G+Ly6Cint granulocytic myeloid cells. In the subset analysis of myeloid cell compartment, we discovered increases of CD1 1b+Ly6G-Ly6C+ monocytic myeloid cells and reduction of granulocytic myeloid cells with CD1 1b+Ly6G+Ly6Cint, indicating significant increase of monocytic to granulocytic ratio by mJX594 administration.

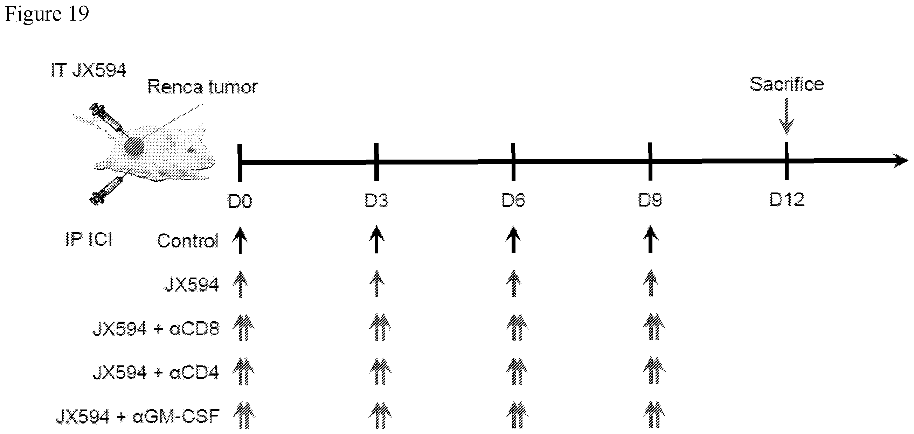

Fig. 19. Data showing a schematic for the treatment with depletion antibody experiment. To figure out which components of immune system were responsible for the therapeutic efficacy after mJX594 treatment, the effect of depletion for CD8+ T-cell, CD4+ T-cell, and GM-CSF in tumor growth and anti-cancer immunity was examined.

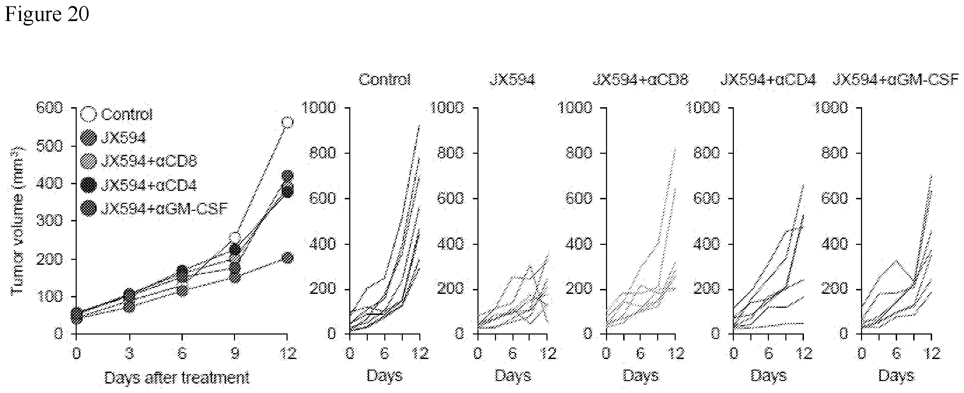

Fig. 20. Data showing that depletion of T-cells or GM-CSF significantly negated the anti-cancer effect of mJX594. Both CD8+ and CD4+ T-cell are indispensable mediators in anti-cancer effect of mJX594 treatment, and GM-CSF could also provide immunotherapeutic benefit. Though efficient tumor inhibition was detected with mJX594 monotherapy, depletion of either CD8+ or CD4+ T-cells resulted in abrogation of therapeutic effect.

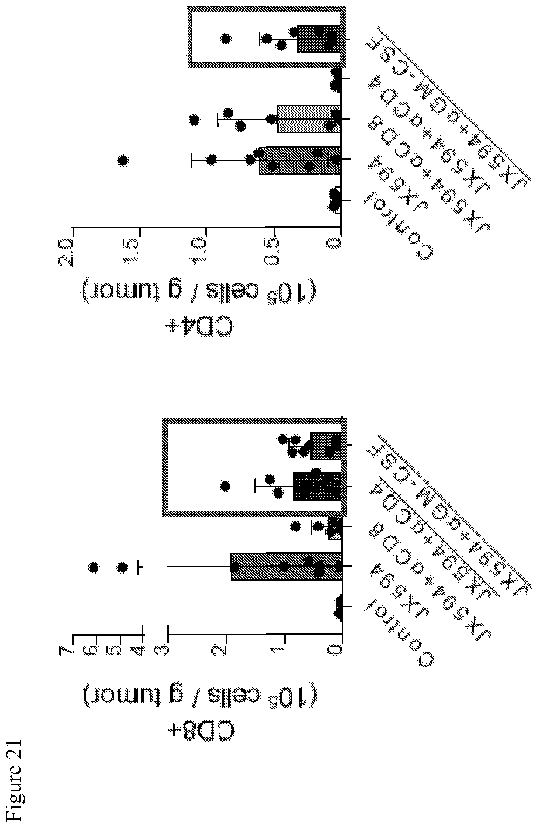

Fig. 21. Data showing that depletion of CD4+ T-cells or GM-CSF abated the intratumoral CD8+ T-cell infiltration after mJX594. Depletion of CD4+ T-cells decreased intratumoral CD8+ T-cells, suggesting that CD4+ T-cells were involved in activation of CD8+ T-cells. Depletion of GM-CSF reduced both CD8+ and CD4+ T-cells. Depletion of CD4+ T-cells with mJX594 injection decreased intratumoral CD8+ T-cells, suggesting that CD4+ T-cells were involved in activation of CD8+ T-cells. In contrast, depletion of CD8+ T-cells did not induce significant change of CD4+ T-cells indicating that CD8+ T-cells did not affect CD4+ T-cells. These data showed that treatment with mJX594 induced CD8+ and CD4+ T-cell priming which are indicative of anti-cancer immunity. Infiltration of CD8+ and CD4+ T-cells is indicative of an anti-cancer effect.

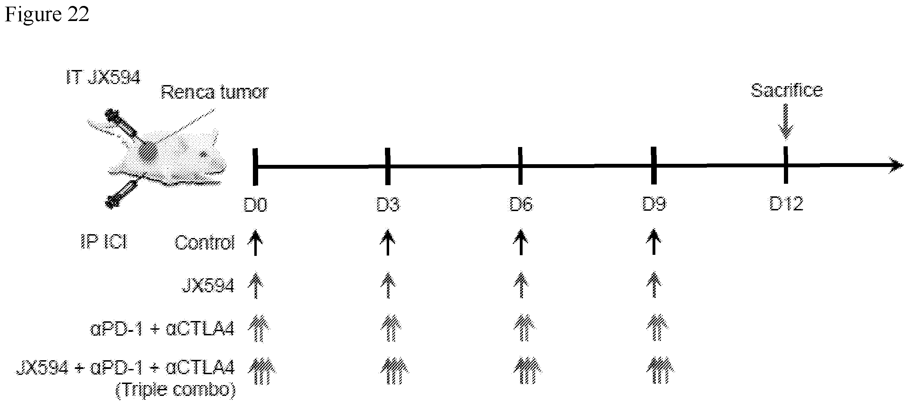

Fig. 22. A schematic of the experiment for the triple combination therapy of mJX594, αPD-1 and αCTLA-4. mJX594

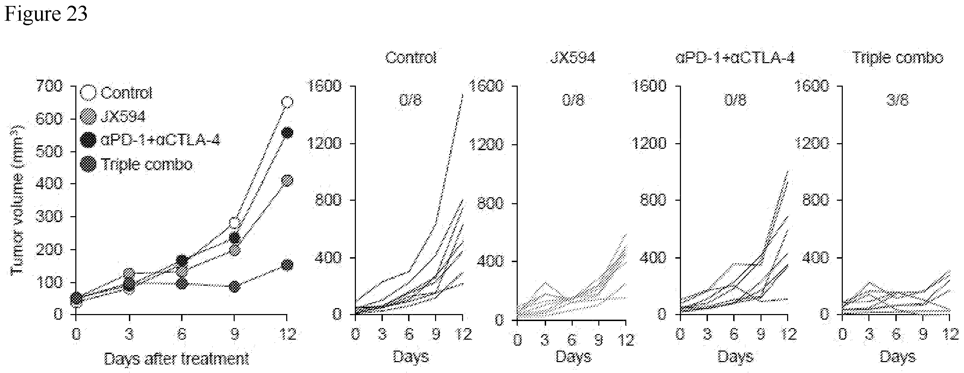

Fig. 23. Data showing the triple combination of mJX594, αPD-1 and αCTLA-4 markedly delayed the tumor growth. Notably, triple combination of mJX594, αPD-1, and αCTLA-4, caused complete regression of Renca tumor in some mice (37.5%). While dual combination of αPD-1 and αCTLA-4 delayed tumor growth by 14.5% and mJX594 monotherapy inhibited tumor growth by 36.9% compared to control, triple combination showed 76.5% tumor growth inhibition. Notably, triple combination of mJX594, αPD-1, and αCTLA-4, caused complete tumor regression (complete response rate: 37.5%), which was not observed in tumors treated with either dual combination or mJX594 monotherapy.

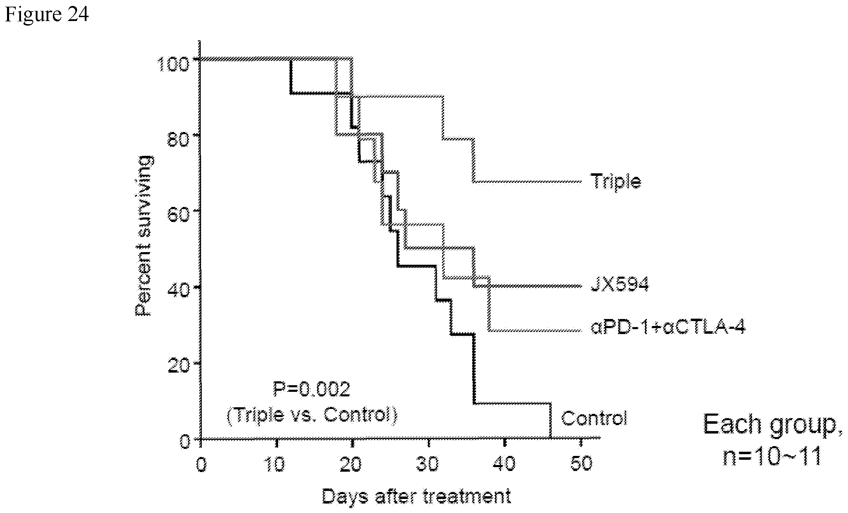

Fig. 24. Data showing the triple combination immunotherapy of mJX594, PD-1, and CTLA-4 prolongs overall survival. Mice treated with triple combination therapy showed remarkable anti-cancer treatment effects. Moreover, to confirm whether these potent anti-cancer effects induced by triple combination therapy could be translated into long term survival benefit, survival analysis of tumor-bearing mice was performed. Mice treated with triple combination therapy showed survival benefit compared to monotherapy or double combination immunotherapy.

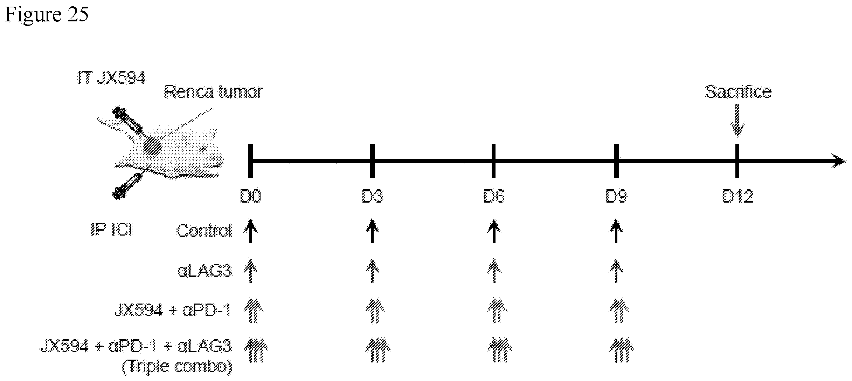

Fig. 25. A schematic of the experiment for the triple combination therapy mJX594, αPD-1 and αLAG3.

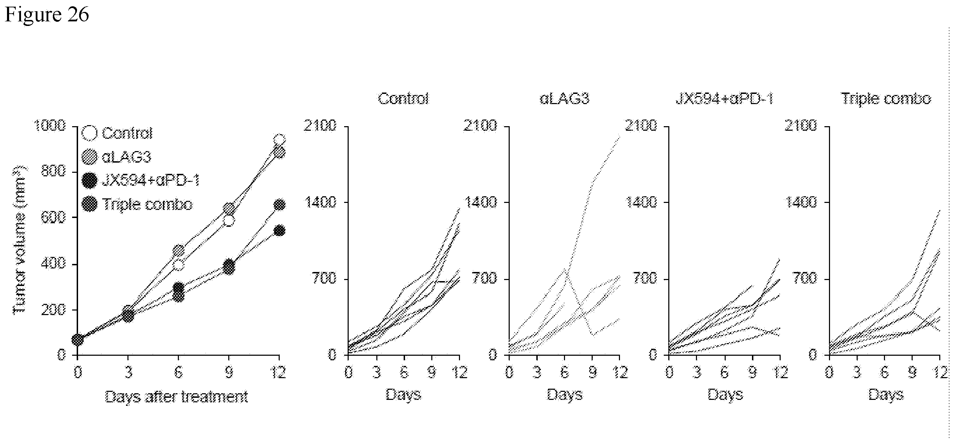

Fig. 26. Data showing the triple combination of mJX594, αPD-1 and αLAG3 moderately delayed the tumor growth. In this experiment, the triple combination did not show a statistically significant difference compared to dual combination of mJX594 and αPD-1. While dual combination of mJX594 and αPD-1 delayed tumor growth by 41.9% and αLAG3 monotherapy inhibited tumor growth by 5.7% compared to control. Triple combination showed 30.1% tumor growth inhibition.

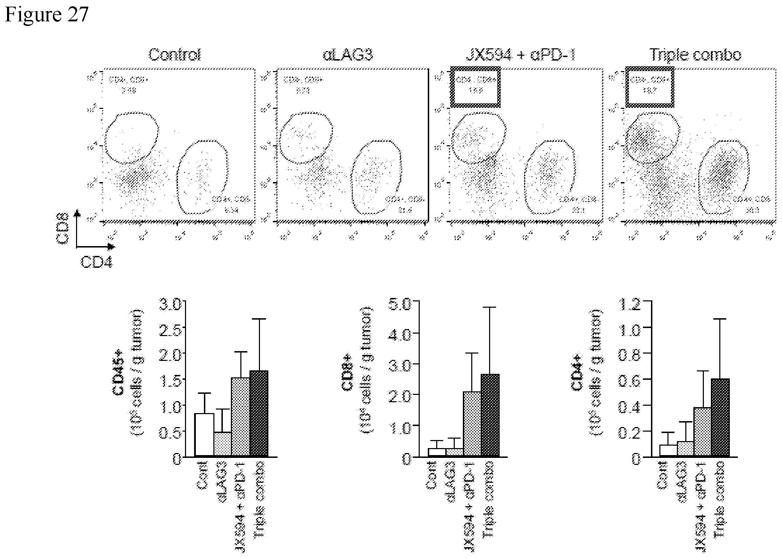

Fig. 27. Data showing the triple combination of mJX594, αPD-1 and αLAG3 increased CD8+ and CD4+ T-cells. Subset analysis of lymphoid cell compartment revealed an increase in the absolute number of intratumoral CD8+ and CD4+ T-cells with dual and triple combination treatments.

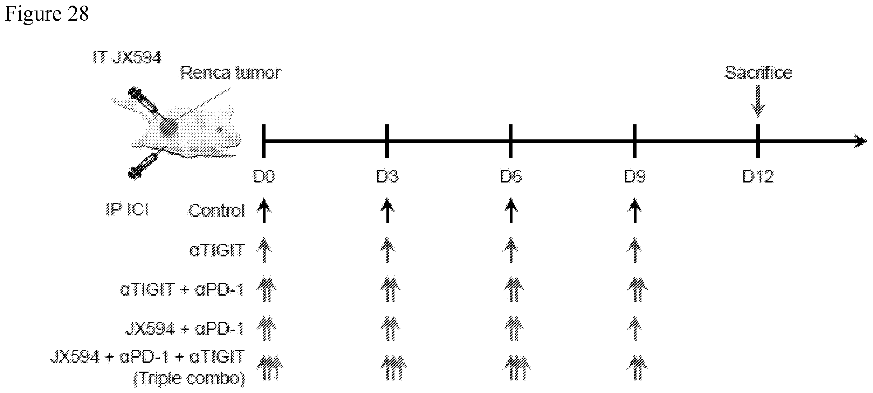

Fig. 28. A schematic of the experiment for the triple combination therapy mJX594, αPD-1 and αTIGIT.

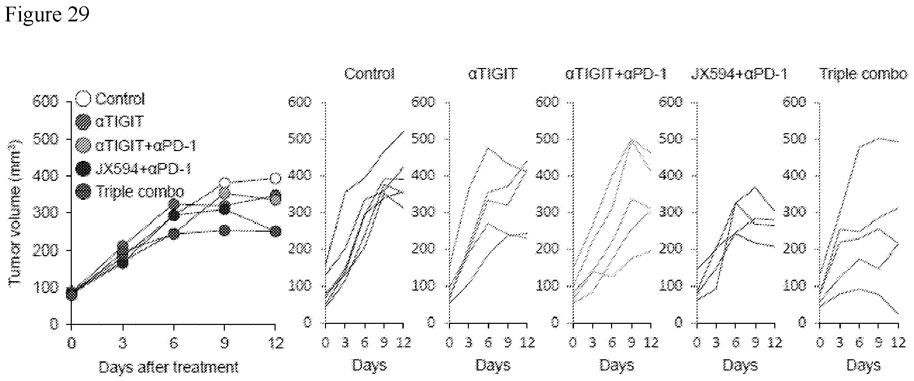

Fig. 29. Data showing the triple combination of mJX594, αPD-1 and αTIGIT moderately delayed the tumor growth. Triple combination did not show significant difference compared to dual combination of mJX594 and αPD-1 in Renca tumor.

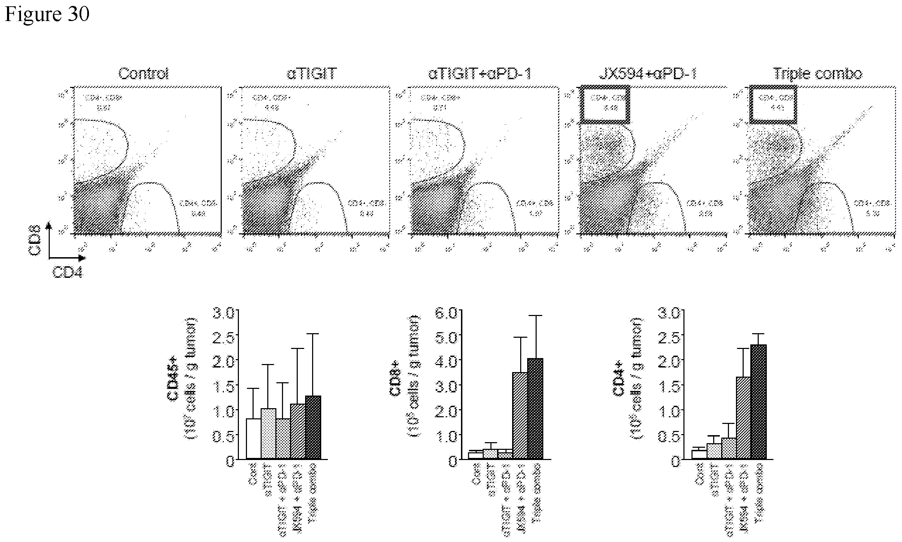

Fig. 30. Data showing the triple combination of mJX594, αPD-1 and αTIGIT increased CD8+ and CD4+ T-cells. Subset analysis of lymphoid cell compartment revealed an increase in the absolute number of intratumoral CD8+ and CD4+ T-cells with dual and triple combination treatments.

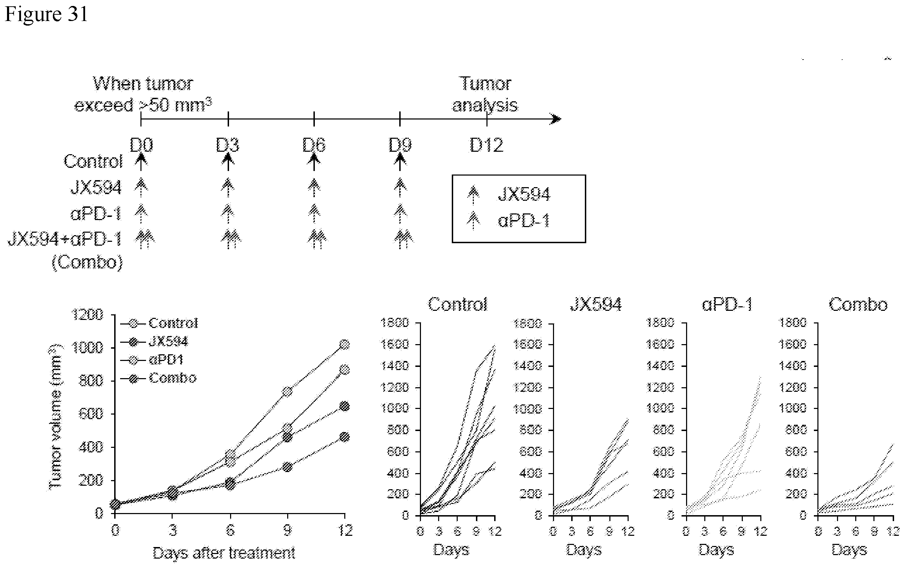

Fig. 31. Data showing that mJX594 synergizes with anti-PD1 treatment to delay colon cancer growth. To overcome the resistance to ICI monotherapy, combination efficacy of mJX594 and immune checkpoint blockade in the CT26 colon cancer model was evaluated. In this model, αPD-1 monotherapy showed little effect on tumor growth with mJX594 monotherapy moderately inhibiting tumor growth. However, combination of mJX594 and αPD-1 antibody dual therapy noticeably impeded tumor growth.

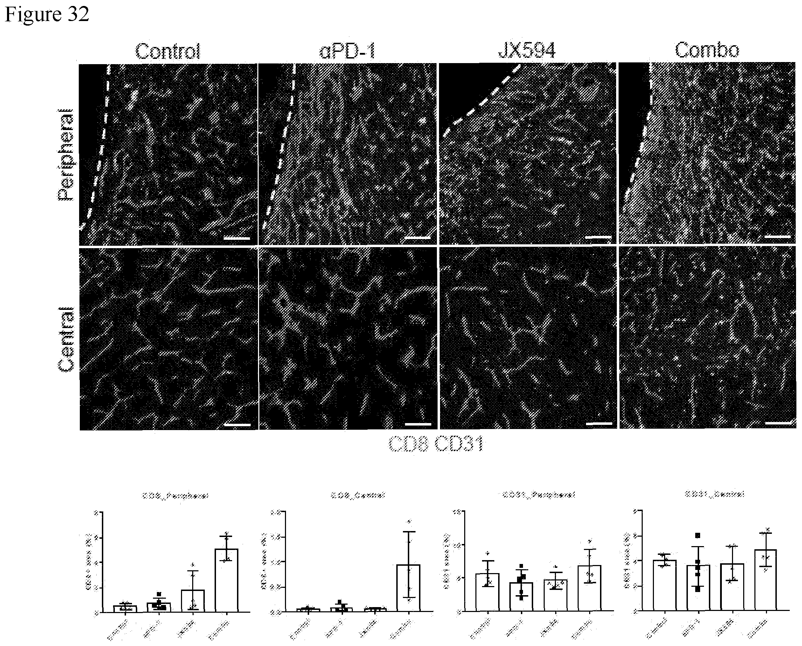

Fig. 32. Data showing that the combination of mJX594 and anti-PD1 treatment increased intratumoral CD8+ T-cells. Along with tumor growth inhibition, microscopic analyses displayed notable recruitment of CD8+ T-cells in both peripheral and central regions of tumors treated with combination therapy.

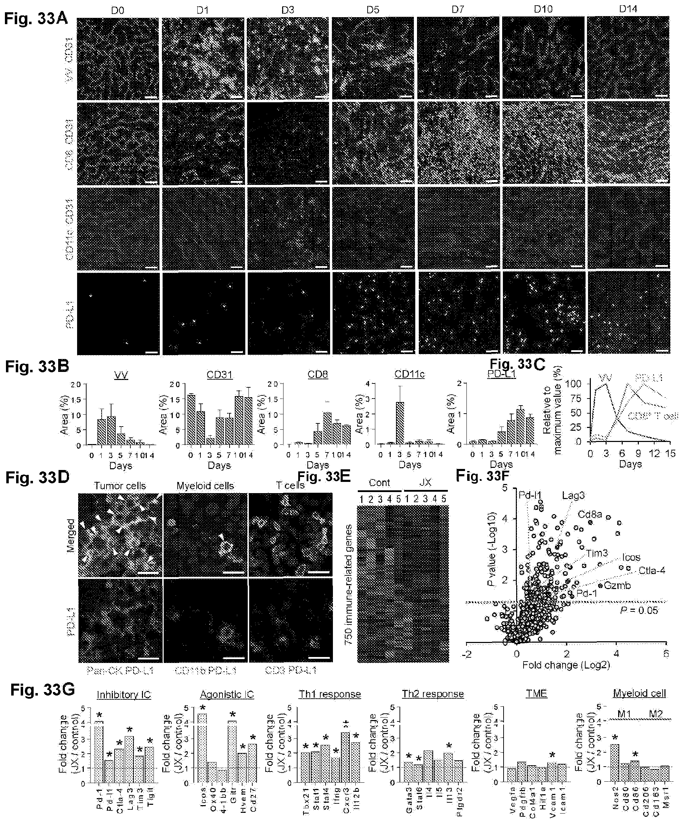

Fig. 33A-33G. mJX594 (JX) elicits dynamic changes of immune-related genes in immunosuppressive TME. Renca tumors were implanted s.c. into BALB/c mice and treated with a single i.t. injection of 1 × 10⁷ pfu of mJX-594 when tumors reached > 50 mm³. (A) Representative images of Renca tumors treated with JX. Tumors stained with vaccinia virus (VV), CD31, CD8, CD11c, and PD-L1. (B) Quantifications of expressions of vaccinia virus⁺, CD31⁺ blood vessels, CD8⁺ cytotoxic T cells, CD11c⁺ dendritic cells, and PD-L1⁺ cells. (C) Temporal changes of VV, CD8, and PD-L1 in tumor microenvironment after JX treatment. (D) Images showing upregulated PD-L1 expression (red) in various cell types (green) within TME after JX treatment. Note that the expression of PD-L1 was mainly observed in Pan-CK⁺ tumor cells (arrowheads), but some CD11b⁺ myeloid cells (arrowhead) also occasionally expressed PD-L1, while CD3⁺ T cells did not. (E) NanoString immune-related gene expression heat map. Red and green color represent up- and down- regulations, respectively. (F) Volcano plot showing gene expressions in JX treated tumors. Genes related to immune stimulation are indicated. Red line indicates p < 0.05. (G) Comparison of gene expression related to inhibitory immune checkpoints (ICs), agonistic ICs, Th1 response, Th2 response, TME, and myeloid cell. Unless otherwise denoted, n = 5 for each group. Values are mean ± SEM. *p < 0.05 versus control. Scale bars, 50 µm. Some data also showin in Figure 13.

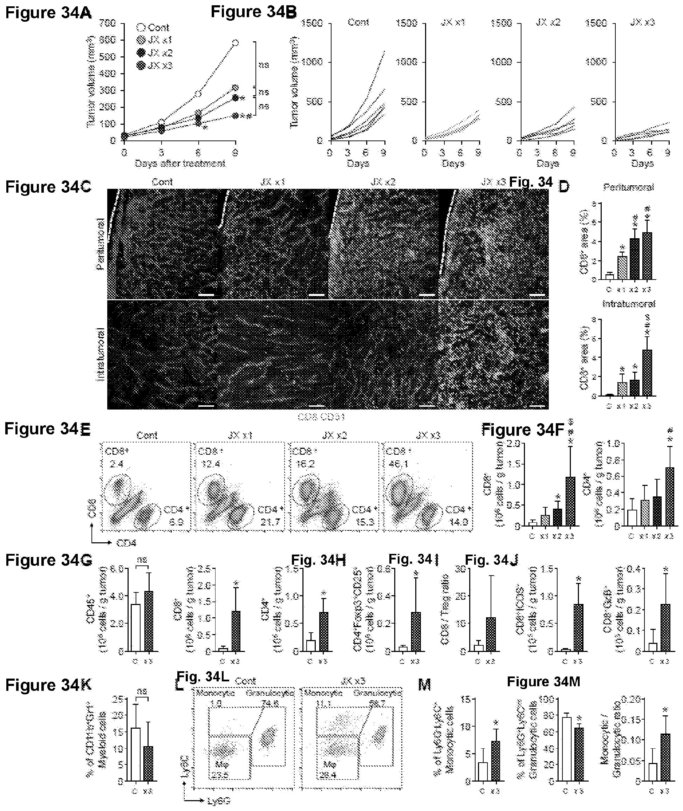

Fig. 34A-34M. JX suppresses tumor growth with increased T cell infiltration and modulation of myeloid cell. Renca tumor bearing mice were i.t. treated with PBS or 1 × 10⁷ pfu of JX 1 to 3 times. (A-B) Comparison of tumor growth in mice treated with JX. Mean (A) and individual (B) tumor growth curve over time. (C-D) Representative images (C) and comparisons (D) of CD8⁺ T cell in peri- or intratumoral regions of tumors treated with 1 to 3 times of JX. (E) Representative flow cytometric plot showing CD8⁺ and CD4⁺ T cell fractions in tumor. (F) Absolute numbers of CD8⁺ and CD4⁺ T cells per gram of tumor calculated from flow cytometry. (G) Comparison of fractions of CD45⁺, CD8⁺, and CD4⁻ in tumors treated with the triple administration of JX. (H-J) Comparison of fractions of CD4⁺Foxp3⁺CD25⁺ (Treg), CD8/Treg ratio, CD8⁺ICOS⁺, and CD8⁺GzB⁺ in tumor. (K) Comparison of fractions of CD1 1b⁺Gr1⁺ myeloid cells in tumor. (L) Representative flow cytometric plot showing CD11b⁺ myeloid cell fractions in tumor. (M) Comparison of fractions of Ly6G⁻Ly6C⁺ monocytic myeloid cells, Ly6G⁺Ly6C^{int} granulocytic myeloid cells, and monocytic/granulocytic ratio on CD11b⁺ in tumor. Unless otherwise denoted, n = 5~6 for each group. Values are mean ± SEM. *p < 0.05 versus control; ^{#}p < 0.05 versus JX x1; ^{$}p < 0.05 versus JX x2. ns, not significant. Scale bars, 100 µm. Some data also showin in Figures 19-21.

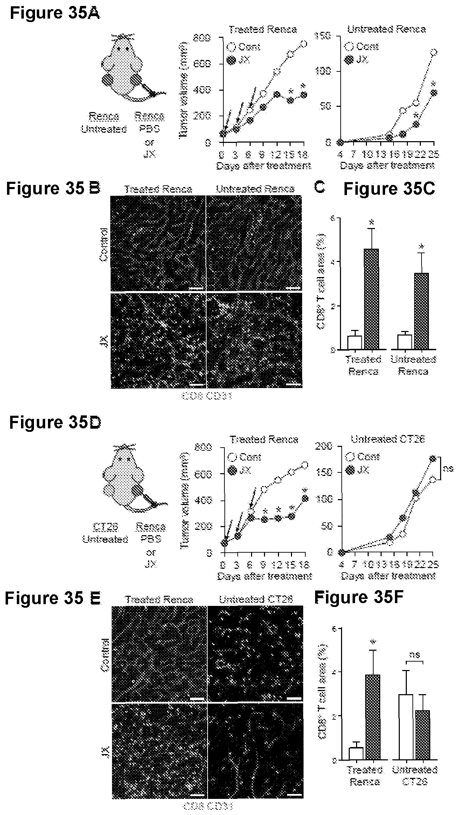

Fig. 35A-35E. Intratumoral injections of JX induce CD8⁺ lymphocyte infiltration in both local and distant tumors. Mice were s.c. injected with Renca in the right flank and with Renca or CT26 tumor in the left flank. Arrows indicated i.t. JX treatment. (A) Schematic diagram of tumor implantation and treatment, and growth curves of JX-injected Renca tumor and non-injected Renca tumor. (B-C) Representative images (B) and comparisons (C) of CD8⁺ T cells (green) in the JX-injected and non-injected tumors. (D) Schematic diagram of implantation and treatment, and growth curve of JX-injected Renca tumor and non-injected CT26 tumor. (E-F) Representative images (E) and comparisons (F) of CD8⁺ T cells (green) in the JX-injected and distant tumors. Unless otherwise denoted, n = 5 for each group. Values are mean ± SEM. *p < 0.05 versus control. ns, not significant. Scale bars, 50 µm.

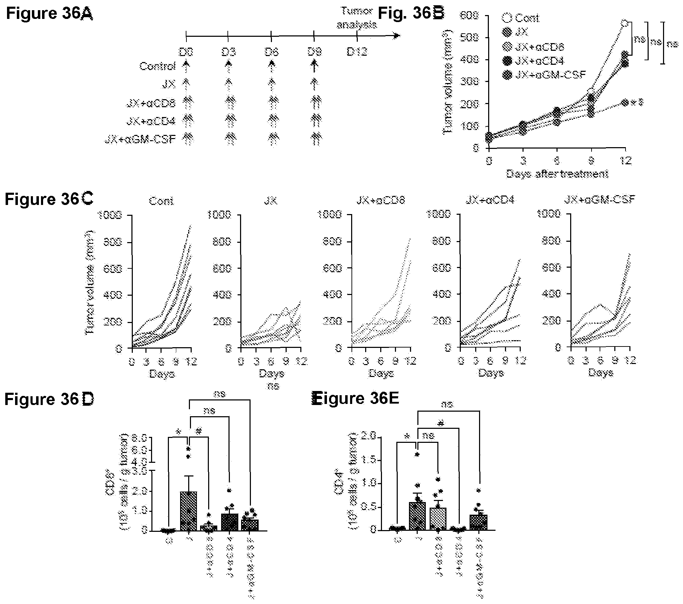

Fig. 36A-36D. Anti-tumor immunity plays an important role in the overall therapeutic efficacy of JX. Mice were s.c. implanted with Renca and treated with i.t. JX or i.p. depleting antibodies for CD8⁺, CD4⁺ T cells or mouse GM-CSF. (A) Treatment scheme. (B-C) Comparison of tumor growth in mice treated with JX or depleting antibodies. Mean (B) and individual (C) tumor growth curve over time. *p < 0.05 versus control; ^{$}p < 0.05 versus αGM-CSF. (D-E) Absolute numbers of CD8⁺ (D) and CD4⁺ (E) T cells per gram of tumors treated with JX and immune cell-depleting antibodies. Unless otherwise denoted, n = 7~8 for each group. Values are mean ± SEM. *p < 0.05 versus control; ^{#}p < 0.05 versus VX; ^{$}p < 0.05 versus αGM-CSF. ns, not significant.

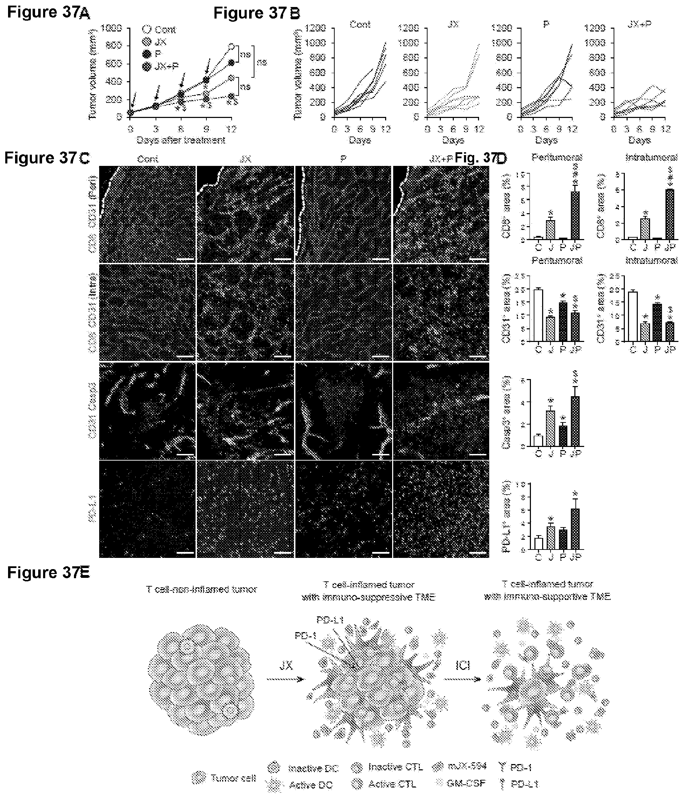

Fig. 37A-37E. Combination therapy of JX and αPD-1 synergistically elicits CD8⁺ T cell-mediated tumor immunity. Renca tumor bearing mice were treated with either PBS, JX, αPD-1 antibody, or JX plus αPD-1 antibody. (A-B) Comparison of tumor growth in mice treated with JX and/or αPD-1 antibody. Mean (A) and individual (B) tumor growth curve over time. (C-D) Representative images (C) and comparisons (D) of CD8⁺ T cells, CD31⁺ blood vessels, activated caspase3 (Casp3)⁺ apoptotic cells, and PD-L1⁺ cells in tumor treated with JX and/or αPD-1 antibody. (E) Diagram depicting overcoming of immunosuppressive TME by combination therapy of JX and αPD-1 blockade. Unless otherwise denoted, n = 7 for each group. Values are mean ± SEM. *p < 0.05 versus control; ^{#}p < 0.05 versus JX; ^{$}p < 0.05 versus αPD-1. ns, not significant. Scale bars, 100 µm. Some data also showin in Figures 19-21.

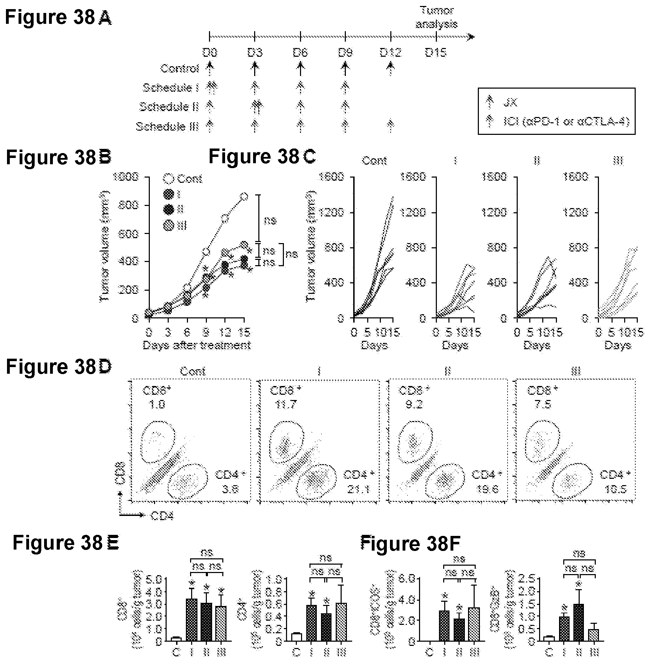

Fig. 38A-38F. The efficacy of combination immunotherapy with intratumoral JX and systemic ICIs is not largely affected by treatment schedule. Mice were s.c. implanted with Renca tumor and treated with JX plus ICIs on various schedules. (A) Diagram depicting various treatment schedule. Arrows indicate treatment with either i.t. delivery of JX (red arrows) or systemic delivery of immune checkpoint blockade (blue arrows). (B-C) Comparison of tumor growth in mice treated with JX and αPD-1 antibody using different time schedules. Mean (B) and individual (C) tumor growth curve over time. (D) Representative flow cytometric plot showing tumor-infiltrating CD8⁺ and CD4⁺ T cell fractions. (E-F) Comparisons of absolute numbers of CD8⁺, CD4⁺, CD8⁺ICOS⁺, and CD8⁺GzB⁺ cells per gram of tumors. Unless otherwise denoted, n = 7 for each group. Values are mean ± SEM. *p < 0.05 versus control. ns, not significant.

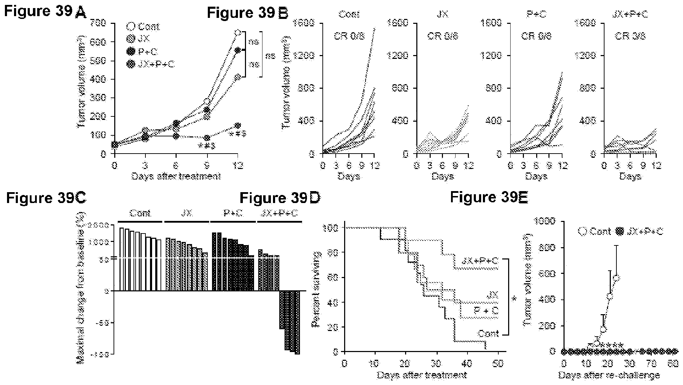

Fig. 39A-39E. The triple combination of JX, αPD-1 and αCTLA-4 antibodies leads to complete regression and improved overall survival. Mice were s.c. implanted with Renca tumor and treated with JX in the presence or absence of immune checkpoint blockade for PD-1 and CTLA-4. (A-B) Comparison of tumor growth in mice treated with JX and/or immune checkpoint blockades. Mean (A) and individual (B) tumor growth curve over time. (C) Waterfall plot showing the maximal percent changes from baseline in tumor size. (D) Kaplan-Meier plot for overall survival. (E) Comparison of tumor size after re-challenge of Renca tumor cells in mice with complete tumor regression. Unless otherwise denoted, n = 8 for each group. *p < 0.05 versus control; ^{#}p < 0.05 versus JX; ^{$}p < 0.05 versus αPD-1+ αCTLA-4. ns, not significant. Some data also showin in Figures 23 and 24.

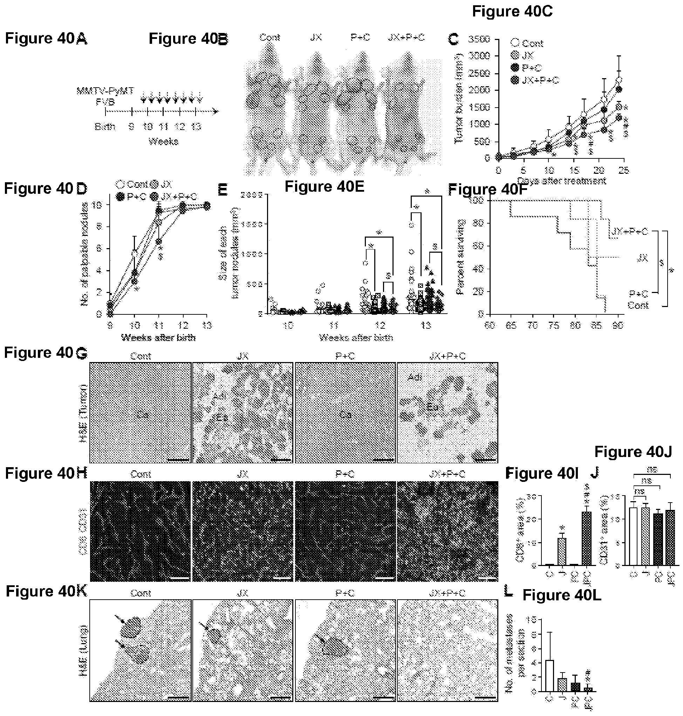

Fig. 40A-40L. The triple combination therapy delays tumor growth and metastasis in spontaneous breast cancer model. Tumor growth was analyzed weekly in spontaneous mammary tumors of *MMTV-PyMT* mice starting from 9 weeks after birth. Samples were harvested 13 weeks after birth. (A) Diagram depicting treatment schedule. Arrows indicate treatment with either i.t. delivery of JX or systemic delivery of αPD-1 and αCTLA-4 antibodies. (B) Representative image showing gross appearance of tumors. Dotted-line circles demarcate palpable mammary tumor nodules. (C) Comparison of total tumor burden. Tumor burden was calculated by summating the volume of every tumor nodules per mouse. (D) Comparison of number of palpable tumor nodules. (E) Comparison of volume of each tumor nodule. Each tumor nodule in *MMTV*-*PyMT* mice were plotted as individual dots. (F) Kaplan-Meier curves for overall survival. (G) Tumor sections with H&E showing intratumoral regions. Acinar structures of JX and JX+P+C groups are early, less-invasive lesions (Ea) showing the distinct boundary with the surrounding mammary adipose tissue (Adi). Whereas, invasive ductal carcinoma regions (Ca) of Cont and P+C that have massively invaded the surrounding tissue and formed solid sheets of tumor cells with no remaining acinar structure. Scale bars, 200 µm. (H-J) Representative images and comparisons of CD8⁺ T cells (H and I) and CD31⁺ tumor blood vessels (H and J) in tumor. (K) Representative lung sections stained with H&E. Arrows indicated metastatic foci. Scale bars, 200 µm. (L) Comparison of number of metastatic colonies per lung section. Unless otherwise denoted, n = 6~7 for each group. Values are mean ± SEM. *p < 0.05 versus control; ^{#}p < 0.05 versus JX; ^{$}p < 0.05 versus αPD-1+ αCTLA-4. ns, not significant. Scale bars, 100 µm.

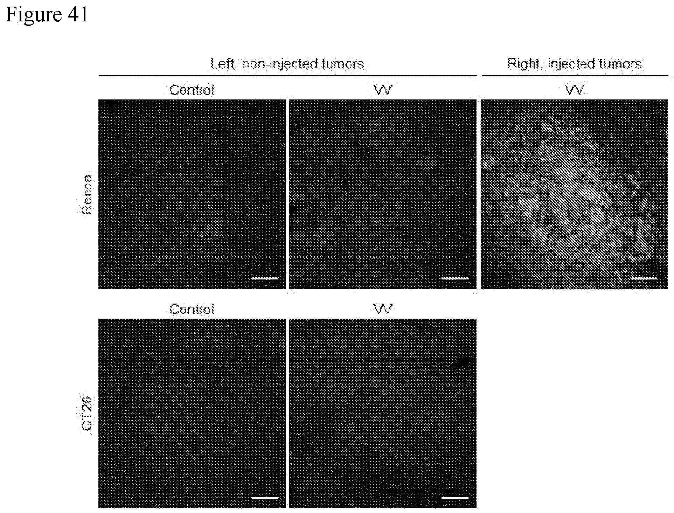

Fig. 41. Vaccinia virus is not detected in distant tumors. Although robust vaccinia virus (VV) replication (green) is observable in right, injected tumors, vaccinia virus was not detected in left, non-injected tumors. Scale bars, 100 µm.

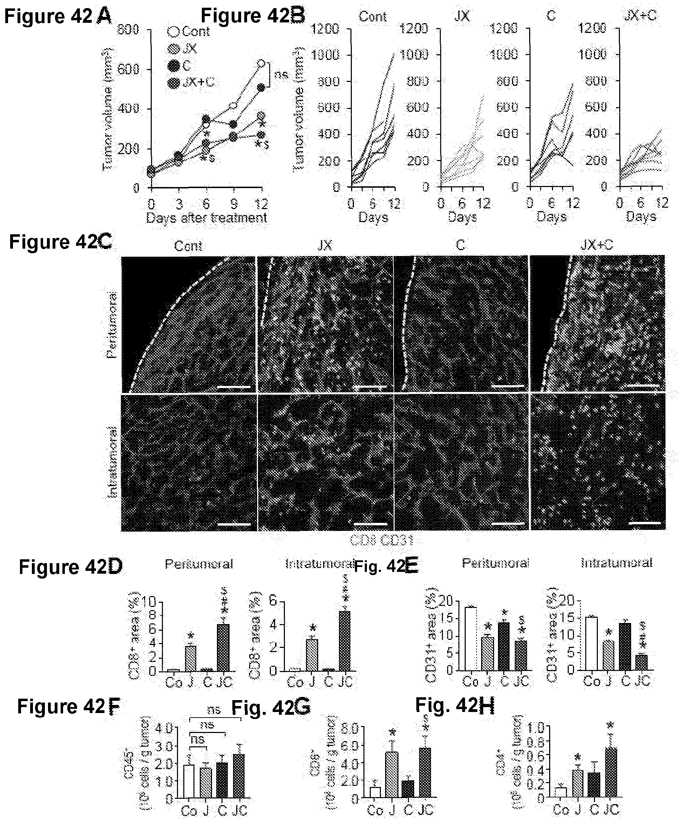

Fig. 42A-42H. Combination therapy of JX and ^CTLA-4 synergistically elicits CD8+ T cell-mediated tumor immunity. Mice bearing Renca tumors were treated with either PBS, JX, αCTLA-4 antibody, or JX plus αCTLA-4 antibody. (A-B) Comparison of tumor growth in mice treated with JX and/or αCTLA-4 antibody. Mean (A) and individual (B) tumor growth curve over time. (C-E) Images and comparisons of CD8+ T cells (C and D) and CD31+ blood vessel (C and E) in tumor. (F-H) Absolute numbers of CD45+ immune cells (F), CD8+ T cells (G), and CD4+ cells (H) per gram of tumors threated with JX and/or αCTLA-4 antibody. Values are mean ± SEM. *p < 0.05 versus control; #p < 0.05 versus JX; $p < 0.05 versus αCTLA-4. ns, not significant. Scale bars, 100 µm.

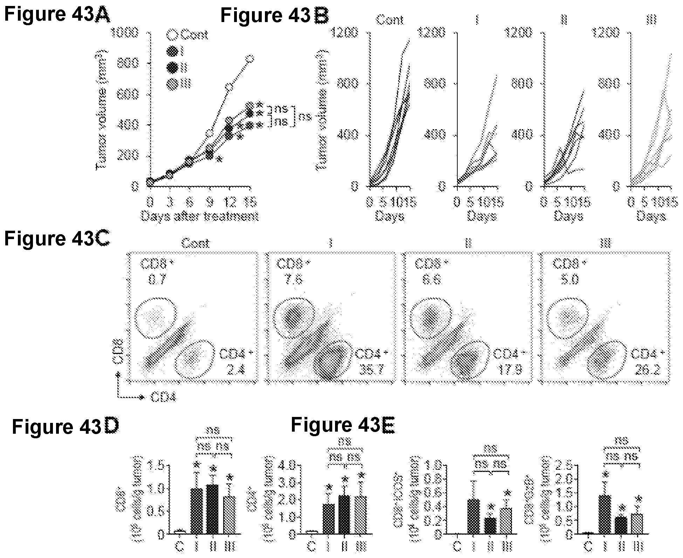

Fig. 43A-43E. JX potentiates the anti-cancer efficacy with immune response of ^CTLA-4 regardless of treatment schedules. (A-B) Comparison of tumor growth in mice treated with JX and αCTLA-4 antibody using different timing schemes. Mean (A) and individual (B) tumor growth curve over time. (C) Representative flow cytometric plot showing tumor-infiltrating CD8+ and CD4+ T cell fractions in tumor. (D-E) Absolute numbers of CD8+, CD4+, CD8+ICOS+, and CD8+GzB+ cells per gram of tumors treated with JX and αCTLA-4 antibody. Values are mean ± SEM. *p < 0.05 versus control. ns, not significant. Some data also showin in Figure 38.

### DETAILED DESCRIPTION OF THE INVENTION

### I. SELECT DEFINITIONS

The terms "inhibiting," "reducing," or "prevention," or any variation of these terms, when used in the claims and/or the specification includes any measurable decrease or complete inhibition to achieve a desired result.

As used herein, the term "combination" means the combined administration of the anti-cancer agents, namely the oncolytic vaccinia virus and the immune checkpoint inhibitor, which can be dosed independently or by the use of different fixed combinations with distinguished amounts of the combination partners. The term "combination" also defines a "kit" comprising the combination partners which are to be administered simultaneously. Preferably, the time intervals between consecutive simultaneous administrations of the combination partners are chosen such that the combination of agents shows a synergistic effect. As used herein, the term "synergistic" or "synergy" means that the effect achieved with the combinations of anticancer agents encompassed in this invention is greater than the sum of the effects that result from using anti-cancer agents namely the oncolytic vaccinia virus and the immune checkpoint inhibitor as a monotherapy. Advantageously, such synergy provides greater efficacy at the same doses, and/or prevents or delays the build-up of multidrug resistance.

The term "refractory cancer," as used herein refers to cancer that either fails to respond favorably to an anti-neoplastic treatment, or alternatively, recurs or relapses after responding favorably to an antineoplastic treatment. Accordingly, "a cancer refractory to a treatment" as used herein means a cancer that fails to respond favorably to, or resistant to, the treatment, or alternatively, recurs or relapses after responding favorably to the treatment. For example, such a prior treatment may be a chemotherapy regimen or may be an immunotherapy regimen comprising administration of a monoclonal antibody that specifically binds to PD-1, PD-L1 or CTLA4.

The use of the word "a" or "an" when used in conjunction with the term "comprising" in the claims and/or the specification may mean "one," but it is also consistent with the meaning of "one or more," "at least one," and "one or more than one."

It is contemplated that any embodiment discussed herein can be implemented with respect to any method or composition of the invention, and vice versa. Furthermore, compositions and kits of the invention can be used to achieve methods of the invention.

Throughout this application, the term "about" is used to indicate that a value includes the standard deviation of error for the device or method being employed to determine the value.

The use of the term "or" in the claims is used to mean "and/or" unless explicitly indicated to refer to alternatives only or the alternatives are mutually exclusive, although the disclosure supports a definition that refers to only alternatives and "and/or."

As used in this specification and claim(s), the words "comprising" (and any form of comprising, such as "comprise" and "comprises"), "having" (and any form of having, such as "have" and "has"), "including" (and any form of including, such as "includes" and "include") or "containing" (and any form of containing, such as "contains" and "contain") are inclusive or open-ended and do not exclude additional, unrecited elements or method steps.

Other objects, features and advantages of the present invention will become apparent from the following detailed description. It should be understood, however, that the detailed description and the specific examples, while indicating specific embodiments of the invention, are given by way of illustration only, since various changes and modifications within the spirit and scope of the invention will become apparent to those skilled in the art from this detailed description.

It has been found that combination therapy with an oncolytic vaccinia virus and a checkpoint inhibitor results in unexpected improvement in the treatment of cancer. When the agents are concurrently administered and the oncolytic vaccinia virus is administered, the agents interact cooperatively and even synergistically to provide significantly improved antitumoral effects relative to single administration of either agent. Surprisingly, these effects are not prominently observed if the agents are administered sequentially. In some embodiments, the combination therapy provides synergistic effects when the replicative oncolytic virus is administered intratumorally. In some embodiments, the combination therapy provides synergistic effects when the replicative oncolytic virus is administered via intravenous administration. In some embodiments, the combination therapy provides synergistic effects when the replicative oncolytic virus is administered via intraperitoneal administration. In some embodiments, the when the replicative oncolytic virus is only delivered via intratumoral administration. In some embodiments, the when the replicative oncolytic virus is only delivered via intra-arterial administration. In some embodiments, the checkpoint inhibitor is administered systemically. In some embodiments, the combination therapy provides synergistic effects when the replicative oncolytic virus is administered intratumorally and the checkpoint inhibitor is administered systemically. In some embodiments, the combination therapy provides synergistic effects when the replicative oncolytic virus is administered intratumorally and the checkpoint inhibitor is administered systemically. In some embodiments, the combination therapy provides synergistic effects when the replicative oncolytic virus is administered intraperitoneally and the checkpoint inhibitor is administered systemically. In some embodiments, the combination therapy provides synergistic effects when the replicative oncolytic virus is administered intra-arterially and the checkpoint inhibitor is administered systemically.

It has further been found that oncolytic vaccinia virus upregulates expression of checkpoint proteins such as PD-1, PD-L1, CTLA-4, TIM3, LAG3 and TIGIT in human tumors thereby sensitizing the tumors to treatment with checkpoint inhibitors and supporting the present combination therapy not only in patients that with tumors that express a checkpoint inhibitor of the combination but also in patients with tumors that do not express a checkpoint inhibitor of the combination or express relatively low levels of checkpoint inhibitor.

In several embodiments, a combination therapy for use in the treatment and/or prevention of cancer and/or the establishment of metastases in a mammal (preferably a human) is provided comprising concurrently administering to the mammal (i) a replication competent oncolytic vaccinia virus and (ii) one or more immune checkpoint inhibitors. In some embodiments, the replication competent oncolytic vaccinia virus is administered intratumorally. In some embodiments, the replication competent oncolytic vaccinia virus is administered intravenously. In some embodiments, the replication competent oncolytic vaccinia virus is administered only intratumorally. In some embodiments, the replication competent oncolytic vaccinia virus is administered only intra-arterially. In some embodiments, the combination therapy provides synergistic effects when the replicative oncolytic virus is administered via intraperitoneal administration. In some embodiments, the checkpoint inhibitor is administered systemically. In some embodiments, the replicative oncolytic virus is administered intratumorally and the checkpoint inhibitor is administered systemically. In some embodiments, the replicative oncolytic virus is administered intratumorally and the checkpoint inhibitor is administered systemically. In some embodiments, the replicative oncolytic virus is administered intraperitoneally and the checkpoint inhibitor is administered systemically. In some embodiments, the replicative oncolytic virus is administered intra-arterially and the checkpoint inhibitor is administered systemically.

### II. ONCOLYTIC VACCINIA VIRUS

Vaccinia virus is a large, complex enveloped virus having a linear double-stranded DNA genome of about 190K bp and encoding for approximately 250 genes. Vaccinia is well-known for its role as a vaccine that eradicated smallpox. Post-eradication of smallpox, scientists have been exploring the use of vaccinia as a tool for delivering genes into biological tissues (gene therapy and genetic engineering). Vaccinia virus is unique among DNA viruses as it replicates only in the cytoplasm of the host cell. Therefore, the large genome is required to code for various enzymes and proteins needed for viral DNA replication. During replication, vaccinia produces several infectious forms which differ in their outer membranes: the intracellular mature virion (IMV), the intracellular enveloped virion (IEV), the cell-associated enveloped virion (CEV) and the extracellular enveloped virion (EEV). IMV is the most abundant infectious form and is thought to be responsible for spread between hosts. On the other hand, the CEV is believed to play a role in cell-to-cell spread and the EEV is thought to be important for long range dissemination within the host organism.

Any known oncolytic strain of vaccinia virus may be concurrently administered with a checkpoint inhibitor according to the methods herein described. In preferred embodiments, the replicative oncolytic vaccinia virus is a Copenhagen, Western Reserve, Lister or Wyeth strain, most preferably a Western Reserve or Wyeth strain. The genome of the Western Reserve vaccinia strain has been sequenced (Accession number AY243312). In some embodiments, the replicative oncolytic vaccinia virus is a Copenhagen strain. In some embodiments, the replicative oncolytic vaccinia virus is a Western Reserve strain. In some embodiments, the replicative oncolytic vaccinia virus is a Lister strain. In some embodiments, the replicative oncolytic vaccinia virus is a Wyeth strain.

The replicative oncolytic vaccinia virus may be engineered to lack one or more functional genes in order to increase the cancer selectivity of the virus. In some preferred embodiments, the oncolytic vaccinia virus is engineered to lack thymidine kinase (TK) activity. A TK-deficient vaccinia virus requires thymidine triphosphate for DNA synthesis, which leads to preferential replication in dividing cells (particularly cancer cells). In another aspect, the oncolytic vaccinia virus may be engineered to lack vaccinia virus growth factor (VGF). This secreted protein is produced early in the infection process, acting as a mitogen to prime surrounding cells for infection. In another aspect, the oncolytic vaccinia virus may be engineered to lack both VFG and TK activity. In other aspects, the oncolytic vaccinia virus may be engineered to lack one or more genes involved in evading host interferon (IFN) response such as E3L, K3L, B18R, or B8R. In some preferred embodiments, the replicative oncolytic vaccinia virus is a Western Reserve, Copenhagen, Lister or Wyeth strain and lacks a functional TK gene. In other embodiments, the oncolytic vaccinia virus is a Western Reserve, Copenhagen, Lister or Wyeth strain lacking a functional B18R and/or B8R gene. In some embodiments, the replicative oncolytic vaccinia virus is a Western Reserve, Copenhagen, Lister or Wyeth strain and lacks a functional TK gene. In some embodiments, the replicative oncolytic vaccinia virus is a Western Reserve strain and lacks a functional TK gene. In some embodiments, the replicative oncolytic vaccinia virus is a Copenhagen strain and lacks a functional TK gene. In some embodiments, the replicative oncolytic vaccinia virus is a Lister strain and lacks a functional TK gene. In some embodiments, the replicative oncolytic vaccinia virus is a Wyeth strain and lacks a functional TK gene. In some embodiments, the oncolytic vaccinia virus is a Western Reserve, Copenhagen, Lister or Wyeth strain lacking a functional B18R and/or B8R gene. In some embodiments, the oncolytic vaccinia virus is a Western Reserve strain lacking a functional B18R and/or B8R gene. In some embodiments, the oncolytic vaccinia virus is a Copenhagen strain lacking a functional B18R and/or B8R gene. In some embodiments, the oncolytic vaccinia virus is a Lister strain lacking a functional B18R and/or B8R gene. In some embodiments, the oncolytic vaccinia virus is a Wyeth strain lacking a functional B18R and/or B8R gene. In some embodiments, the replicative oncolytic vaccinia virus is a Western Reserve, Copenhagen, Lister or Wyeth strain and lacks a functional TK gene as well as lacking a functional B18R and/or B8R gene. In some embodiments, the replicative oncolytic vaccinia virus comprises functional 14L and/or F4L genes. In some embodiments, the replicative oncolytic vaccinia virus does not express a chemokine (e.g., the vaccinia virus does not express CXCL-11).

In some embodiments, the replicative oncolytic vaccinia virus of the combination comprises functional 14L and/or F4L genes.

In other embodiments, the replicative oncolytic vaccinia virus of the combination does not express a chemokine (e.g. the vaccinia virus does not express CXCL-11).

Heterologous sequence (e.g. encoding a cytokine and/or a tumor antigen) can be placed under the control of a vaccinia virus promoter and integrated into the genome of the vaccinia virus. Alternatively, expression of the heterologous sequence can be achieved by transfecting a shuttle vector or plasmid such as those found in Table 1 of Current Techniques in Molecular Biology, (Ed. Ausubel, et al.) Unit 16.17.4 (1998) containing the vaccinia promoter-controlled sequence into a cell that has been infected with vaccinia virus and introducing the heterologous sequence by homologous recombination. Strong late vaccinia virus promoters are preferred when high levels of expression of are desired. Early and intermediate-stage promoters can also be utilized. In some embodiments, the heterologous sequence is under the control of a vaccinia virus promoter containing early and late promoter elements. Suitable early promoters include without limitation, a promoter of vaccinia virus gene coding for 42K, 19K or 25K polypeptide. Suitable early late promoters include, without limitation, a promoter of vaccinia virus gene coding for 7.5K polypeptide. Suitable late promoters include, without limitation, a promoter of vaccinia virus gene coding for 11K or 28K polypeptide. In related embodiments, the heterologous sequence is inserted into a TK and/or VGF sequence to inactivate the TK and/or VGF sequence.

In some embodiments, the replicative oncolytic vaccinia viruses described herein are administered in combination with one or more checkpoint inhibitors. In some embodiments, the replicative oncolytic vaccinia viruses described herein are administered intratumorally in combination with one or more checkpoint inhibitors. In some embodiments, the replicative oncolytic vaccinia viruses described herein are administered intravenously (IV; or intravascularly) in combination with one or more checkpoint inhibitors. In some embodiments, the replicative oncolytic vaccinia viruses described herein are administered intraperitoneally (IP) in combination with one or more checkpoint inhibitors. In some embodiments, the replicative oncolytic vaccinia viruses described herein are administered intra-arterially in combination with one or more checkpoint inhibitors. In some embodiments, the replicative oncolytic virus is only delivered via intratumoral administration. In some embodiments, the replicative oncolytic virus is administered intratumorally and the checkpoint inhibitor is administered systemically. In some embodiments, the replicative oncolytic virus is administered intravenously and the checkpoint inhibitor is administered systemically. In some embodiments, the replicative oncolytic virus is administered intraperitoneally and the checkpoint inhibitor is administered systemically. In some embodiments, the replicative oncolytic virus is administered intra-arterially and the checkpoint inhibitor is administered systemically. Intratumoral administration generally entails injection into a tumor mass or into tumor associated vasculature. In certain aspects, the tumor is imaged prior to or during administration of the virus. Intravascular administration generally entails injection into the vascular system, and is a form of systemic administration. Intraperitoneal administration generally entails injection into the peritoneum (e.g., body cavity). In some embodiments, the replicative oncolytic vaccinia viruses described herein are administered in combination with one or more checkpoint inhibitors and both are administered systemically, for example, by IV administration. In some embodiments, the replicative oncolytic vaccinia viruses described herein are administered in combination with one or more checkpoint inhibitors, wherein the replicative oncolytic vaccinia virus is administered intratumorally and the one or more checkpoint inhibitors are administered systemically, for example, by IV administration. In some embodiments, the replicative oncolytic vaccinia viruses described herein are administered in combination with one or more checkpoint inhibitors, wherein the replicative oncolytic vaccinia virus is administered intraperitoneally and the one or more checkpoint inhibitors are administered systemically, for example, by IV administration. In some embodiments, the replicative oncolytic vaccinia viruses described herein are administered in combination with one or more checkpoint inhibitors, wherein the replicative oncolytic vaccinia virus is administered intra-arterially and the one or more checkpoint inhibitors are administered systemically, for example, by IV administration.

Oncolytic vaccinia viruses as described herein may be administered in a single administration or multiple administrations (e.g. 2, 3, 4, 5, 6, 7, 8 or more times). The virus may be administered at dosage of 1 × 10⁵ plaque forming units (PFU), 5 × 10⁵ PFU, 1 × 10⁶ PFU, at least 1 × 10⁶ PFU, 5 × 10⁶ or about 5 × 10⁶ PFU, 1 × 10⁷, at least 1 × 10⁷ PFU, 1 × 10⁸ or about 1 × 10⁸ PFU, at least 1 × 10⁸ PFU, about or at least 5 × 10⁸ PFU, 1 × 10⁹ or at least 1 × 10⁹ PFU, 5 × 10⁹ or at least 5 × 10⁹ PFU, 1 × 10¹⁰ PFU or at least 1 × 10¹⁰ PFU, 5 × 10¹⁰ or at least 5 × 10¹⁰ PFU, 1 × 10¹¹ or at least 1 ×10¹¹, 1 × 10¹² or at least 1 × 10¹², 1 × 10¹³ or at least 1 × 10¹³. For example, the virus may be administered at a dosage of between about 10⁶-10¹³ pfu, between about 10⁷-10¹³ pfu, between about 10⁸-10¹³ pfu, between about 10⁹-10¹² pfu, between about 10⁸-10¹² pfu, between about 10⁷-10¹² pfu, between about 10⁶-10¹² pfu, between about 10⁶-10⁹ pfu, between about 10⁶-10⁸ pfu, between about 10⁷-10¹⁰ pfu, between about 10⁷-10⁹ pfu, between about 10⁸-10¹⁰ pfu, or between about 10⁸-10⁹ pfu Preferably, the virus is administered at a dosage of at least 10⁷ pfu, between 10⁷ and 10¹⁰ pfu, between 10⁷-10⁹ pfu, between 10⁷-10⁸ pfu, between 10⁸-10¹⁰ pfu, between 10⁸-10⁹ pfu or between 10⁹ and 10¹⁰ pfu.

It is contemplated that a single dose of virus refers to the amount administered to a subject or a tumor over a 0.1, 0.5, 1, 2, 5, 10, 15, 20, or 24 hour period, including all values there between. The dose may be spread over time or by separate injection. Typically, multiple doses are administered to the same general target region, such as in the proximity of a tumor. In certain aspects, the viral dose is delivered by injection apparatus comprising a syringe or single port needle or multiple ports in a single needle or multiple prongs coupled to a syringe, or a combination thereof. A single dose of the vaccinia virus may be administered or the multiple doses may be administered over a treatment period which may comprise 1, 2, 3, 4, 5, 6, 7, 8, 9, 10, 11, 12 or more weeks. For example, the vaccinia virus may be administered every other day, weekly, every other week, every third week for a period of 1, 2, 3, 4, 5, 6 or more months.

Vaccinia virus may be propagated using the methods described by Earl and Moss in Ausubel *et al.,* 1994 or the methods described in WIPO Publication No. WO2013/022764, both of which are incorporated herein by reference.

### III. IMMUNE CHECKPOINT INHIBITORS (ICIs)

Immune checkpoint proteins interact with specific ligands which send a signal into T-cells that inhibits T-cell function. Cancer cells exploit this by driving high level expression of checkpoint proteins on their surface thereby suppressing the anti-cancer immune response.

An immune checkpoint inhibitor (also referred to as an ICI) for use in the pharmaceutical combination herein described is any compound capable of inhibiting the function of an immune checkpoint protein. Inhibition includes reduction of function as well as full blockade. In particular, the immune checkpoint protein is a human checkpoint protein. Thus, the immune checkpoint inhibitor is preferably an inhibitor of a human immune checkpoint.

Checkpoint proteins include, without limitation, CTLA-4, PD-1 (and its ligands PD-L1 and PD-L2), B7-H3, B7-H4, HVEM, TIM3, GAL9, LAG3, VISTA, KIR, BTLA, TIGIT and/or IDO. The pathways involving LAG3, BTLA, B7-H3, B7-H4, TIM3 and KIR are recognized in the art to constitute immune checkpoint pathways similar to the CTLA-4 and PD-1 dependent pathways (see *e*.*g*., Pardoll, 2012, Nature Rev Cancer 12:252-264; Mellman et al., 2011, Nature 480:480-489). In some embodiments, the immune checkpoint inhibitor is an inhibitor of CTLA-4, PD-1(and its ligands PD-L1 and PD-L2), B7-H3, B7-H4, HVEM, TIM3, GAL9, LAG3, VISTA, KIR, BTLA, TIGIT and/or IDO. In some embodiments, the immune checkpoint inhibitor is an inhibitor of PD-1, PD-L1, CTLA-4, LAG3, TIGIT, and/or TIM3. In some embodiments, the immune checkpoint inhibitor is an inhibitor of PD-1. In some embodiments, the immune checkpoint inhibitor is an inhibitor of PD-L1. In some embodiments, the immune checkpoint inhibitor is an inhibitor of CTLA-4. In some embodiments, the immune checkpoint inhibitor is an inhibitor of TIGIT. In some embodiments, the immune checkpoint inhibitor is an inhibitor of LAG3. In some embodiments, the immune checkpoint inhibitor is an inhibitor of TIM3.

In some embodiments, the immune checkpoint inhibitor of the combination is an antibody. The term "antibody" as used herein encompasses naturally occurring and engineered antibodies as well as full length antibodies or functional fragments or analogs thereof that are capable of binding *e.g.* the target immune checkpoint or epitope *(e.g.* retaining the antigen-binding portion). The antibody for use according to the methods described herein may be from any origin including, without limitation, human, humanized, animal or chimeric and may be of any isotype with a preference for an IgG1 or IgG4 isotype and further may be glycosylated or non-glycosylated. The term antibody also includes bispecific or multispecific antibodies so long as the antibody(s) exhibit the binding specificity herein described.

Humanized antibodies refer to non-human (*e*.*g*. murine, rat, etc.) antibody whose protein sequence has been modified to increase similarity to a human antibody. Chimeric antibodies refer to antibodies comprising one or more element(s) of one species and one or more element(s) of another specifies, for example a non-human antibody comprising at least a portion of a constant region (Fc) of a human immunoglobulin.

Many forms of antibody can be engineered for use in the combination of the invention, representative examples of which include an Fab fragment (monovalent fragment consisting of the VL, VH, CL and CH1 domains), an F(ab')2 fragment (bivalent fragment comprising two Fab fragments linked by at least one disulfide bridge at the hinge region), a Fd fragment (consisting of the VH and CH1 domains), a Fv fragment (consisting of the VL and VH domains of a single arm of an antibody), a dAb fragment (consisting of a single variable domain fragment (VH or VL domain), a single chain Fv (scFv) comprising the two domains of a Fv fragment, VL and VH, that are fused together, eventually with a linker to make a single protein chain.

In some embodiments, immune checkpoint protein inhibitors (also referred to as ICIs) of the combination therapy are antibodies or fragments thereof that specifically bind to an immune checkpoint protein selected from the group consisting of: CTLA4, PD-1, PD-L1, PD-L2, B7-H3, B7-H4, TIM3, GAL9, LAG3, VISTA, KIR, BTLA and TIGIT. In particularly preferred embodiments, the immune checkpoint inhibitor is a monoclonal antibody, a fully human antibody, a chimeric antibody, a humanized antibody or fragment thereof that capable of at least partly antagonizing CTLA4, PD-1, PD-L1, PD-L2, TIM3, LAG3 or TIGIT. In some embodiments, the immune checkpoint protein inhibitor of the combination therapy is an antibody or fragment thereof that specifically binds to CTLA4. In some embodiments, the immune checkpoint protein inhibitor of the combination therapy is an antibody or fragment thereof that specifically binds to PD-1. In some embodiments, the immune checkpoint protein inhibitor of the combination therapy is an antibody or fragment thereof that specifically binds to PD-L1. In some embodiments, the immune checkpoint protein inhibitor of the combination therapy is an antibody or fragment thereof that specifically binds to PD-L2. In some embodiments, the immune checkpoint protein inhibitor of the combination therapy is an antibody or fragment thereof that specifically binds to B7-H3. In some embodiments, the immune checkpoint protein inhibitor of the combination therapy is an antibody or fragment thereof that specifically binds to B7-H4. In some embodiments, the immune checkpoint protein inhibitor of the combination therapy is an antibody or fragment thereof that specifically binds to TIM3. In some embodiments, the immune checkpoint protein inhibitor of the combination therapy is an antibody or fragment thereof that specifically binds to GAL9. In some embodiments, the immune checkpoint protein inhibitor of the combination therapy is an antibody or fragment thereof that specifically binds to LAG3. In some embodiments, the immune checkpoint protein inhibitor of the combination therapy is an antibody or fragment thereof that specifically binds to VISTA. In some embodiments, the immune checkpoint protein inhibitor of the combination therapy is an antibody or fragment thereof that specifically binds to KIR. In some embodiments, the immune checkpoint protein inhibitor of the combination therapy is an antibody or fragment thereof that specifically binds to BTLA. In some embodiments, the immune checkpoint protein inhibitor of the combination therapy is an antibody or fragment thereof that specifically binds to TIGIT.

In some embodiments, the pharmaceutical combination comprises a Western Reserve, Wyeth, Lister or Copenhagen vaccinia virus strain and a CTLA-4 inhibitor, preferably a monoclonal antibody that specifically binds to (and inhibits) CTLA-4. The complete human CTLA-4 nucleic acid sequence can be found under GenBank Accession No. LI 5006. Monoclonal antibodies that specifically bind to CTLA4 include, without limitation, Ipilimumab (Yervoy^{®}; BMS) and Tremelimumab (AstraZeneca/MedImmune), as well as antibodies disclosed in U.S. Patent Application Publication Nos. 2005/0201994, 2002/0039581, and 2002/086014, the contents of each of which are incorporated herein by reference, and antibodies disclosed in U.S. Patent Nos. 5,811,097, 5,855,887, 6,051,227, 6,984,720, 6,682,736, 6,207,156, 5,977,318, 6,682,736, 7,109,003,7,132,281, and 8,491,895 the contents of each of which are incorporated herein by reference, or an antibody comprising the heavy and light chain variable regions of any of these antibodies.

In some embodiments, the pharmaceutical combination comprises a Western Reserve, Wyeth, Lister or Copenhagen vaccinia virus strain and a PD-1 inhibitor, preferably a monoclonal antibody that specifically binds to (and inhibits) PD-1. The complete nucleotide and amino acid sequences of human PD-1 can be found under GenBank Accession No. U64863 and NP_005009.2. Monoclonal antibodies against PD-1 include, without limitation, lambrolizumab (*e*.*g*. disclosed as hPD109A and its humanized derivatives h409A11, h409A16 and h409A17 in U.S. Patent No. 8,354,509, incorporated herein by reference), Nivolumab (Opdivo^{®}; Bristol-Myers Squibb; code name BMS-936558) disclosed in U.S. Patent No. 8,008,449, incorporated herein by reference, Pembrolizumab (Keytruda^{®}) and Pidilizumab (CT-011; disclosed in Rosenblatt et al., Immunother. 34:409-418 (2011)) or an antibody comprising the heavy and light chain regions of these antibodies. Other anti-PD-1 antibodies are described in *e*.*g*. WO2004/004771, WO2004/056875, WO2006/121168, WO2008/156712, WO2009/014708, WO2009/114335, WO2013/043569 and WO2014/047350. In a related embodiment, the checkpoint inhibitor of the pharmaceutical combination is an anti-PD-1 fusion protein such as AMP-224 (composed of the extracellular domain of PD-L2 and the Fc region of human IgG1).

In some embodiments, the pharmaceutical combination comprises a Western Reserve, Wyeth, Lister or Copenhagen vaccinia virus strain and a PD-L1 inhibitor, preferably a monoclonal antibody that specifically binds to (and inhibits) PD-L1. Monoclonal antibodies against PD-L1 include, without limitation, pembrolizumab (MK-3475, disclosed in WO2009/114335)), BMS-936559 (MDX-1105), Atezolizumab (Genentech/Roche; MPDL33280A) disclosed in U.S. Patent NO. 8,217,149, the contents of which are incorporated herein by reference, Durvalumab (AstraZeneca/MedImmune; MEDI4736) disclosed in U.S. Patent No. 8,779,108, incorporated herein by reference, MIH1 (Affymetrix obtainable via eBioscience (16.5983.82)) and Avelumab (MSB0010718C; Merck KGaA) or an antibody comprising the heavy and light chain variable regions of any of these antibodies. In a related embodiment, the immune checkpoint inhibitor is an anti-PD-L1 fusion protein such as the PD-L2-Fc fusion protein known as AMP-224 (disclosed in Mkritchyan M., et al., J. Immunol., 189:2338-47 (2010).

In some embodiments, the pharmaceutical combination comprises a Western Reserve, Wyeth, Lister or Copenhagen vaccinia virus strain and a PD-L2 inhibitor such as MIH18 (described in Pfistershammer et al., Eur J Immunol. 36:1104-1113 (2006).

In some embodiments, the pharmaceutical combination comprises a Western Reserve, Wyeth, Lister or Copenhagen vaccinia virus strain and a LAG3 inhibitor such as soluble LAG3 (IMP321, or LAG3-Ig disclosed in U.S. Patent Application Publication No. 2011-0008331, incorporated herein by reference, and in Brignon et al., Clin. Cancer Res. 15:6225-6231 (2009)), IMP701 or other humanized antibodies blocking human LAG3 described in U.S. Patent Application Publication No. 2010-0233183, incorporated herein by reference, U.S. Patent No. 5,773,578, incorporated herein by reference, or BMS-986016 or other fully human antibodies blocking LAG3 described in U.S. Patent Application Publication No. 2011-0150892, incorporated herein by reference.

In some embodiments, the pharmaceutical combination comprises a Western Reserve, Wyeth, Lister or Copenhagen vaccinia virus strain and a BLTA inhibitor such as the antibody 4C7 disclosed in U.S. Patent No. 8,563,694, incorporated herein by reference.

In some embodiments, the pharmaceutical combination comprises a Western Reserve, Wyeth, Lister or Copenhagen vaccinia virus strain and a B7H4 checkpoint inhibitor such as an antibody as disclosed in U.S. Patent Application Publication No. 2014/0294861, incorporated herein by reference or a soluble recombinant form of B7H4 *e.g.* as disclosed in U.S. Patent Application Publication No. 20120177645, incorporated herein by reference.

In some embodiments, the pharmaceutical combination comprises a Western Reserve, Wyeth, Lister or Copenhagen vaccinia virus strain and a B7-H3 checkpoint inhibitor such as the antibody MGA271 disclosed as BRCA84D or a derivative as disclosed in U.S. Patent Application Publication No. 20120294796, incorporated herein by reference.

In some embodiments, the pharmaceutical combination comprises a Western Reserve, Wyeth, Lister or Copenhagen vaccinia virus strain and a TIM3 checkpoint inhibitor such as an antibody as disclosed in U.S. Patent No. 8,841,418, incorporated herein by reference or the anti-human TIM3 blocking antibody F38-2E2 disclosed by Jones et al., J. Exp. Med., 205(12):2763-79 (2008).

In some embodiments, the pharmaceutical combination comprises a Western Reserve, Wyeth, Lister or Copenhagen vaccinia virus strain and a KIR checkpoint inhibitor such as the antibody lirilumab (described in Romagne et al., Blood, 114(13):2667-2677 (2009)).

In some embodiments, the pharmaceutical combination comprises a Western Reserve, Wyeth, Lister or Copenhagen vaccinia virus strain and a TIGIT inhibitor. TIGIT checkpoint inhibitors preferably inhibit interaction of TIGIT with poliovirus receptor (CD15 5) and include, without limitation, antibodies targeting human TIGIT, such as those disclosed in U.S. Patent No. 9,499,596 (incorporated herein by reference) and U.S. Patent Application Publication Nos. 20160355589, 20160176963 (incorporated herein by reference) and poliovirus receptor variants such as those disclosed in U.S. Patent No. 9,327,014 (incorporated herein by reference).

In some embodiments, the pharmaceutical combination comprises a Western Reserve, Wyeth, Lister or Copenhagen vaccinia virus strain and an IDO inhibitor. IDO is recognized as an immune checkpoint protein its expression in tumor cells contributes to immune tolerance by shutting down effector T-cells. IDO is thought to contribute to resistance of anti-CLTA-4 therapies. Inhibitors of IDO for use according to the methods described herein include, without limitation, tryptophan mimetics such as D-1MT (D isoform of 1-methyl-DL-tryptophan (MT)), L-1MT (L isoform of MT), MTH-Trp (methylthiohydantoin-dl-tryptophan; transcriptional suppressor of IDO), and β-carbolines, indole mimetics such as napthoquinone-based agents, S-allyl-brassinin, S-benzyl-brassinin, 5-Bromo-brassinin, as well as phenylimidazole-based agents, 4-phenylimidazole, exiguamine A, epacadostat, rosmarinic acid, norharmane and NSC401366. Preferred IDO inhibitors include INCB 024360 (epacadostat; 1,2,5-Oxadiazole-3-carboximidamide, 4-((2-((Aminosulfonyl)amino)ethyl)amino)-N-(3-bromo-4-fluorophenyl)-N'-hydroxy-, (C(Z))- ; Incyte), indoximod (NLG2101; D-1MT; NewLink Genetics), IDO peptide vaccine (Copenhagen University) and NLG919 (NewLink Genetics).

In some embodiments, the pharmaceutical combination comprises a Western Reserve, Wyeth, Lister or Copenhagen vaccinia virus strain, a PD-1 inhibitor, preferably a monoclonal antibody that specifically binds to (and inhibits) PD-1, and a CTLA-4 inhibitor, preferably a monoclonal antibody that specifically binds to (and inhibits) CTLA-4. The complete nucleotide and amino acid sequences of human PD-1 can be found under GenBank Accession No. U64863 and NP_005009.2. Monoclonal antibodies against PD-1 include, without limitation, lambrolizumab (*e*.*g*., disclosed as hPD109A and its humanized derivatives h409A11, h409A16 and h409A17 in U.S. Patent No. 8,354,509, incorporated herein by reference), Nivolumab (Opdivo^{®}; Bristol-Myers Squibb; code name BMS-936558) disclosed in U.S. Patent No. 8,008,449, incorporated herein by reference, Pembrolizumab (Keytruda^{®}) and Pidilizumab (CT-011; disclosed in Rosenblatt et al., Immunother. 34:409-418 (2011)) or an antibody comprising the heavy and light chain regions of these antibodies. Other anti-PD-1 antibodies are described in *e.g.* WO2004/004771, WO2004/056875, WO2006/121168, WO2008/156712, WO2009/014708, WO2009/114335, WO2013/043569 and WO2014/047350. In a related embodiment, the checkpoint inhibitor of the pharmaceutical combination is an anti-PD-1 fusion protein such as AMP-224 (composed of the extracellular domain of PD-L2 and the Fc region of human IgG1). The complete human CTLA-4 nucleic acid sequence can be found under GenBank Accession No. LI 5006. Monoclonal antibodies that specifically bind to CTLA4 include, without limitation, Ipilimumab (Yervoy^{®}; BMS) and Tremelimumab (AstraZeneca/MedImmune), as well as antibodies disclosed in U.S. Patent Application Publication Nos. 2005/0201994, 2002/0039581, and 2002/086014, the contents of each of which are incorporated herein by reference, and antibodies disclosed in U.S. Patent Nos. 5,811,097, 5,855,887, 6,051,227, 6,984,720, 6,682,736, 6,207,156, 5,977,318, 6,682,736, 7,109,003,7,132,281, and 8,491,895 the contents of each of which are incorporated herein by reference, or an antibody comprising the heavy and light chain variable regions of any of these antibodies.

In some embodiments, the pharmaceutical combination comprises a Western Reserve, Wyeth, Lister or Copenhagen vaccinia virus strain, a PD-L1 inhibitor, preferably a monoclonal antibody that specifically binds to (and inhibits) PD-L1, and a CTLA-4 inhibitor, preferably a monoclonal antibody that specifically binds to (and inhibits) CTLA-4. Monoclonal antibodies against PD-L1 include, without limitation, pembrolizumab (MK-3475, disclosed in WO2009/114335)), BMS-936559 (MDX-1105), Atezolizumab (Genentech/Roche; MPDL33280A) disclosed in U.S. Patent NO. 8,217,149, the contents of which are incorporated herein by reference, Durvalumab (AstraZeneca/MedImmune; MEDI4736) disclosed in U.S. Patent No. 8,779,108, incorporated herein by reference, MIH1 (Affymetrix obtainable via eBioscience (16.5983.82)) and Avelumab (MSB0010718C; Merck KGaA) or an antibody comprising the heavy and light chain variable regions of any of these antibodies. In a related embodiment, the immune checkpoint inhibitor is an anti-PD-L1 fusion protein such as the PD-L2-Fc fusion protein known as AMP-224 (disclosed in Mkritchyan M., et al., J. Immunol., 189:2338-47 (2010). The complete human CTLA-4 nucleic acid sequence can be found under GenBank Accession No. LI 5006. Monoclonal antibodies that specifically bind to CTLA4 include, without limitation, Ipilimumab (Yervoy^{®}; BMS) and Tremelimumab (AstraZeneca/MedImmune), as well as antibodies disclosed in U.S. Patent Application Publication Nos. 2005/0201994, 2002/0039581, and 2002/086014, the contents of each of which are incorporated herein by reference, and antibodies disclosed in U.S. Patent Nos. 5,811,097, 5,855,887, 6,051,227, 6,984,720, 6,682,736, 6,207,156, 5,977,318, 6,682,736, 7,109,003,7,132,281, and 8,491,895 the contents of each of which are incorporated herein by reference, or an antibody comprising the heavy and light chain variable regions of any of these antibodies.

In some embodiments, the pharmaceutical combination comprises a Western Reserve, Wyeth, Lister or Copenhagen vaccinia virus strain, a PD-1 inhibitor, preferably a monoclonal antibody that specifically binds to (and inhibits) PD-1, and a LAG3 inhibitor such as soluble LAG3 (IMP321, or LAG3-Ig disclosed in U.S. Patent Application Publication No. 2011-0008331, incorporated herein by reference, and in Brignon et al., Clin. Cancer Res. 15:6225-6231 (2009)), IMP701 or other humanized antibodies blocking human LAG3 described in U.S. Patent Application Publication No. 2010-0233183, incorporated herein by reference, U.S. Patent No. 5,773,578, incorporated herein by reference, or BMS-986016 or other fully human antibodies blocking LAG3 described in U.S. Patent Application Publication No. 2011-0150892, incorporated herein by reference. The complete nucleotide and amino acid sequences of human PD-1 can be found under GenBank Accession No. U64863 and NP_005009.2. Monoclonal antibodies against PD-1 include, without limitation, lambrolizumab (*e*.*g*. disclosed as hPD109A and its humanized derivatives h409A11, h409A16 and h409A17 in U.S. Patent No. 8,354,509, incorporated herein by reference), Nivolumab (Opdivo^{®}; Bristol-Myers Squibb; code name BMS-936558) disclosed in U.S. Patent No. 8,008,449, incorporated herein by reference, Pembrolizumab (Keytruda^{®}) and Pidilizumab (CT-011; disclosed in Rosenblatt et al., Immunother. 34:409-418 (2011)) or an antibody comprising the heavy and light chain regions of these antibodies. Other anti-PD-1 antibodies are described in *e*.*g*. WO2004/004771, WO2004/056875, WO2006/121168, WO2008/156712, WO2009/014708, WO2009/114335, WO2013/043569 and WO2014/047350. In a related embodiment, the checkpoint inhibitor of the pharmaceutical combination is an anti-PD-1 fusion protein such as AMP-224 (composed of the extracellular domain of PD-L2 and the Fc region of human IgG1).

In some embodiments, the pharmaceutical combination comprises a Western Reserve, Wyeth, Lister or Copenhagen vaccinia virus strain, a PD-L1 inhibitor, preferably a monoclonal antibody that specifically binds to (and inhibits) PD-L1, and a LAG3 inhibitor such as soluble LAG3 (IMP321, or LAG3-Ig disclosed in U.S. Patent Application Publication No. 2011-0008331, incorporated herein by reference, and in Brignon et al., Clin. Cancer Res. 15:6225-6231 (2009)), IMP701 or other humanized antibodies blocking human LAG3 described in U.S. Patent Application Publication No. 2010-0233183, incorporated herein by reference, U.S. Patent No. 5,773,578, incorporated herein by reference, or BMS-986016 or other fully human antibodies blocking LAG3 described in U.S. Patent Application Publication No. 2011-0150892, incorporated herein by reference. Monoclonal antibodies against PD-L1 include, without limitation, pembrolizumab (MK-3475, disclosed in WO2009/114335)), BMS-936559 (MDX-1105), Atezolizumab (Genentech/Roche; MPDL33280A) disclosed in U.S. Patent NO. 8,217,149, the contents of which are incorporated herein by reference, Durvalumab (AstraZeneca/MedImmune; MEDI4736) disclosed in U.S. Patent No. 8,779,108, incorporated herein by reference, MIH1 (Affymetrix obtainable via eBioscience (16.5983.82)) and Avelumab (MSB0010718C; Merck KGaA) or an antibody comprising the heavy and light chain variable regions of any of these antibodies. In a related embodiment, the immune checkpoint inhibitor is an anti-PD-L1 fusion protein such as the PD-L2-Fc fusion protein known as AMP-224 (disclosed in Mkritchyan M., et al., J. Immunol., 189:2338-47 (2010).

In some embodiments, the pharmaceutical combination comprises a Western Reserve, Wyeth, Lister or Copenhagen vaccinia virus strain, a PD-1 inhibitor, preferably a monoclonal antibody that specifically binds to (and inhibits) PD-1, and a TIM3 checkpoint inhibitor such as an antibody as disclosed in U.S. Patent No. 8,841,418, incorporated herein by reference or the anti-human TIM3 blocking antibody F38-2E2 disclosed by Jones et al., J. Exp. Med., 205(12):2763-79 (2008). The complete nucleotide and amino acid sequences of human PD-1 can be found under GenBank Accession No. U64863 and NP_005009.2. Monoclonal antibodies against PD-1 include, without limitation, lambrolizumab (*e*.*g*. disclosed as hPD109A and its humanized derivatives h409A11, h409A16 and h409A17 in U.S. Patent No. 8,354,509, incorporated herein by reference), Nivolumab (Opdivo^{®}; Bristol-Myers Squibb; code name BMS-936558) disclosed in U.S. Patent No. 8,008,449, incorporated herein by reference, Pembrolizumab (Keytruda^{®}) and Pidilizumab (CT-011; disclosed in Rosenblatt et al., Immunother. 34:409-418 (2011)) or an antibody comprising the heavy and light chain regions of these antibodies. Other anti-PD-1 antibodies are described in *e*.*g*. WO2004/004771, WO2004/056875, WO2006/121168, WO2008/156712, WO2009/014708, WO2009/114335, WO2013/043569 and WO2014/047350. In a related embodiment, the checkpoint inhibitor of the pharmaceutical combination is an anti-PD-1 fusion protein such as AMP-224 (composed of the extracellular domain of PD-L2 and the Fc region of human IgG1).

In some embodiments, the pharmaceutical combination comprises a Western Reserve, Wyeth, Lister or Copenhagen vaccinia virus strain, a PD-L1 inhibitor, preferably a monoclonal antibody that specifically binds to (and inhibits) PD-L1, and a TIM3 checkpoint inhibitor such as an antibody as disclosed in U.S. Patent No. 8,841,418, incorporated herein by reference or the anti-human TIM3 blocking antibody F38-2E2 disclosed by Jones et al., J. Exp. Med., 205(12):2763-79 (2008). Monoclonal antibodies against PD-L1 include, without limitation, pembrolizumab (MK-3475, disclosed in WO2009/114335)), BMS-936559 (MDX-1105), Atezolizumab (Genentech/Roche; MPDL33280A) disclosed in U.S. Patent NO. 8,217,149, the contents of which are incorporated herein by reference, Durvalumab (AstraZeneca/MedImmune; MEDI4736) disclosed in U.S. Patent No. 8,779,108, incorporated herein by reference, MIH1 (Affymetrix obtainable via eBioscience (16.5983.82)) and Avelumab (MSB0010718C; Merck KGaA) or an antibody comprising the heavy and light chain variable regions of any of these antibodies. In a related embodiment, the immune checkpoint inhibitor is an anti-PD-L1 fusion protein such as the PD-L2-Fc fusion protein known as AMP-224 (disclosed in Mkritchyan M., et al., J. Immunol., 189:2338-47 (2010).

In some embodiments, the pharmaceutical combination comprises a Western Reserve, Wyeth, Lister or Copenhagen vaccinia virus strain and a TIGIT inhibitor. TIGIT checkpoint inhibitors preferably inhibit interaction of TIGIT with poliovirus receptor (CD155) and include, without limitation, antibodies targeting human TIGIT, such as those disclosed in U.S. Patent No. 9,499,596 (incorporated herein by reference) and U.S. Patent Application Publication Nos. 20160355589, 20160176963 (incorporated herein by reference) and poliovirus receptor variants such as those disclosed in U.S. Patent No. 9,327,014 (incorporated herein by reference).

In some embodiments, the pharmaceutical combination comprises a Western Reserve, Wyeth, Lister or Copenhagen vaccinia virus strain and an IDO inhibitor (Indoleamine-pyrrole 2,3-dioxygenase. IDO inhibitors include metabolic, inhibitors preferably inhibit metabolic pathways and include, without limitation, Norharmane, (see, Chiarugi A, et al., "Combined inhibition of indoleamine 2,3-dioxygenase and nitric oxide synthase modulates neurotoxin release by interferon-gamma-activated macrophages", Journal of Leukocyte Biology. 68 (2): 260-6. (2000)), rosmarinic acid (see, Lee HJ, et al., "Rosmarinic acid inhibits indoleamine 2,3-dioxygenase expression in murine dendritic cells", Biochemical Pharmacology. 73 (9): 1412-21 (2007)), COX-2 inhibitors (see, Cesario A, et al., "The interplay between indoleamine 2,3-dioxygenase 1 (IDO1) and cyclooxygenase (COX)-2 in chronic inflammation and cancer", Current Medicinal Chemistry. 18 (15): 2263-71 (2011)), 1-methyltryptophan (Hou DY, et al., "Inhibition of indoleamine 2,3-dioxygenase in dendritic cells by stereoisomers of 1-methyl-tryptophan correlates with antitumor responses". Cancer Research. 67 (2): 792-801 (2007) and Chauhan N, et al., (April 2009). "Reassessment of the reaction mechanism in the heme dioxygenases". Journal of the American Chemical Society. 131 (12): 4186-7 (2009)), including for example, the specific racemer 1-methyl-D-tryptophan (known as indoximod) (a clinical trial candidate), Epacadostat (INCB24360), navoximod (GDC-0919) (see, Jochems C, et al., "The IDO1 selective inhibitor epacadostat enhances dendritic cell immunogenicity and lytic ability of tumor antigen-specific T cells", Oncotarget. 7 (25): 37762-37772. (2016)), and or BMS-986205. In some embodiments, the IDO inhibitor is selected from the group consisting of Norharmane, rosmarinic acid, COX-2 inhibitors, 1-methyltryptophan, Indoximod, Epacadostat (INCB24360), navoximod (GDC-0919) and/or BMS-986205.

As the skilled person will know, alternative and/or equivalent names may be in use for certain antibodies mentioned above. Such alternative and/or equivalent names are interchangeable in the context of the present invention.

In some aspects, the pharmaceutical combination described herein includes (i) more than one immune checkpoint inhibitor and (ii) a replicative oncolytic vaccinia virus. In a preferred embodiment, a PD-1 inhibitor and a CTLA-4 inhibitor are concurrently administered with the vaccinia virus. Other examples include, without limitation, concurrent administration of a LAG3 inhibitor and a PD-1 inhibitor with the vaccinia virus, or concurrent administration of a LAG3 inhibitor and a PD-L1 inhibitor. Other examples include concurrent administration of an IDO inhibitor and a CTLA-4 inhibitor and/or PD-1 inhibitor. In some embodiments, the IDO inhibitor is selected from the group consisting of Norharmane, rosmarinic acid, COX-2 inhibitors, 1-methyltryptophan, Indoximod, Epacadostat (INCB24360), navoximod (GDC-0919) and/or BMS-986205.

### IV. CYTOKINES

In some embodiments, the replicative oncolytic vaccinia virus of the pharmaceutical combination comprises heterologous sequence encoding a cytokine, wherein the cytokine is expressed by the virus.

In some embodiments, a replicative oncolytic vaccinia virus is provided that is engineered to express an a cytokine selected from the group consisting of granulocyte-macrophage colony stimulating factor (GM-CSF), interleukin-2 (IL-2), interleukin-4 (IL-4), interleukin-5 (IL-5), interleukin-7 (IL-7), interleukin-12 (IL-12), interleukin-15 (IL-15), interleukin-18 (IL-18), interleukin-21 (IL-21), interleukin-24 (IL-24), interferon-y (IFN-y), and tumor necrosis factor-α (TNF-α). In particularly preferred embodiments, the replicative oncolytic vaccinia virus is a Wyeth, Western Reserve, Copenhagen or Lister strain. In some embodiments, a replicative oncolytic vaccinia virus is engineered to express GM-CSF. In some embodiments, a replicative oncolytic vaccinia virus is engineered to express interleukin-2 (IL-2). In some embodiments, a replicative oncolytic vaccinia virus is engineered to express interleukin-4 (IL-4). In some embodiments, a replicative oncolytic vaccinia virus is engineered to express interleukin-5 (IL-5). In some embodiments, a replicative oncolytic vaccinia virus is engineered to express interleukin-7 (IL-7). In some embodiments, a replicative oncolytic vaccinia virus is engineered to express interleukin-12 (IL-12). In some embodiments, a replicative oncolytic vaccinia virus is engineered to express interleukin-15 (IL-15). In some embodiments, a replicative oncolytic vaccinia virus is engineered to express interleukin-18 (IL-18). In some embodiments, a replicative oncolytic vaccinia virus is engineered to express interleukin-21 (IL-21). In some embodiments, a replicative oncolytic vaccinia virus is engineered to express interleukin-24 (IL-24), interferon-γ (IFN-γ). In some embodiments, a replicative oncolytic vaccinia virus is engineered to express tumor necrosis factor-α (TNF-α). In some embodiments, a replicative oncolytic vaccinia virus is mJX594, which is engineered to express GM-CSF.

### V. TUMOR ANTIGENS

In several embodiments, the replicative oncolytic vaccinia virus comprises heterologous nucleic acid sequence encoding a tumor antigen and optionally a cytokine, wherein the tumor antigen and optionally the cytokine are expressed in a cell infected with the virus, preferably a tumor cell. Tumor antigens encompass tumor-specific antigens and tumor-associated antigens. The replication-competent oncolytic vaccinia virus may express the full length tumor antigen or an immunogenic peptide thereof. In some embodiments, the cytokine is expressed in a cell infected with the replicative oncolytic vaccinia virus. In some embodiments, the replicative oncolytic vaccinia virus comprises a heterologous nucleic acid sequence encoding a tumor antigen and a cytokine, wherein the tumor antigen and the cytokine are expressed in a cell infected with the replicative oncolytic vaccinia virus. In some embodiments, the cell is a tumor cell.

In some embodiments, tumor antigens can include, without limitation, MAGE-1, MAGE-3, BAGE, GAGE-1, GAGE-2, N-acetylglucosaminyltransferase-V, p-15, gp100, MART-1/MelanA, TRP-1 (gp75), TRP-2, Tyrosinase, cyclin-dependent kinase 4, β-catenin, MUM-1, CDK4, HER-2/neu, human papillomavirus-E6, human papillomavirus E7, CD20, carcinoembryonic antigen (CEA), epidermal growth factor receptor, MUC-1, caspase-8, CD5, mucin-1, Lewisx, CA-125, p185HER2, IL-2R, Fap-α, tenascin, antigens associated with a metalloproteinase, CAMPATH-1, RCC: Regulator of G-protein signaling 5 (RGS5), Surivin (BIRC5=baculoviral inhibitor of apoptosis repeat-containg 5), Insulin-like growth factor-binding protein 3 (IGF-BP3), thymidylate synthetase (TYMS), hypoxia-inducible protein 2 = hypoxial inducible lipid droplet associated (HIG2), matrix metallopeptidase 7 (MMP7), prune homolog 2 (PRUNE2), RecQ protein-like (DNA helicase Q1-like) (RECQL); leptin receptor (LEPR); ERBB receptor feedback inhibitor 1 (ERRFI1); lysosomal protein transmembrane 4 alpha (LAPTM4A); RAB1B, RAS oncogene family (RAB1B); CD24; homo sapiens thymosin beta 4, X-linked (TMSB4X); homo sapiens S100 calcium binding protein A6 (S100A6); homo sapiens adenosine A2 receptor (ADORA2B); chromosome 16 open reading frame 61 (C16orf61); ROD1 regulator of differentiation 1 (ROD1); NAD-dependent deacetylase sirtuin-2 (SIR2L); tubulin alpha 1c (TUBA1C); ATPase inhibitory factor 1 (ATPIF1); stromal antigen 2 (STAG2); and nuclear casein kinase and cyclin-dependent substrate 1 (NUCKS1). In some embodiments, the antigen is one listed in U.S. Patent No. 9,919,047, incorporated herein by reference in its entirety). In some embodiments, the tumor antigen is a renal cell carcinoma tumor antigen. In some embodiments, the renal cell carcinoma tumor antigen is selected from the group consisting of regulator of G-protein signaling 5 (RGS5), Surivin (BIRC5=baculoviral inhibitor of apoptosis repeat-containg 5), Insulin-like growth factor-binding protein 3 (IGF-BP3), thymidylate synthetase (TYMS), hypoxia-inducible protein 2, hypoxial inducible lipid droplet associated (HIG2), matrix metallopeptidase 7 (MMP7), prune homolog 2 (PRUNE2), RecQ protein-like (DNA helicase Q1-like) (RECQL), leptin receptor (LEPR), ERBB receptor feedback inhibitor 1 (ERRFI1), lysosomal protein transmembrane 4 alpha (LAPTM4A); RAB1B, RAS oncogene family (RAB1B), CD24, homo sapiens thymosin beta 4, X-linked (TMSB4X), homo sapiens S100 calcium binding protein A6 (S100A6), homo sapiens adenosine A2 receptor (ADORA2B), chromosome 16 open reading frame 61 (C16orf61), ROD1 regulator of differentiation 1 (ROD1), NAD-dependent deacetylase sirtuin-2 (SIR2L), tubulin alpha 1c (TUBA1C), ATPase inhibitory factor 1 (ATPIF1), stromal antigen 2 (STAG2), and nuclear casein kinase and cyclin-dependent substrate 1 (NUCKS1).In some embodiments, the tumor antigens include, without limitation, KS 1/4 pan-carcinoma antigen, ovarian carcinoma antigen (CA125), prostatic acid phosphate, prostate specific antigen, melanoma-associated antigen p97, melanoma antigen gp75, high molecular weight melanoma antigen (HMW-MAA), prostate specific membrane antigen, CEA, polymorphic epithelial mucin antigen, milk fat globule antigen, colorectal tumor-associated antigens (such as: CEA, TAG-72, CO17-1A, GICA 19-9, CTA-1 and LEA), Burkitt's lymphoma antigen-38.13, CD19, B-lymphoma antigen-CD20, CD33, melanoma specific antigens (such as ganglioside GD2, ganglioside GD3, ganglioside GM2, ganglioside GM3), tumor-specific transplantation type of cell-surface antigen (TSTA) (such as virally-induced tumor antigens including T-antigen DNA tumor viruses and Envelope antigens of RNA tumor viruses), oncofetal antigen-alpha-fetoprotein such as CEA of colon, bladder tumor oncofetal antigen, differentiation antigen (such as human lung carcinoma antigen L6 and L20), antigens of fibrosarcoma, leukemia T-cell antigen-Gp37, neoglycoprotein, sphingolipids, breast cancer antigens (such as EGFR (Epidermal growth factor receptor), HER2 antigen (p185^{HER2}) and HER2 neu epitope), polymorphic epithelial mucin (PEM), malignant human lymphocyte antigen-APO-1, differentiation antigen (such as I antigen found in fetal erythrocytes, primary endoderm, I antigen found in adult erythrocytes, preimplantation embryos, I(Ma) found in gastric adenocarcinomas, M18, M39 found in breast epithelium, SSEA-1 found in myeloid cells, VEP8, VEP9, Myl, VIM-D5, D₁₅₆-22 found in colorectal cancer, TRA-1-85 (blood group H), C14 found in colonic adenocarcinoma, F3 found in lung adenocarcinoma, AH6 found in gastric cancer, Y hapten, Le^{y} found in embryonal carcinoma cells, TL5 (blood group A), EGF receptor found in A431 cells, E₁ series (blood group B) found in pancreatic cancer, FC10.2 found in embryonal carcinoma cells, gastric adenocarcinoma antigen, CO-514 (blood group Le^{a}) found in Adenocarcinoma, NS-10 found in adenocarcinomas, CO-43 (blood group Le^{b}), G49 found in EGF receptor of A431 cells, MH2 (blood group ALe^{b}/Le^{y}) found in colonic adenocarcinoma, 19.9 found in colon cancer, gastric cancer mucins, T₅A₇ found in myeloid cells, R₂₄ found in melanoma, 4.2, G_{D3}, D1.1, OFA-1, G_{M2}, OFA-2, G_{D2}, and M1:22:25:8 found in embryonal carcinoma cells, and SSEA-3 and SSEA-4 found in 4 to 8-cell stage embryos), T-cell receptor derived peptide from a Cutaneous T-cell Lymphoma, C-reactive protein (CRP), cancer antigen-50 (CA-50), cancer antigen 15-3 (CA15-3) associated with breast cancer, cancer antigen-19 (CA-19) and cancer antigen-242 associated with gastrointestinal cancers, carcinoma associated antigen (CAA), chromogranin A, epithelial mucin antigen (MC5), human epithelium specific antigen (E1A), Lewis(a)antigen, melanoma antigen, melanoma associated antigens 100, 25, and 150, mucin-like carcinoma-associated antigen, multidrug resistance related protein (MRPm6), multidrug resistance related protein (MRP41), Neu oncogene protein (C-erbB-2), neuron specific enolase (NSE), P-glycoprotein (mdr1 gene product), multi drug-resistance-related antigen, p170, multi drug-resistance-related antigen, prostate specific antigen (PSA), CD56, and NCAM.

In some embodiments, other tumor antigens include, without limitation, AIM2 (absent in melanoma 2), BMI1 (BMI1 polycomb ring finger oncogene), COX-2 (cyclooxygenase-2), EGFRvIII (epidermal growth factor receptor variant III), EZH2 (enhancer of zeste homolog 2), LICAM (human L1 cell adhesion molecule), Livin, Livinβ, MRP-3 (multidrug resistance protein 3), Nestin, OLIG2 (oligodendrocyte transcription factor), SOX2 (SRY-related HMG-box 2), ART1 (antigen recognized by T-cells 1), ART4 (antigen recognized by T-cells 4), SART1 (squamous cell carcinoma antigen recognized by T-cells 1), SART2, SART3, B-cyclin, Gli1 (glioma-associated oncogene homlog 1), Cav-1 (caveolin-1), cathepsin B, CD74 (cluster of Differentiation 74), E-cadherin (epithelial calcium-dependent adhesion), EphA2/Eck (EPH receptor A2/epithelial kinase), Fra-1/Fosl 1 (fos-related antigen 1), Ki67 (nuclear proliferation-associated antigen of antibody Ki67), Ku70/80 (human Ku heterodimer proteins subunits), IL-13Ra2 (interleukin-13 receptor subunit alpha-2), NY-ESO-1 (New York esophageal squamous cell carcinoma 1), PROX1 (prospero homeobox protein 1), PSCA (prostate stem cell antigen), SOX10 (SRY-related HMG-box 10), SOX11, Survivin, UPAR (urokinase-type plasminogen activator receptor, and WT-1 (Wilms' tumor protein 1).

### VI. TREATMENT REGIMENS AND PHARMACEUTICAL FORMULATIONS

The replicative oncolytic vaccinia virus and the immune checkpoint inhibitor of the pharmaceutical combination are administered simultaneously, wherein the oncolytic vaccinia virus is delivered by intratumoral administration. Simultaneous administration may, *e.g.,* take place in the form of one fixed combination comprising these agents, or by simultaneously administering each agent in independent formulations. In some embodiments, replicative oncolytic vaccinia virus and the PD-1 or PD-L1 immune checkpoint inhibitor of the pharmaceutical combination are administered simultaneously. In some embodiments, replicative oncolytic vaccinia virus and the CTLA-4 immune checkpoint inhibitor of the pharmaceutical combination are administered simultaneously. In some embodiments, replicative oncolytic vaccinia virus and the TIGIT immune checkpoint inhibitor of the pharmaceutical combination are administered simultaneously. In some embodiments, replicative oncolytic vaccinia virus, the PD-1 or PD-L1 immune checkpoint inhibitor, and the CTLA-4 immune checkpoint inhibitor of the pharmaceutical combination are administered simultaneously. In some embodiments, replicative oncolytic vaccinia virus, the PD-1 or PD-L1 immune checkpoint inhibitor, and the TIGIT immune checkpoint inhibitor of the pharmaceutical combination are administered simultaneously. In some embodiments, the replicative oncolytic vaccinia virus is administered by intratumoral, intravenous, intra-arterial, and/or intraperitoneal administration. In some embodiments, the replicative oncolytic vaccinia virus is administered by intratumoral administration. In some embodiments, the replicative oncolytic vaccinia virus is administered by intravenous administration. In some embodiments, the replicative oncolytic vaccinia virus is administered by intraperitoneal administration. In some embodiments, the replicative oncolytic vaccinia virus is administered by intra-arterial administration. In some embodiments, the replicative oncolytic vaccinia virus is administered only by intratumoral administration. In some embodiments, the immune checkpoint inhibitor is administered systemically. In some embodiments, the replicative oncolytic virus is administered intratumorally and the checkpoint inhibitor is administered systemically. In some embodiments, the replicative oncolytic virus is administered intravenously and the checkpoint inhibitor is administered systemically. In some embodiments, the replicative oncolytic virus is administered intraperitoneally and the checkpoint inhibitor is administered systemically. In some embodiments, the replicative oncolytic virus is administered intra-arterially and the checkpoint inhibitor is administered systemically. In some embodiments, the replicative oncolytic vaccinia virus comprises heterologous nucleic acid sequence encoding a tumor antigen and optionally a cytokine, wherein the tumor antigen and optionally the cytokine are expressed in a cell infected with the virus, preferably a tumor cell.

In some embodiments, the present invention provides a method of treating a tumor in a human comprising concurrently administering to the human a combination comprising (a) a replicative oncolytic vaccinia virus and (b) an inhibitor of the immune checkpoint protein. In some embodiments of the method of treatment, the immune checkpoint protein is selected from PD-1, PD-L1, CTLA-4, LAG3, TIM3, and TIGIT. In some embodiments of the method of treatment, the immune checkpoint protein is CTLA-4. In some embodiments of the method of treatment, the immune checkpoint protein is PD-L1. In some embodiments of the method of treatment, the immune checkpoint protein is LAG3. In some embodiments of the method of treatment, the immune checkpoint protein is TIGIT. In some embodiments of the method of treatment, the immune checkpoint protein is PD-1. In some embodiments of the method of treatment, the immune checkpoint protein is TIM3. In some embodiments of the method of treatment, the tumor is a solid cancer. In some embodiments of the method of treatment, the tumor is a colorectal cancer. In some embodiments of the method of treatment, the tumor is a renal cell carcinoma. In some embodiments, the replicative oncolytic vaccinia virus is administered by intratumoral, IV and/or intraperitoneal administration. In some embodiments, the replicative oncolytic vaccinia virus is administered by intratumoral administration. In some embodiments, the replicative oncolytic vaccinia virus is administered by IV administration. In some embodiments, the replicative oncolytic vaccinia virus is administered by intraperitoneal administration. In some embodiments, the replicative oncolytic vaccinia virus is administered by intra-arterial administration. In some embodiments, the replicative oncolytic vaccinia virus is administered only by intratumoral administration. In some embodiments, the immune checkpoint inhibitor is administered systemically. In some embodiments, the replicative oncolytic virus is administered intratumorally and the checkpoint inhibitor is administered systemically. In some embodiments, the replicative oncolytic virus is administered intravenously and the checkpoint inhibitor is administered systemically. In some embodiments, the replicative oncolytic virus is administered intraperitoneally and the checkpoint inhibitor is administered systemically. In some embodiments, the replicative oncolytic virus is administered intra-arterially and the checkpoint inhibitor is administered systemically. In some embodiments, the replicative oncolytic vaccinia virus comprises heterologous nucleic acid sequence encoding a tumor antigen and optionally a cytokine, wherein the tumor antigen and optionally the cytokine are expressed in a cell infected with the virus, preferably a tumor cell.

In some embodiments, the present invention provides a method of treating a tumor in a human comprising concurrently administering to the human a combination comprising (a) a replicative oncolytic vaccinia virus, (b) an inhibitor of PD-1 and/or PD-L1, and (c) an inhibitor of the immune checkpoint protein. In some embodiments of the method of treatment, the immune checkpoint protein is selected from CTLA-4, LAG3, TIM3, and TIGIT. In some embodiments of the method of treatment, the immune checkpoint protein is CTLA-4. In some embodiments of the method of treatment, the immune checkpoint protein is LAG3. In some embodiments of the method of treatment, the immune checkpoint protein is TIGIT. In some embodiments of the method of treatment, the immune checkpoint protein is TIM3. In some embodiments of the method of treatment, the tumor is a solid cancer. In some embodiments of the method of treatment, the tumor is a colorectal cancer. In some embodiments of the method of treatment, the tumor is a renal cell carcinoma. In some embodiments, the replicative oncolytic vaccinia virus is administered by intratumoral, IV and/or intraperitoneal administration. In some embodiments, the replicative oncolytic vaccinia virus is administered by intratumoral administration. In some embodiments, the replicative oncolytic vaccinia virus is administered by IV administration. In some embodiments, the replicative oncolytic vaccinia virus is administered by intraperitoneal administration. In some embodiments, the replicative oncolytic vaccinia virus is administered only by intratumoral administration. In some embodiments, the immune checkpoint inhibitor is administered systemically. In some embodiments, the replicative oncolytic virus is administered intratumorally and the checkpoint inhibitor is administered systemically. In some embodiments, the replicative oncolytic virus is administered intravenously and the checkpoint inhibitor is administered systemically. In some embodiments, the replicative oncolytic virus is administered intraperitoneally and the checkpoint inhibitor is administered systemically. In some embodiments, the replicative oncolytic virus is administered intra-arterially and the checkpoint inhibitor is administered systemically. In some embodiments, the replicative oncolytic vaccinia virus comprises heterologous nucleic acid sequence encoding a tumor antigen and optionally a cytokine, wherein the tumor antigen and optionally the cytokine are expressed in a cell infected with the virus, preferably a tumor cell.

In some embodiments of the method of treatment, the tumor does not express the immune checkpoint protein or expresses the immune checkpoint protein at a relatively low level prior to administering the replicative oncolytic vaccinia virus. In some embodiments, a high level is indicated by a tumor proportional score equal or greater than 50%. In some embdoiments, a high level is indicated by a tumor proportional score greater than 60%, greater than 70%, greater than 80%, greater than 90%, greater than 95%, about 99%, or about 100%. In some embodiments, a high level for a previously treated tumor is indicated by a tumor proportional score greater than 1%. In some embodiments, a high level is indicated by PD-L1 expressing tumor cells equal or greater than 50% *(e.g.,* more than 50% of the tumor cells express PD-L1). In some embdoiments, a high level is indicated by PD-L1 expressing tumor cells greater than 60%, greater than 70%, greater than 80%, greater than 90%, greater than 95%, about 99%, or about 100%. In some embodiments, a high level for a previously treated tumor is indicated by a PD-L1 expressing tumor cells greater than 1% (e.g., more than 1% of the tumor cells express PD-L1). In some embodiments, any PD-L1 diagnostic test can be employed to measure PD-L1 expression. In some embodiments, when the PD-L1 checkpoint is measured, the PD-L1 DaKo Companion Diagnostic test is employed to measure the PD-L1 level.

In some embodiments of the method of treatment, the method comprises a step of measuring the expression level of the checkpoint protein in the tumor prior to administering the combination.

Administration of the oncolytic vaccinia virus and the immune checkpoint inhibitor will follow general protocols for the administration of each particular therapy, taking into account the toxicity, if any, of the treatment. It is expected that the treatment cycles would be repeated as necessary. It also is contemplated that various standard therapies, as well as surgical intervention, may be applied in addition to combination therapy of the invention.

Treatment regimens may vary and often depend on tumor type, tumor location, disease progression, and health and age of the patient. Certain types of tumor will require more aggressive treatment, while at the same time, certain patients cannot tolerate more taxing protocols.

In certain embodiments, the tumor being treated may not, at least initially, be resectable. Treatment with a combination therapy of the invention may increase the resectability of the tumor due to shrinkage at the margins or by elimination of certain particularly invasive portions. Following treatment, resection may be possible. Additional treatments subsequent to resection will serve to eliminate microscopic residual disease at the tumor site

Determining a synergistic interaction between one or more components, the optimum range for the effect and absolute dose ranges of each component for the effect may be definitively measured by administration of the components over different w/w ratio ranges and doses to patients in need of treatment. For humans, the complexity and cost of carrying out clinical studies on patients renders impractical the use of this form of testing as a primary model for synergy. However, the observation of synergy in one species can be predictive of the effect in other species and animal models exist, as described herein, to measure a synergistic effect and the results of such studies can also be used to predict effective dose and plasma concentration ratio ranges and the absolute doses and plasma concentrations required in other species by the application of pharmacokinetic/pharmacodynamic methods. Established correlations between tumor models and effects seen in man suggest that synergy in animals may *e.g.* be demonstrated in a human xenograft tumor model.

In some embodiments, the combination is used to treat and/or prevent cancer in a mammal. In some embodiments, the cancer includes but is not limited to a brain cancer, head & neck cancer, esophageal cancer, skin cancer, lung cancer, thymic cancer, stomach cancer, colon cancer, liver cancer, ovarian cancer, uterine cancer, bladder cancer, renal cancer, testicular cancer, rectal cancer, breast cancer, and pancreatic cancer. In some embodiments, the cancer selected from the group consisting of brain cancer, head & neck cancer, esophageal cancer, skin cancer, lung cancer, thymic cancer, stomach cancer, colon cancer, liver cancer, ovarian cancer, uterine cancer, bladder cancer, renal cancer, testicular cancer, rectal cancer, breast cancer, and pancreatic cancer. In a preferred embodiment, the combination is used to treat and/or prevent a metastasis. In other preferred embodiments, the combination is used to treat a cancer including but not limited to hepatocellular carcinoma, colorectal cancer, renal cell carcinoma, bladder cancer, lung cancer (including non-small cell lung cancer), stomach cancer, esophageal cancer, sarcoma, mesothelioma, melanoma, pancreatic cancer, head and neck cancer, ovarian cancer, cervical and liver cancer. In some embodiments, the combination is used to treat a cancer selected from the group consisting of hepatocellular carcinoma, colorectal cancer, renal cell carcinoma, bladder cancer, lung cancer (including non-small cell lung cancer), stomach cancer, esophageal cancer, sarcoma, mesothelioma, melanoma, pancreatic cancer, head and neck cancer, ovarian cancer, cervical and liver cancer. In some embodiments, the combination is used to treat colorectal cancer, particularly metastatic colorectal cancer. In some embodiments, the mammal to be treated is a human. In another preferred aspect, the combination is used to treat a cancer that is resistant to one or more immune checkpoint inhibitors (e.g., the cancer is resistant to immunotherapy with PD-1, CTLA-4, LAG3, and/or TIGIT inhibitors). In some embodiments, the cancer is a solid cancer or solid tumor.

The methods include concurrently administering therapeutically effective amounts of a replicative oncolytic vaccinia virus and an immune checkpoint inhibitor. A therapeutically effective amount of oncolytic virus is defined as that amount sufficient to induce oncolysis - the disruption or lysis of a cancer cell. Preferably, the oncolytic vaccinia virus and the immune checkpoint inhibitor are administered in synergistically effective amounts. The term includes the slowing, inhibition, or reduction in the growth or size of a tumor and includes the eradication of the tumor in certain instances. In some embodiments, an effective amount of oncolytic vaccinia virus results in systemic dissemination of the therapeutic virus to tumors, e.g., infection of non-injected tumors. In some embodiments, an effective amount of the oncolytic vaccinia virus is an amount sufficient to induce oncolysis - the disruption or lysis of a cancer cell.

In some embodiments, cancer treatment and/or prevention indicates an at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, at least 99%, or about 100% reduction and/or decrease in tumor size and/or presence after treatment. In some embodiments, cancer treatment and/or prevention indicates complete tumor regression after the treatment. In some embodiments, cancer treatment and/or prevention indicates complete tumor remission after the treatment. In some embodiments, the cancer is refractory or resistant to an immune checkpoint inhibitor therapy. In some embodiments, the cancer is refractory or resistant to treatment with anti-PD-1 antibodies, anti-PD-L1 antibodies, and/or anti-CTLA-4 antibodies. In some embodiments, the cancer is resistant to treatment with anti-PD-1 antibodies. In some embodiments, the cancer is resistant to treatment with anti-CTLA-4 antibodies. In some embodiments, the treatment comprises administering a replicative oncolytic vaccinia virus. In some embodiments, the treatment comprises administering a replicative oncolytic vaccinia virus and an immune checkpoint inhibitor. In some embodiments, the treatment comprises administering a replicative oncolytic vaccinia virus and an immune checkpoint inhibitor, wherein the immune checkpoint inhibitor is an inhibitor of PD-1, PD-L1, CTLA-4, LAG3, TIGIT, and/or TIM3. In some embodiments, the treatment comprises administering a replicative oncolytic vaccinia virus, an inhibitor of PD-1 or PD-L1, and an immune checkpoint inhibitor. In some embodiments, the treatment comprises administering a replicative oncolytic vaccinia virus, an inhibitor of PD-1 or PD-L1, and an immune checkpoint inhibitor, wherein the immune checkpoint inhibitor is an inhibitor of CTLA-4, an inhibitor of LAG3, an inhibitor of TIGIT, or an inhibitor of TIM3. In some embodiments, the treatment comprises administering a replicative oncolytic vaccinia virus, an inhibitor of PD-1, and an immune checkpoint inhibitor, wherein the immune checkpoint inhibitor is an inhibitor of CTLA-4. In some embodiments, the treatment comprises administering a replicative oncolytic vaccinia virus, an inhibitor of PD-L1, and an immune checkpoint inhibitor, wherein the immune checkpoint inhibitor is an inhibitor of CTLA-4. In some embodiments, the treatment comprises administering a replicative oncolytic vaccinia virus, an inhibitor of PD-1 and an immune checkpoint inhibitor, wherein the immune checkpoint inhibitor is an inhibitor of LAG3. In some embodiments, the treatment comprises administering a replicative oncolytic vaccinia virus, an inhibitor of PD-L1, and an immune checkpoint inhibitor, wherein the immune checkpoint inhibitor is an inhibitor of LAG3. In some embodiments, the treatment comprises administering a replicative oncolytic vaccinia virus, an inhibitor of PD-1, and an immune checkpoint inhibitor, wherein the immune checkpoint inhibitor is an inhibitor of TIGIT. In some embodiments, the treatment comprises administering a replicative oncolytic vaccinia virus, an inhibitor of PD-L1, and an immune checkpoint inhibitor, wherein the immune checkpoint inhibitor is an inhibitor of TIGIT. In some embodiments, the treatment comprises administering a replicative oncolytic vaccinia virus, an inhibitor of PD-1, and an immune checkpoint inhibitor, wherein the immune checkpoint inhibitor is an inhibitor of TIM3. In some embodiments, the treatment comprises administering a replicative oncolytic vaccinia virus, an inhibitor of PD-L1, and an immune checkpoint inhibitor, wherein the immune checkpoint inhibitor is an inhibitor of TIM3.

In some embodiments, the replicative oncolytic vaccinia virus is administered by intratumoral, IV and/or intraperitoneal administration. In some embodiments, the replicative oncolytic vaccinia virus is administered by intratumoral administration. In some embodiments, the replicative oncolytic vaccinia virus is administered by IV administration. In some embodiments, the replicative oncolytic vaccinia virus is administered by intraperitoneal administration. In some embodiments, the replicative oncolytic vaccinia virus is administered by intra-arterial administration. In some embodiments, the replicative oncolytic vaccinia virus is administered only by intratumoral administration. In some embodiments, the replicative oncolytic vaccinia virus is administered only by intratumoral administration.

The checkpoint inhibitor as disclosed herein can be administered by various routes including, for example, orally or parenterally, such as intravenously, intramuscularly, subcutaneously, intraorbitally, intracapsularly, intraperitoneally, intrarectally, intracisternally, intratumorally, intravasally, intradermally or by passive or facilitated absorption through the skin using, for example, a skin patch or transdermal iontophoresis, respectively. In some embodiments, the checkpoint inhibitor is administered systemically. The checkpoint inhibitor also can be administered to the site of a pathologic condition, for example, intravenously or intra-arterially into a blood vessel supplying a tumor. In some embodiments, the checkpoint inhibitor is an inhibitor of PD-1, PD-L1, CTLA-4, LAG3, TIGIT, and/or TIM3.

In some embodiments, the replicative oncolytic virus is administered intratumorally and the checkpoint inhibitor is administered systemically. In some embodiments, the replicative oncolytic virus is administered intravenously and the checkpoint inhibitor is administered systemically. In some embodiments, the replicative oncolytic virus is administered intraperitoneally and the checkpoint inhibitor is administered systemically. In some embodiments, the replicative oncolytic virus is administered intra-arterially and the checkpoint inhibitor is administered systemically.

The total amount of an agent to be administered in practicing a method of the invention can be administered to a subject as a single dose, either as a bolus or by infusion over a relatively short period of time, or can be administered using a fractionated treatment protocol, in which multiple doses are administered over a prolonged period of time. One skilled in the art would know that the amount of the composition to treat a pathologic condition in a subject depends on many factors including the age and general health of the subject as well as the route of administration and the number of treatments to be administered. In view of these factors, the skilled artisan would adjust the particular dose as necessary. In general, the formulation of the composition and the routes and frequency of administration are determined, initially, using Phase I and Phase II clinical trials.

In certain embodiments, the checkpoint inhibitor is administered in 0.01-0.05 mg/kg, 0.05-0.1 mg/kg, 0.1-0.2 mg/kg, 0.2-0.3 mg/kg, 0.3-0.5 mg/kg, 0.5-0.7 mg/kg, 0.7-1 mg/kg, 1-2 mg/kg, 2-3 mg/kg, 3-4 mg/kg, 4-5 mg/kg, 5-6 mg/kg, 6-7 mg/kg, 7-8 mg/kg, 8-9 mg/kg, 9-10 mg/kg, at least 10 mg/kg, or any combination thereof doses. Suitable dosages of the checkpoint inhibitor range from about 0.5 mg/kg to 25 mg/kg, preferably from about 1 mg/kg to about 20 mg/kg, more preferably from about 2 mg/kg to about 15 mg/kg. In certain embodiments the checkpoint inhibitor is administered at least once a week, at least twice a week, at least three times a week, at least once every two weeks, or at least once every month or multiple months. In certain embodiments, the checkpoint inhibitor is administered as a single dose, in two doses, in three doses, in four doses, in five doses, or in 6 or more doses. Preferably, the checkpoint inhibitor is administered intravenously (e.g. by intravenous infusion or injection) or intratumorally. By way of non-limiting example, ipilimumab is preferably administered by intravenous infusion at a dose of 3mg/kg every three weeks for a total of four doses. In some embodiments, the checkpoint inhibitor is an inhibitor of PD-1, PD-L1, CTLA-4, LAG3, TIGIT, and/or TIM3.

### A. Additional Anticancer Therapeutics

One or more additional chemotherapeutic agents may be administered with the combination of the invention, including, without limitation, 5-fluorouracil (FU), folinic acid (FA) (or leucovorin), methotrexate, capecitabine (Xeloda; an oral prodrug of 5-FU), oxaliplatin (Eloxatin), bevacizumab (Avastin), cetuximab (Erbitux) and panitumumab (Vectibix), in any combination. These agents may be administered according to known treatment protocols. Generally, the additional chemotherapeutic agent is administered intravenously, with the exception of capecitabine which is an oral formulation.

In other aspects, methods of the invention further comprise administering an additional cancer therapy such as radiotherapy, hormone therapy, surgery and combinations thereof.

Radiotherapy includes, without limitation, γ-rays, X-rays, and/or the directed delivery of radioisotopes to tumor cells. Other forms of DNA damaging factors are also contemplated such as microwaves and UV-irradiation. It is most likely that all of these factors effect a broad range of damage on DNA, on the precursors of DNA, on the replication and repair of DNA, and on the assembly and maintenance of chromosomes. Dosage ranges for X-rays range from daily doses of 50 to 200 roentgens for prolonged periods of time (3 to 4 wk), to single doses of 2000 to 6000 roentgens. Dosage ranges for radioisotopes vary widely, and depend on the half-life of the isotope, the strength and type of radiation emitted, and the uptake by the neoplastic cells.

Approximately 60% of persons with cancer will undergo surgery of some type, which includes preventative, diagnostic or staging, curative and palliative surgery. Curative surgery is a cancer treatment that may be used in conjunction with other therapies, such as the treatment of the present invention, chemotherapy, radiotherapy, hormonal therapy, gene therapy and/or alternative therapies.

Curative surgery includes resection in which all or part of cancerous tissue is physically removed, excised, and/or destroyed. Tumor resection refers to physical removal of at least part of a tumor. In addition to tumor resection, treatment by surgery includes laser surgery, cryosurgery, electrosurgery, and microscopically controlled surgery (Mohs' surgery). It is further contemplated that the present invention may be used in conjunction with removal of superficial cancers, precancers, or incidental amounts of normal tissue.

Upon excision of part of or all of cancerous cells, tissue, or tumor, a cavity may be formed in the body. Treatment may be accomplished by perfusion, direct injection or local application of the area with an additional anti-cancer therapy. Such treatment may be repeated, for example, every 1, 2, 3, 4, 5, 6, or 7 days, or every 1, 2, 3, 4, and 5 weeks or every 1, 2, 3, 4, 5, 6, 7, 8, 9, 10, 11, or 12 months. These treatments may be of varying dosages as well.

Another form of therapy for use in conjunction with the current methods includes hyperthermia, which is a procedure in which a patient's tissue is exposed to high temperatures (up to 106°F). External or internal heating devices may be involved in the application of local, regional, or whole-body hyperthermia. Local hyperthermia involves the application of heat to a small area, such as a tumor. Heat may be generated externally with high-frequency waves targeting a tumor from a device outside the body. Internal heat may involve a sterile probe, including thin, heated wires or hollow tubes filled with warm water, implanted microwave antennae, or radiofrequency electrodes.

A patient's organ or a limb is heated for regional therapy, which is accomplished using devices that produce high energy, such as magnets. Alternatively, some of the patient's blood may be removed and heated before being perfused into an area that will be internally heated. Whole-body heating may also be implemented in cases where cancer has spread throughout the body. Warm-water blankets, hot wax, inductive coils, and thermal chambers may be used for this purpose.

Hormonal therapy may also be used in conjunction with the present invention or in combination with any other cancer therapy previously described. The use of hormones may be employed in the treatment of certain cancers such as breast, prostate, ovarian, or cervical cancer to lower the level or block the effects of certain hormones such as testosterone or estrogen.

### B. Compositions And Formulations

The replicative oncolytic vaccinia virus of the pharmaceutical combination is administered to treat cancer and/or directly to tumor cells and accordingly, the pharmaceutical compositions disclosed herein are formulated for the desired administration route (*e.g.* by intratumoral injection, intravenously, intra-arterially, and/or intraperitoneal administration). In some embodiments, the replicative oncolytic vaccinia virus of the pharmaceutical combination is formulated for administration by intratumoral, intravenously, intra-arterially, and/or intraperitoneal administration routes. In some embodiments, the replicative oncolytic vaccinia virus of the pharmaceutical combination is formulated for administration by intratumoral administration. In some embodiments, the replicative oncolytic vaccinia virus of the pharmaceutical combination is formulated for administration by intravenous administration. In some embodiments, the replicative oncolytic vaccinia virus of the pharmaceutical combination is formulated for intra-arterial administration. In some embodiments, the replicative oncolytic vaccinia virus of the pharmaceutical combination is formulated for administration by intraperitoneal administration. In some embodiments, the replicative oncolytic vaccinia virus of the pharmaceutical combination is formulated for administration only by intratumoral administration.

Intratumoral injection of the oncolytic vaccinia virus may be by syringe or any other method used for injection of a solution, as long as the expression construct can pass through the particular gauge of needle required for injection. A novel needleless injection system has recently been described (U.S. Patent 5,846,233, incorporated herein by reference) having a nozzle defining an ampule chamber for holding the solution and an energy device for pushing the solution out of the nozzle to the site of delivery. A syringe system has also been described for use in gene therapy that permits multiple injections of predetermined quantities of a solution precisely at any depth (U.S. Patent 5,846,225, incorporated herein by reference).

Solutions of the active compounds as free base or pharmacologically acceptable salts may be prepared in water suitably mixed with a surfactant, such as hydroxypropylcellulose. Dispersions may also be prepared in glycerol, liquid polyethylene glycols, and mixtures thereof and in oils. Under ordinary conditions of storage and use, these preparations contain a preservative to prevent the growth of microorganisms. The pharmaceutical forms suitable for injectable use include sterile aqueous solutions or dispersions and sterile powders for the extemporaneous preparation of sterile injectable solutions or dispersions (U.S. Patent 5,466,468, specifically incorporated herein by reference in its entirety). In all cases the form must be sterile and must be fluid to the extent that easy syringability exists. It must be stable under the conditions of manufacture and storage and must be preserved against the contaminating action of microorganisms, such as bacteria and fungi. The carrier can be a solvent or dispersion medium containing, for example, water, ethanol, polyol (e.g., glycerol, propylene glycol, and liquid polyethylene glycol, and the like), suitable mixtures thereof, and/or vegetable oils. Proper fluidity may be maintained, for example, by the use of a coating, such as lecithin, by the maintenance of the required particle size in the case of dispersion and by the use of surfactants. The prevention of the action of microorganisms can be brought about by various antibacterial and antifungal agents, for example, parabens, chlorobutanol, phenol, sorbic acid, thimerosal, and the like. In many cases, it will be preferable to include isotonic agents, for example, sugars or sodium chloride. Prolonged absorption of the injectable compositions can be brought about by the use in the compositions of agents delaying absorption, for example, aluminum monostearate and gelatin.

For intratumoral injection in an aqueous solution, for example, the solution may be suitably buffered if necessary and the liquid diluent first rendered isotonic with sufficient saline or glucose. In this connection, sterile aqueous media that can be employed will be known to those of skill in the art in light of the present disclosure. For example, one dosage may be dissolved in 1 ml of isotonic NaCl solution and either added to 1000 ml of hypodermoclysis fluid or injected at the proposed site of infusion, (see for example, "Remington's Pharmaceutical Sciences" 15th Edition, pages 1035-1038 and 1570-1580). Some variation in dosage will necessarily occur depending on the condition of the subject being treated. The person responsible for administration will, in any event, determine the appropriate dose for the individual subject. Moreover, for human administration, preparations should meet sterility, pyrogenicity, general safety and purity standards as required by FDA Office of Biologics standards.

Sterile injectable solutions are prepared by incorporating the active compounds in the required amount in the appropriate solvent with various of the other ingredients enumerated above, as required. Generally, dispersions are prepared by incorporating the various sterilized active ingredients into a sterile vehicle which contains the basic dispersion medium and the required other ingredients from those enumerated above. In the case of sterile powders for the preparation of sterile injectable solutions, the preferred methods of preparation are vacuum-drying and freeze-drying techniques which yield a powder of the active ingredient plus any additional desired ingredient from a previously sterile-filtered solution thereof.

The compositions disclosed herein may be formulated in a neutral or salt form. Pharmaceutically-acceptable salts, include the acid addition salts (formed with the free amino groups of the protein) and which are formed with inorganic acids such as, for example, hydrochloric or phosphoric acids, or such organic acids as acetic, oxalic, tartaric, mandelic, and the like. Salts formed with the free carboxyl groups can also be derived from inorganic bases such as, for example, sodium, potassium, ammonium, calcium, or ferric hydroxides, and such organic bases as isopropylamine, trimethylamine, histidine, procaine and the like. Upon formulation, solutions will be administered in a manner compatible with the dosage formulation and in such amount as is therapeutically effective. The formulations are easily administered in a variety of dosage forms such as injectable solutions, drug release capsules and the like.

As used herein, "carrier" includes any and all solvents, dispersion media, vehicles, coatings, diluents, antibacterial and antifungal agents, isotonic and absorption delaying agents, buffers, carrier solutions, suspensions, colloids, and the like. The use of such media and agents for pharmaceutical active substances is well known in the art. Except insofar as any conventional media or agent is incompatible with the active ingredient, its use in the therapeutic compositions is contemplated. Supplementary active ingredients can also be incorporated into the compositions.

The phrase "pharmaceutically-acceptable" or "pharmacologically-acceptable" refers to molecular entities and compositions that do not produce an allergic or similar untoward reaction when administered to a human. The preparation of an aqueous composition that contains a protein as an active ingredient is well understood in the art. Typically, such compositions are prepared as injectables, either as liquid solutions or suspensions; solid forms suitable for solution in, or suspension in, liquid prior to injection can also be prepared.

### EXAMPLES

The following examples are given for the purpose of illustrating various embodiments of the invention and are not meant to limit the present invention in any fashion. One skilled in the art will appreciate readily that the present invention is well adapted to carry out the objects and obtain the ends and advantages mentioned, as well as those objects, ends and advantages inherent herein. The present examples, along with the methods described herein are presently representative of preferred embodiments, are exemplary, and are not intended as limitations on the scope of the invention. Changes therein and other uses which are encompassed within the spirit of the invention as defined by the scope of the claims will occur to those skilled in the art.

### EXAMPLE 1

The ability of combination treatment with oncolytic vaccinia virus and checkpoint inhibitor(s) to regress tumors in the syngeneic mouse model of metastatic renal cell carcinoma (Renca) was assessed. The pattern of growth of this tumor accurately mimics that of human adult renal cell carcinoma, particularly with regard to spontaneous metastasis to lung and liver. This Renca model is a hypervascular, resistant to anti-PD-1 immunotherapy and is immune responsive. Because infection is independent of tissue of origin, this Renca model is relevant to all cancers.

### Materials and Methods

Mice and Cell Lines - Specific pathogen-free BALB/c male mice were housed under filter topped cages with water and food supplied with an inverse 12 hours day and night cycle. All mice were anesthetized by intramuscular injection of a combination of anesthetics (80mg/kg ketamine and 12mg/kg of xylazine) before being sacrificed. The Renca renal carcinoma cell line and CT26 colon cancer cell line was obtained from ATCC and cultured in RPMI-1640 medium containing 10% FBS and 1% Penicillin-Streptomycin antibiotics at 37°C with 5% CO₂.

### Virus Amplification -

mJX594 is a Western Reserve vaccinia virus engineered to contain a disruption of the viral thymidine kinase gene and insertion of murine GMCSF-GFP (mGMSCF-GFP) under the control of the synthetic early late promoter. 100% confluency level of HeLaS3 cells were infected with mJX594 with multiplicity of infection (moi) = 1-3 and placed in a CO₂ incubator at 37°C for 1.5 hr followed by applying DMEM containing 2.5% FBS and incubating for 48 to 72 hours. Cells were collected by centrifugation and the supernatant was discarded. The cells were resuspended in 10mM Tris-Cl, pH 9.0, homogenized in a Dounce homogenizer and centrifuged. The cell pellet was resuspended in 10mM Tris-Cl, pH 9.0, centrifuged, and the supernatant was combined with the first supernatant. The supernatant was combined with the first supernatant. The sonicated lysate was placed on top of 36% sucrose and centrifuged at 32,900 × g for 80 mins at 4°C. Then the pellet was resuspended in 10mM Tris-Cl, pH 9.0, stored below -60°C.

### ICI inhibitors (also referred to herein as immune checkpoint inhibitors) -

Antibodies against CTLA-4 and PD-1 were purchased from BioXcell. The 9D9 monoclonal antibody reacts with mouse CTLA-4. Isotype: Mouse IgG2b. The J43 monoclonal antibody reacts with mouse PD-1. Isotype: Armenian Hamster IgG.

### Tumor Model and Treatment Schedule -

To generate a clinically relevant kidney tumor model, suspensions of Renca tumor cells (kidney cancer, syngeneic) (5 × 10⁵ cells in 100µl) were subcutaneously injected into the right dorsal flank of 8- to 10-week-old immune competent Balb/c male mice. When the average tumor volume exceeded 50 mm³, mice were randomized and received the treatment regimens described below.

Tumor size was measured every 3 days in all groups with a digital caliper. Tumor volume was calculated according to the formula 0.5 X A X B², where A is the largest diameter of a tumor and B is its perpendicular diameter. Indicated days later, mice were sacrificed by CO₂ and tissues harvested for further analysis.

### Histological Analyses -

For immunofluorescence studies, samples were fixed in 1% PFA, dehydrated in 20% sucrose solution overnight, and embedded in tissue freezing medium (Leica). Frozen blocks were cut into 50-µm sections. Samples were blocked with 5% goat (or donkey) serum in PBST (0.03% Trition X-100 in PBS) and then incubated for 3 hr at room temperature (RT) with the following primary antibodies: anti-GFP (rabbit, Millipore), anti-CD31 (hamster, clone 2H8, Millipore), anti-VEGFR2 (rabbit, Cell signaling), anti-CD8a (rat, BD pharmingen), anti-CD11b (rat, BD pharmingen), anti-FoxP3 (rat, eBioscience), anti-caspase3 (rabbit, R&D systems), anti-Vaccinia (rabbit, Abcam), and anti-PD-L1 (rat, eBioscience). After several washes, the samples were incubated for 2 hr at room temperature with the following secondary antibodies: FITC-, Cy3-, or Cy5-conjugated anti-hamster IgG (Jackson ImmunoResearch), FITC- or Cy3-conjugated anti-rabbit IgG (Jackson ImmunoResearch), Cy3-conjugated anti-rat IgG (Jackson ImmunoResearch), or Cy3-conjugated anti-mouse IgG (Jackson ImmunoResearch). Nuclei were stained with 4',6-diamidino-2-phenylindole (DAPI, Invitrogen). Then the samples were mounted with fluorescent mounting medium (DAKO) and immunofluorescent images were acquired using a Zeiss LSM880 confocal microscope (Carl Zeiss).

### Morphometric Analyses -

Density measurement of blood vessels, CD8 T-cells or apoptotic area were performed with ImageJ software (http:/lrsb.info.nih.gov/ij). CD31, CD8, or caspase3+ area per random 0.42 mm² areas was measured in the peri- and intratumoral regions. All measurements were performed at least five different fields per mice.

### Flow cytometry -

Collected tumor tissues were minced and incubated in FACS buffer (1% FBS in PBS) containing collagenase D (Roche) and DNase I (Roche) at 37°C for 1-2 hours in shaking water bath. The digested cells were filtered with a 40 µm nylon mesh to remove cell clumps. RBC was removed by incubating cell suspension in ACK lysis buffer for 5 min at RT. The resulting single cells were incubated for 30 minutes with the following antibodies in FACS buffer: PerCP-cy5.5-conjugated anti-mouse CD45 (rat, eBioscience), APC-conjugated anti-mouse CD3e (hamster, eBioscience), FITC-conjugated anti-mouse CD4 (rat, ebioscience), PE-conjugated anti-CD8a (rat, eBioscience), FITC-conjugated anti-mouse CD11b (rat, eBioscience), APC-conjugated anti-mouse Gr1 (rat, eBioscience), and APC-conjugated anti-mouse CD11c (hamster, eBioscience).

### Statistical Analyses -

Values are presented as mean ± standard deviation. Statistical differences between means were determined by unpaired Student t-test or analysis of variance with one-way followed by the Student-Newman-Keuls test. Statistical significance was set at p<0.05.

### Results

### Anti-Tumor Effects of Vaccinia Virus + anti-PD-1 Combination Therapy

For combination therapy with anti-PD-1 and mJX-594, 5 × 10⁵ Renca tumor cells were injected into the right dorsal flank of BALB/c mice and treatment was initiated (Day 0) when the tumor size reached 50-100 mm³. Mice were randomized into four treatment groups: (i) Control group: PBS was injected intratumorally every three days; (ii) mJX-594 monotherapy group: 1 × 10⁷ pfu of mJX-594 was injected intratumorally every 2 days and 3 times total on days 0, 2 and 4; (iii) anti-PD-1 monotherapy group: 10 mg/kg of antibody was injected intraperitoneally every 3 days and 4 times total on days 0, 3, 6 and 9; (iv) mJX-594 + anti-PD-1 combination group: mJX-594 and anti-PD-1 were administered concurrently, with mJX-594 intratumorally injected every two days on days 0, 2 and 4 for a total of three injections and anti-PD-1 intraperitoneally administered on days 0, 2, 4, 6 and 9 for a total of five injections (see **Figure 1A**).

Tumor growth suppression was observed in the mJX-594 monotherapy group and the mJX-594/anti-PD-1 combination group ("Combination group") compared to control (see **Figure 2A**). The suppression of tumor growth was more significant in the Combination group (see **Figure 2A****,** compare "PD1+mJX594" to "mJX594" and "PD1"). Tumor growth suppression was not observed in the anti-PD-1 monotherapy group (Figure 2A, compare "PD1" to "Control"). Tumor weight was shown to be decreased in the mJX-594 monotherapy group (See **Figure** 2**B**). A significant decrease in tumor weight was observed in the Combination group, which was substantially greater than the reduction observed in the mJX-594 monotherapy group (see **Figure 2B**). Thus, concurrent administration of mJX-594 and anti-PD-1 resulted in a marked decrease in tumor weight and volume compared to either monotherapy.

CD8 T-cell infiltration in both peritumoral and intratumoral regions was increased in PD-1 monotherapy, mJX-594 monotherapy and concurrent combination groups. (See **Figure 3A**). Whereas CD8 T-cell infiltration was increased in peripheral region rather than in central region in anti-PD-1 monotherapy group, mJX-594 group showed higher number of CD8 T-cell infiltration both in central and peripheral region. (See **Figure 3B**). Concurrently administered mJX-594 and anti-PD-1 antibody markedly increased intratumoral T-cell infiltration compared to control and to monotherapy with either agent alone as measured by peritumoral and intratumoral CD8+ staining. (See **Figure 3B**). Decreased vascular density was also observed in the treatment groups compared to control (See **Figure 3B**).

PD-L1 expression level in both peripheral and central tumor region was increased in anti-PD-1 monotherapy, mJX-594 monotherapy and concurrent combination groups compared to control. (See **Figure 4A**). Whereas the anti-PD-1 monotherapy group showed increased PD-L1 expression level in peripheral region rather than central region, the mJX-594 monotherapy group showed similar increase in PD-L1 expression level in both peripheral and central regions (See **Figure 4A**). Concurrent administration of JX-595 and anti-PD-1 antibody resulted in an increase in intratumoral PD-L1 expression compared to monotherapy with either agent alone (See **Figure 4A**). Renca tumors are resistant to anti-PD1 immunotherapy. An increase in tumor PD-L1 expression in the concurrent combination treatment group reflects sensitization of these tumors to anti-PD-1 therapy concomitant with infiltration of CD8+ T-cells into the tumor and is an indicator of treatment efficacy. These patterns suggest that at baseline, T-cells are immunosuppressed and unable to infiltrate the tumor microenvironment. mJX-594 causes inflammation and vasodilation, enabling T-cells to exert anti-tumor effects. Concurrent administration of mJX-594 and anti-PD-1 antibody leads to the activation and infiltration of T-cells into central tumor regions.

Intratumoral apoptosis was observed to be increased in PD-1 monotherapy, mJX-594 monotherapy and concurrent combination groups compared to control as measured by caspase3 staining (See **Figure 4B**). A marked increase in intratumoral apoptosis was confirmed in the concurrent combination group compared to either monotherapy group (See **Figure 4B**). The anti-vascular effects (shown in **Figure 3B**) combined with apoptosis in the concurrent combination group suggests significant tumor necrosis in the concurrent combination group.

A change in the immune microenvironment after concurrent mJX-594 and anti-PD-1 antibody was observed compared to control and to monotherapy with either agent as measured by CD4 and CD11b. (See **Figure 5A**). Importantly, tumor infiltration of CD8 T-cells was highest in the concurrent combination group. Depletion of CD8+ cells using anti-CD8 antibody resulted in decreased tumor growth inhibition confirming the role of these cells in the anti-tumor effect (data not shown). Myeloid-derived suppressor cells (MDSCs) were increased in mJX-594 and concurrent combination groups compared to control. Traditionally, CD11b+Gr1+ cells have been simply regarded as immune-suppressive cells; however, recent evidence has demonstrated that these cells cannot be so simply defined (contrast the relative increase of MDSCs observed in the concurrent combination group with αPD1 with the decrease of MDSCs observed in the concurrent combination group with aCTLA4 (**Figs. 10A-10B**).

The effect of co-administering mJX-594 and PD-1 ± CTLA4 sequentially versus concurrently on tumor growth was assessed. 5 × 10⁵ Renca (kidney cancer, syngeneic) cells were injected subcutaneously into the right flank of 8 week old BALB/c immune competent mice. Treatment was initiated (Day 0) when the tumor size reached 50 mm³

Mice were separated into four treatment groups: (i) Control group: PBS was injected on days 0, 3, 6, 9, 12 and 15; (ii) mJX-594 + anti-PD-1 sequential combination group: mJX-594 was intratumorally injected on days 0, 3, 6 and 9 and anti-PD-1 was intraperitoneally injected on days 6, 9, 12 and 15 (iii) mJX-594 and anti-PD-1 concurrent combination group: mJX-594 and anti-PD-1 were administered concurrently, with mJX-594 intratumorally injected on days 0, 3, 6 and 9 and anti-PD-1 intraperitoneally administered on days 0, 3, 6, 9, 12, and 15; (iv) mJX-594 + anti-PD-1 + anti-CTLA4 triple concurrent combination group: mJX-594 was intratumorally injected on days 0, 3, 6 and 9; anti-PD-1 and CTLA4 were intraperitoneally injected on days 0, 3, 6, 9, 12 and 15 (See **Figure 6**).

Tumor growth was suppressed in the mJX-594 + anti-PD-1 sequential and concurrent administration groups as well as in the mJX-594 + anti-PD-1 + anti-CTLA4 triple concurrent administration group compared to control (See **Figure 7**). Surprisingly, concurrent administration of mJX-594 and anti-PD-1 resulted in more significant suppression (delay) of tumor growth than sequential administration of these agents (See Figure 7). Further delay of tumor growth was observed in in the triple concurrent administration group ("Combi (mJX-594+ αPD1 + αCTLA4"). (See **Figure 7**).

When tumor size of each mouse was compared among the treatment groups, tumor regression was confirmed in the combination groups compared to control. Whereas the tumor regression was observed from day 12 in the sequential group, tumors tend to be regressed from day 6 in the concurrent and triple concurrent combination groups.

These results surprisingly suggest that the administration regimen and potentially the route of administration can significantly affect the anti-tumor effects of combination therapy. In particular, vaccinia virus synergizes with checkpoint inhibitor (anti-PD-1, CTLA-4) to induce a strong anti-tumor immune reaction if the vaccinia virus and checkpoint inhibitor are concurrently administered. In some instances, there was a strong anti-tumor immune reaction when the vaccinia virus was administered intratumorally as part of the concurrent administration. The synergistic anti-tumor effects observed for concurrent administration of vaccinia virus and checkpoint inhibitor are particularly surprising because immune checkpoint inhibitors are understood in the art to inhibit replication of oncolytic viruses such as vaccinia (Rojas etal., J. Immunol., 192 (1 Supplement): 142.3 (2014)).

### Vaccinia Virus + anti-CTLA4

5 × 10⁵ Renca (kidney cancer) cells were injected subcutaneously into the right flank of 8 week old BALB/c immune competent mice. Treatment was initiated (Day 0) when the tumor size reached 50-100 mm³.

Mice were randomized into five treatment groups: (i) Control group: PBS was injected intratumorally on days 0, 3, 6, 9, 12 and 15; (ii) mJX-594 monotherapy group: 1 × 10⁷ pfu of mJX-594 was injected intratumorally on days 0, 3, 6, and 9; (iii) anti-CTLA4 monotherapy group: 4 mg/kg of antibody was injected intraperitoneally on days 0, 3, 6, 9, 12, 15; (iv) mJX-594 + anti-CTLA4 sequential combination group: mJX-594 and anti-CTLA4 were administered sequentially, with mJX-594 intratumorally injected on days 0, 3, 6 and 9 and anti-CTLA4 intraperitoneally administered on days 6, 9, 12, and 15; (v) mJX-594 and anti-CTLA4 simultaneous combination group: mJX-594 and anti-CTLA4 were administered sequentially, with mJX-594 intratumorally injected on days 0, 3, 6 and 9 and anti-CTLA4 intraperitoneally injected on days 0, 3, 6, 9, 12 and 15 (See **Figure 8**).

Tumors size was measured every 3 days in all groups. Mice were sacrificed when the observation was done (day 16) by CO₂ and tumor was taken and subjected to flow cytometry analysis (CD4+ and CD8+ tumor infiltrating lymphocyte (TIL), Gr1+/CD11b+ MDSC).

It was confirmed that tumor growth was suppressed in all treatment groups compared to control. (See Figure 9). Significantly more tumor growth suppression was observed in the combination treatment groups, with the greatest tumor growth suppression observed in the concurrent administration group (See **Figure 9**).

When tumor size of each mouse was compared among the treatment groups, tumor regression was confirmed in the combination groups compared to monotherapy and control groups. Increased CD8 T-cell infiltration was observed in all treatment groups compared to control. (See **Figure 10A**). Whereas MDSC level increased in the mJX-594 monotherapy group compared to control, no significant change was observed in the sequential combination treatment group and a decrease in MDSC level was observed in the concurrent combination treatment group (See **Figure 10B**).

### Concurrent administration of JX929 and immune checkpoint inhibitors

Antitumor effects were tested in the mouse Renca model described above for JX929 administered IT (6 x 10⁷ pfu) concurrently with intraperitoneally administered PD-1 checkpoint inhibitor. JX929 is a Western Reserve strain vaccinia virus with disruptions in the viral TK and VGF genes (TK⁻/VGF⁻phenotype) and does not express GM-CSF.

### Cell Lines -

The murine RENCA cells (ATCC) were cultured in RPMI 1640 supplemented with 10 % fetal bovine serum (FBS), 1% penicillin-streptomycin and were maintained at 37°C with 5% CO₂.

### In Vivo Studies -

Eight-week-old female BALB/c mice were injected with RENCA cells (2×10⁶ cells) in 100µl of PBS into the subcapsule of the left kidney. On day 10 post-implantation, mice harboring Renca tumors (50 mm³-100 mm³ as visualized with the IVIS^{®} Spectrum in vivo imaging system) were treated intraperitoneally (i.p) with (i) PBS (control) (ii) vaccinia virus (JX-929) monotherapy (6×10⁷PFU on days 10, 11 and 12 post-implantation for a total of 3 doses) (iii) anti-PD1 monotherapy (BioXcell, West Lebanon, NH, 100µl) (days 10, 11 and 12 post-implantation for a total of 3 doses) or (iv) concurrent JX929 + anti-PD1 treatment (each administered on days 10, 11 and 12 post-implantation, with JX-929 administered on the morning and ICI in the afternoon of the same day with a 9-hour interval) according to the regimen shown in **Figure 1B****.**

Mice were sacrificed 2 days after final treatment for further histological and flow cytometric analysis.

### Flow Cytometry -

Peripheral blood samples were collected and red blood cells were lysed with RBC lysis buffer. Cells were washed in PBS containing 1% FBS, then stained with monoclonal mouse anti-CD8, rabbit anti-CD4, rabbit anti-CD3 antibodies (Santa Cruz Biotechnology, CA, USA). Cells were fixed with 4% paraformaldehyde then incubated with FITC-conjugated goat anti-rabbit or goat APC-conjugated anti-mouse antibodies (Santa Cruz Biotechnology). For each sample, 10,000 cells were analyzed using FACS Calibur instrument (BD biosciences, CA, USA).

### Histological Analysis -

The mice were euthanized and vital organs including tumor-bearing kidneys and lungs were obtained, fixed with 10% neutered formalin (BBC Biochemical, WA, USA). The tissues were embedded in paraffin and sections (4µm in thickness) were stained using hematoxylin and eosin for basic histological analysis. For immunofluorescence and immunohistochemistry, sections were stained by standard method using a mouse monoclonal antibody specific for CD8 (Santa Cruz Biotechnology, CA, USA). Then the sections were either incubated with FITC-conjugated goat anti-mouse antibody (Santa Cruz Biotechnology) for immunofluorescence, or with Vectastain^{®} Elite ABC-Peroxidase kit (Vector Laboratories, CA, USA) and visualized by Vector SG (Vector Laboratories) for immunohistochemistry. The weight and volume of the harvested tumors from each treatment group was measured and compared.

### ELISpot Assay -

The IPNγ-secreting cells were assessed using the ELISpot mouse IPNγ kit (Mabtech, Cincinnati, OH) according to the manufacturer's protocol. Spleens were isolated and prepared as single-cell suspensions. Splenocytes were mixed with RENCA tumor cells or splenocytes from mice infected with Vaccinia virus at a ratio of 5:1, incubated for 24 hours at 37°C. The intensity of specific spots was analyzed using ImageJ software (NIH).

### Statistical Analysis -

All the values were presented as mean ± standard deviation (SD). Statistical analysis was performed using Instat 3 (GraphPad Software, CA, USA). Multiple comparisons were analyzed using one-way analysis of variance (ANOVA) and a Bonferroni post-hoc paired comparison test.

### mJX594 treatment induces expression of checkpoint proteins

Balb/c mice carrying Renca tumors exceeding 50 mm³ were administered four intratumoral doses of mJX594 (1 × 10⁷ on each of days 0, 3, 6, and 9) or PBS control according to the treatment regimen shown in **Figure 1C****.**

The level of immune checkpoint protein in the tumors of control and mJX594-treated animals was measured at day 0 (prior to treatment) and at day 12 after sacrifice. Figure 13 illustrates fold-changes in checkpoint proteins after treatment in mJX594-treated mice relative to control mice. As shown in Figure 13, mJX594 treatment induces expression of the checkpoint proteins including PD-1 (4-fold increase), PD-L1, PD-L2, CTLA4 (over 2-fold increase), LAG3, TIM3 (over 3-fold increase) and TIGIT (over 2-fold increase). Treatment with replicative oncolytic vaccinia virus results in dynamic changes in the tumor immune microenvironment including significant increases in checkpoint proteins PD-1, PD-L1, CTLA-4, LAG3, TIM3 and TIGIT, thereby sensitizing tumors to blockade of each of these checkpoint proteins with their respective checkpoint inhibitors. Clinical trials have demonstrated that tumor-infiltrating cells and checkpoint protein (*e.g*. PD-L1) expression are indicators for the potential for treatment with checkpoint inhibitors (*e.g*. anti-PD-L1 treatment), supporting the efficacy of the combination therapy described herein not just in patients with tumors that express particular checkpoint proteins but also in patients with tumors that express low levels of (or do not express) particular checkpoint proteins.

### EXAMPLE 2

### Tumor Microenvironment Remodeling by Intratumoral Oncolytic Vaccinia Virus Enhances The Efficacy of Immune Checkpoint Blockade

### SUMMARY

Cancer immunotherapy is a potent and durable treatment modality, but its clinical benefit is still not universal. Here, we employed mJX-594, a targeted and GM-CSF-armed oncolytic vaccinia virus (VV), as a combination partner for immune checkpoint inhibitors (ICIs) in mice with implanted kidney cancer, colon cancer, and those with spontaneous breast cancer. The intratumoral injection of VV induced a profound remodeling of tumor microenvironment, transforming the tumor from non-T cell-non-inflamed to T cell-inflamed tumor with increased number and enhanced effector function of CD8⁺ T cells. Moreover, the combination therapy of VV and ICIs was capable of inducing tumor regression with improved survival and anti-metastatic effect. Our findings indicate that VV elicits robust anti-cancer immunity in combination with ICIs, overcoming immunotherapy resistance.

### INTRODUCTION

Cancer immunotherapy with immune checkpoints inhibitors (ICIs) targeting PD-1 or CTLA-4 have demonstrated a potent and durable therapeutic efficacy and emerged as a new weapon in war on cancer (Hegde et al., 2016; Topalian et al., 2015; Wolchok and Chan, 2014). However, the clinical efficacy of ICIs is confined to tumors with T cell-inflamed tumor microenvironment (TME) (Gajewski, 2015; Topalian et al., 2016). In poorly immunogenic tumors with few tumor-infiltrating lymphocytes (TILs), TME lacks type I interferon signature and chemokines for T cell recruitment (Gajewski et al., 2013). Moreover, tumor vasculatures and stromal components may pose a barrier against intratumoral trafficking of T cells and their effector functions on tumor cells (De Palma and Jain, 2017; Rivera and Bergers, 2015; Sharma et al., 2017). Therefore, additional therapeutic interventions are required for these non-T cell-inflamed tumors to appropriately remodel the TME to render these tumors more sensitive to ICI treatments.

Oncolytic viruses (OVs) have been proposed as a novel class of anti-cancer therapy, and OVs with different backbones and transgenes are currently being evaluated in clinical trials (Bell, 2014; Lichty et al., 2014). Although the success of OVs was initially evaluated by their faster replication and enhanced oncolytic capability during the past decade, they are now beginning to be recognized as an immunotherapeutic because the most strong and durable responses after oncolytic virotherapy was coupled with successful induction of anti-tumor immunity with increased tumor-specific effector and memory T cells (Bell, 2014; Chiocca and Rabkin, 2014; Thorne, 2014). Nonetheless, because the therapeutic efficacy of OV was greatly hindered by immunosuppressive TME, releasing the brakes of the immune system is critical to maximize the immunotherapeutic efficacy of OVs (Bell and Ilkow, 2017; Hou et al., 2016; Liu et al., 2017). Therefore, the combination of OVs and ICIs is a rational and appealing strategy to overcome poorly immunogenic and immunosuppressive TME.

JX-594 (pexastimogene devacirepvec, Pexa-vec) is an oncolytic vaccinia virus (VV) that is engineered to express an immune-activating transgene, GM-CSF, and has the viral thymidine kinase gene disrupted (Kirn and Thorne, 2009). JX-594 showed impressive anti-cancer activity with low toxicity in preclinical and clinical studies and became one of the most feasible and promising OV platform in clinical development (Breitbach et al., 2011a; Cripe et al., 2015; Heo et al., 2013; Park et al., 2008). Besides its oncolytic and vascular disrupting activity, JX-594 is proposed to display *in situ* cancer vaccination effect because it can elicit adaptive immune response against tumor antigens for selective tumor disruption and subsequent additional tumor antigen release (Breitbach et al., 2011b; Breitbach et al., 2015a). Although JX-594 is now undergoing phase III randomized clinical trial in advanced hepatocellular carcinoma (Abou-Alfa et al., 2016), very few studies characterized its immune modulatory functions in primary TME as well as distant lesions after JX-594 treatment (Kim et al., 2018). Moreover, the optimal combination of JX-594 with immunotherapeutics such as ICIs was not yet pursued and verified.

Here, we comprehensively dissected the dynamic remodeling of TME with mouse variant of JX-594 (mJX-594, WR.TK⁻mGM-CSF) and investigated its immunotherapeutic potential to providez a rational combinatorial strategy with ICIs in poorly immunogenic tumor models.

### RESULTS

### mJX-594 converts immunosuppressive non-inflamed tumors into inflamed tumors

To determine the immunomodulatory potential of the oncolytic virus mJX-594, we examined the temporal changes of tumor microenvironment in the poorly immunogenic Renca tumors after a single mJX-594 injection. The level of mJX-594 was already high at day 1, peaked at day 3, and almost undetectable at day 7 after the injection **(****Figures 33A and 33B****)**. In contrast, tumor vasculature showed the opposite response to the viral levels; tumor vascular density was markedly reduced between day 1 and day 3 but was recovered at day 7 and thereafter after the injection **(****Figures 33A and 33B****),** indicating that mJX-594 induces a potent but transient tumor vascular disruption. Of note, population of CD8⁺ cytotoxic T cells within intratumoral area, which comprise the most critical aspect of anticancer immunity, began to rise strikingly at day 5, peaked at day 7, and remained at a high density at 2 weeks after injection **(****Figures 33A and 33B****),** clearly demonstrating distinct and long-lasting conversion of non-inflamed tumor into T-cell-inflamed tumor by mJX-594. In comparison, CD11c⁺ dendritic cells (DCs) was transiently emerged at day 3 and decreased thereafter **(****Figures 33A and 33B****)**. The level of PD-L1 expression was minimal at day 0 and being upregulated after mJX-594 treatment (Figures 1A and 1B). Intriguingly, the timing PD-L1 upregulation follows immediately after the massive influx of CD8⁺ TILs (Figure 1C), indicating the activation of negative feedback pathway that attempt to suppress T cellmediated immunity. Most of the PD-L1 expressing cells were cytokeratin⁺ tumor cells, and some CD11b⁺ myeloid cells also express PD-L1, whereas T cells did not (Figure 1D). Thus, mJX-594 is a potent and durable anti-cancer immunity enhancer by recruiting cytotoxic CD8⁺ T cell into the cold tumors as well as a transient tumor vasculature disruptor.

To elucidate the cancer immune pathways modulated by mJX-594, we comprehensively analyzed the changes in the expression levels of 750 immune-related genes following mJX-594 monotherapy using a PanCancer Immune Profiling panel. The results showed prominent differences in the genes related immune signatures between control- and mJX-594-treated tumors (**Figure 33E**). Approximately 100 immunomodulatory genes displayed statistically significant changes in their expression levels, including those involved in activation of type I IFN signaling, DC maturation, and T cell activation (**Figure 33F**). In particular, we observed generally higher expressions of both inhibitory (including Pd-1, Pd-l1, Ctla-4, and Lag-3) and agonistic (including Icos, Gitr, and Cd27) immune checkpoint molecules in TME compared with control (**Figures 33G**). Moreover, further analyses of the TME revealed increases in Th1 and Th2 response-related genes, which suggests immunomodulation by mJX-594 monotherapy (**Figures 33G**). We also found that several genes involved in TME and myeloid cells were significantly escalated (**Figures 33G**) Notably, increases in Nos2 and Cd86 expressions represent polarization of myeloid cells to M1 macrophages. These results indicate that mJX-594 elicits long-term immune activation through dynamic changes in the TME to remodel non-inflamed tumors into T cell-inflamed tumors that can respond to immune checkpoint blockade.

### mJX-594 augments intratumoral infiltration of CD8⁺ T cells and induces myeloid cell repolarization

mJX-594-induced tumor growth delay was dose-dependent (**Figures 34A and 34B**). In parallel, mJX-594-induced increases in infiltration of CD8⁺ T cells in both peritumoral and intra-tumoral regions were also dose-dependent (**Figures 34C and 34D**). Indeed, flow cytometric subset analysis of the lymphoid cell compartment also revealed that mJX-594-induced increased absolute numbers of intra-tumoral CD8⁺ and CD4⁺ T cells were dose-dependent (**Figure 34E and 34G**). Although the number of CD4⁺Foxp3⁺CD25⁺ regulatory T cells also increased following the triple administration of mJX-594 (**Figure 34H**), the ratio of CD8⁺ T cells to regulatory T cells was 5.3-fold higher compared with that of control treatment (**Figure 34I**), implying an overall increase in T cell effector function in TME by mJX-594 treatment. Additionally, the expressions of ICOS and granzyme B (GzB), which are co-stimulatory and T cell activation markers, were increased in CD8⁺ T cells following mJX-594 treatment (**Figure 34J**). Further subset analysis of the myeloid cell compartment revealed that there are no significant changes in CD1 1b+Gr1+ myeloid cell fraction in tumors treated with mJX-594 treatment (**Figure 34K**). However, the CD11b⁺Ly6G⁻Ly6C⁺ monocytic myeloid cell fraction was increased, while the CD11b⁺Ly6G⁺Ly6C^{int} granulocytic myeloid cell fraction was reduced, indicating polarization of myeloid cells following mJX-594 administration (**Figures 34L** and **34M**). These findings demonstrate that repeated mJX-594 administration enhanced anti-cancer immunity, resulting in increased infiltration of activated T cells and repolarization of myeloid cells.

### Intratumoral injection of mJX-594 leads to systemic and cancer-specific immune responses

To determine whether local injection of mJX-594 could induce a systemic immune response in non-injected distant tumors, we administered mJX-594 into the right side tumor after implantation of Renca tumors into both side flanks. This treatment suppressed the growth of both right and left (opposite, not injected side) Renca tumors (**Figure 35A**). In line with tumor growth inhibition in the bilateral sides, infiltrations of CD8⁺ T cells at intra-tumoral regions were 7.9- and 5.5-fold increases in both right and left Renca tumors (**Figures 35B** and **35C**), suggesting that local mJX-594 virotherapy is able to strongly activate systemic anti-cancer immunity.

Next, to exclude the possibility of direct viral spread to distant tumors through systemic circulation after local virotherapy, we examined viral replication in the left, non-injected Renca tumors and found no detectable vaccinia virus in the left tumors (**Figure 41**), indicating that the anti-cancer activity of mJX-594 was immune-mediated and was not a result of systemic viral spread.

To evaluate whether the observed systemic immune response was tumor-specific, we performed a similar experiment using mice implanted with Renca tumors on the right flank and CT26 tumors on the left flank. Intratumoral treatment of the right, Renca tumor with mJX-594 markedly decreased the growth of the injected tumor, while the growth of left, untreated CT26 tumor was unaffected (**Figure 35D**). Microscopic analyses provided consistent results in that CD8⁺ T cells accumulated in Renca but not CT26 tumors (**Figures 35E** and **35F**), indicating that mJX-594 virotherapy induced a tumor-specific CD8⁺ T cell response. Thus, these results suggest that local mJX-594 treatment can elicit systemic anticancer immunity, with tumor-specific lymphocyte infiltration even in distant tumors.

### Anti-cancer immunity plays a critical role in the overall therapeutic efficacy of JX.

To determine which components of the immune system were responsible for the therapeutic efficacy of mJX-594, we examined its effect on tumors in mice treated with neutralizing antibodies against CD8, CD4, or GM-CSF (**Figure 36A**). Of special note, depletion of either CD8⁺ or CD4⁺ T cells abrogated the effective inhibition of tumor growth by mJX-594 monotherapy (**Figures 36B** and **36C**), emphasizing the importance of immune-mediated mechanism rather than direct oncolysis, in mJX-594-induced tumor inhibition. Intriguingly, depletion of CD4⁺ T cells at the time of mJX-594 injection intriguingly decreased intra-tumoral infiltration of CD8⁺ T cell (**Figure 36D**), indicating that CD4⁺ T cells are involved in activation and recruitment of CD8⁺ T cells in TME. However, depletion of CD8⁺ T cell depletion did not significantly alter infiltration of CD4⁺ T cell (**Figure 36E**), indicating that CD8⁺ T cells did not affect CD4⁺ T cells in TME. These data show that intratumoral treatment of mJX-594 induces priming of CD8⁺ and CD4⁺ T cells, which may interact with each other to mediate anti-cancer immunity. Previous virotherapy based on herpes and vaccinia virus utilized GM-CSF as an immune-activating transgene, which recruit and activates antigen-presenting cells (APCs) that subsequently trigger T cell response. However, the use of GM-CSF is still controversial because its potential immunosuppressive roles in tumor progression such as inducing of proliferation of myeloid-derived suppressor cells (MDSCs) (Thorne, 2014). Consequently, we explored whether GM-CSF is required for the therapeutic effect of mJX-594. Interestingly, depletion of GM-CSF negated the anti-tumor effect of mJX-594 and reduced both CD8⁺ and CD4⁺ T cell levels, suggesting that GM-CSF is important for the immunotherapeutic efficacy of mJX-594 (**Figures 36C-36E**). Thus, both CD8⁺ and CD4⁺ T cells are indispensable mediators of the anti-cancer effect of mJX-594, and that GM-CSF could provide an immunotherapeutic benefit.

### Combination of mJX-594 with immune checkpoint blockade elicits a synergistic anticancer effect with enhanced infiltration of T lymphocytes into tumor

To overcome resistance to ICI monotherapy, we evaluated the benefit of combining mJX-594 with ICI. Combination of αPD-1 and mJX-594 reduced tumor growth by 70%, while each αPD-1 and mJX-594 monotherapy delayed tumor growth by 22.8% and 44% at 12 days after the treatments (**Figures 37A** and **37B**). In support of these findings, microscopic analyses identified a notable increase in recruitment of CD8⁺ T cells in both peritumoral (18.8-fold) and intratumoral (21.4-fold) regions of tumors treated with combination therapy compared with control (**Figures 37C** and **37D**). CD31⁺tumor blood vessels were meaningfully reduced in these regions (1.8-fold and 2.6-fold, respectively; **Figures 37C** and **37D**). In addition, more extensive tumor apoptosis was noted in tumors treated with combination therapy compared with control (Figures 5C and 5D). Though the PD-L1 expression was minimal in control tumors, it is highly upregulated following mJX-594 treatment (**Figures 37C** and **37D**). This finding suggests that the induced PD-L1 expression in TME is an adaptive negative feedback mechanism that dampens anti-cancer immunity after oncolytic virotherapy, therefore providing a rational for the combination therapy of mJX-594 and PD-1/PD-L1 blockade to potentiate the immunotherapeutic effect of mJX-594 (**Figure 37E**).

Overall, these results suggest that combination therapy can overcome resistance to mJX594 or αPD-1 monotherapy through enhanced anti-cancer immunity by increasing CD8⁺ T cell infiltration.

Similarly, combination treatment with αCTLA-4 and mJX-594 was also synergistic. Although tumor growth was modestly inhibited by either mJX-594 (42.0%) or αCTLA-4 (20.0%) monotherapy, stronger inhibition (57.6%) was observed with the combination therapy (**Figures 42A** and **42B**). In addition, after combination therapy, higher accumulation of CD8⁺ T cells was observed in both peripheral (27.0-fold increase) and central (26.4-fold increase) regions of tumors compared with controls (**Figures 42C** and **42D**). Along with increased levels of intratumoral CD8⁺ T cells, CD31⁺ vessels were also markedly disrupted in both peripheral and central regions compared with control (2.1-fold and 3.8-fold reductions, respectively; **Figures 42C** and **42E**). Furthermore, flow cytometry revealed that intratumoral infiltration of CD8⁺ and CD4⁺ T cells was also increased by mJX-594 and αCTLA-4 combination therapy (**Figures 42F-42H**).

Taken together, these results indicate that combination therapy using mJX-594 and ICI can overcome the immunotherapy resistance in immunosuppressive TMEs, resulting in synergistic anti-cancer effects.

### The efficacy of combination immunotherapy with intratumoral mJX-594 and ICIs is not largely affected by treatment schedule

Because ICIs can negatively affect viral replication and lead to premature clearance of OV, several studies explored optimal schedules of treatment using combinations of systemic oncolytic virotherapy and ICIs, and reported that some combination schedules could induce antagonize the therapeutic efficacy (Liu et al., 2017; Rojas et al., 2015). However, similar studies examining local oncolytic virotherapy have not been reported. To establish the optimal combination schedule for intratumoral mJX-594 and ICIs, we compared the following: (1) simultaneous administration of mJX-594 and ICI (schedule I); (2) initiation of ICI 3 days after administration of mJX-594 (schedule II); and (3) administration of mJX-594 3 days after initiation of ICI (schedule III; **Figure 38A**). All combination schedules delayed tumor growth by -40% (**Figures 38B** and **38C**). Likewise, levels of tumor-infiltrating CD8⁺ and CD4⁺ T cells were increased by >8-fold and >4.0-fold, respectively, which also showed remarkably increased levels of ICOS and GzB expression in CD8⁺ T cells (**Figures 38D-38F**).

Similarly to combination therapy with mJX-594 and αPD-1, the combination of mJX-594 and αCTLA-4 inhibited tumor growth by -40% regardless of the treatment schedule (**Figures 43A** and **43B**). Furthermore, intratumoral infiltration of CD8⁺ and CD4⁺ T lymphocytes (>7-fold and >7-fold increases, respectively), as well as GzB and ICOS expression in CD8⁺ T cells, were greater regardless of treatment schedule (Figures 43C-43E).

Collectively, combination therapy with intratumoral mJX-594 injection and systemic immune checkpoint blockade led to an effective anti-cancer immune response regardless of treatment schedule, suggesting that intratumoral administration of mJX-594 is not significantly affected by varying schedule of ICI administration.

### The triple combination of mJX-594, αPD1, and αCTLA4 can induce complete tumor regression and provides a long-term survival benefit in implanted kidney cancer

As dual combinations of mJX-594 and ICIs did not induce complete tumor regression, we explored triple combination therapy using mJX-594, αPD-1, and αCTLA-4. While the dual combination of αPD-1 and αCTLA-4 delayed tumor growth by 14.5%, and mJX-594 monotherapy inhibited tumor growth by 36.9%, the triple combination inhibited tumor growth by 76.5% (**Figures 39A** and **39B**). Notably, a few (-40%) of this triple combination group resulted in complete tumor regression, which was not observed in any other groups (**Figure 39C**).

To confirm whether the potent anti-cancer effects induced by triple combination therapy could translate into a long-term survival benefit, we performed survival analyses of tumor-bearing mice. Indeed, mice treated with triple combination therapy displayed a remarkable survival benefit compared with the other treatments (**Figure 39D**). Furthermore, mice with complete tumor regression were tumor-free for more than 12 weeks after the end of treatment and were fully protected against re-challenge with tumor cells, suggesting the establishment of an effective, long-term immune memory (**Figure 39E**).

These findings demonstrate that triple combination immunotherapy has the potential to induce complete tumor regression and long-term survival.

### Triple combination therapy enhances anti-cancer immune responses in a spontaneous breast cancer model

To definitely validate the long-term immunotherapeutic efficacy of triple combination therapy in immune-resistant tumor, we employed the *MMTV-PyMT* transgenic mouse model, which is a spontaneous breast cancer model with intrinsic resistance to cancer immunotherapy (Schmittnaegel et al., 2017). After 4 weeks of treatment, mice treated with the triple combination of mJX-594, αPD-1, and αCTLA-4 exhibited a significant reduction in overall tumor burden by 38.7% and number of palpable mammary tumor nodules compared with control mice (**Figure 40A-40D**). Furthermore, triple combination therapy led to a 48.1% reduction in the average size of each tumor nodule and improved overall survival compared with other treatments (**Figure 40E-40F**). Histological analyses (**Figure 40G**, see legend for a detailed explanation) revealed less invasive carcinoma with well-preserved tumor margins in triple combination group, indicating that triple combination effectively delays tumor progression and invasion. On the other hands, tumors treated with a dual combination of αPD-1 and αCTLA-4 showed invasive carcinoma phenotype that is comparable to control group, which had invaded into the surrounding tissues and formed solid sheets of tumor cells. Accordingly, after triple combination therapy, intratumoral recruitment of CD8⁺ T cells was notably increased by > 50-fold compared with any other treatments (**Figures 40H** and **40I**). However, tumor vascular density was similar among the treatment groups (**Figure 40J**). These findings indicate that the enhanced anti-cancer immunity, not vascular disrupting effect, is critical for the long-lasting therapeutic efficacy of triple combination immunotherapy with mJX-594 and ICIs. Finally, the number of hematogenous lung metastases was significantly reduced in triple combination group (**Figures 40K** and **40L**), indicating an effective anti-metastatic effects by the triple combination therapy.

Taken together, these results demonstrated that triple combination immunotherapy with mJX-594 and ICIs can elicit robust anti-cancer immune response even in a poorly immunogenic spontaneous breast cancer model.

### DISCUSSION

Here, we demonstrated that the combination therapy with mJX-594 and ICIs is an effective therapeutic strategy for immune-resistant tumors. The combination therapy led to an immunological "boiling point" in which a cold, non-inflamed tumor is sufficiently flamed to enable the host immune system to eradicate tumor cells. The most profound effect was obseved with triple immunotherapy with mJX-594, anti-PD-1, and anti-CTLA4, which induced complete regression in -40% of Renca tumors, which is one of the most resistant syngeneic tumors to immunotherapy. This strong synergism can be explained by the mutually complementary cooperation of OV and ICIs.

JX-594 is an OV in the most advanced stage of clinical trials, which is known to act through various mechanisms (Abou-Alfa et al., 2016). Though it can rapidly induce direct oncolysis and vascular disruption in tumor, these effects are transient and mostly diminish within 1 week of injection. Thereafter, CD8⁺ T cells extensively infiltrate the tumor to initiate anti-cancer immune responses. However, at the same time, tumors begin to evolve to avoid immune-mediated elimination by upregulating immune inhibitory checkpoint molecules such as PD-1, PD-L1, or CTLA-4 in the TME. Because the most potent and durable anti-cancer effects of OV is achieved when it is coupled with successful induction and maintenance of anti-tumor immunity, it is reasonable to combine ICIs with OV to prevent early shutdown of OV-induced anti-cancer immunity.

Although ICI monotherapy revolutionized the treatment landscape of cancer, its dramatic therapeutic response is confined to a subset of patients. This gave rise to the concept of immunologically 'hot' or 'cold' tumors; hot tumors respond well to ICIs as they are immunologically inflamed with TILs and have high expression of PD-L1, while cold tumors are refractory to ICIs because of the paucity of CD8⁺ TILs and immunosuppressive TME (Bell and Ilkow, 2017; Gajewski et al., 2013). Therefore, current efforts are focused on overcoming resistance to ICIs by converting immunologically cold tumor to hot tumors. In this aspects, our result present mJX-594 as an ideal combination partner for ICIs. It can selectively replicate in tumor cells, destroy them, and release tumor antigens to stimulate the host immune system. Moreover, our study shows that it can dramatically convert the TME from cold to hot state by inducing intratumoral inflammatory responses: induction of Th1 responses, activation and recruitment of T cells, upregulation of PD-L1, and polarization of myeloid cells toward anti-tumor activity. Intriguingly, the replication and spread of OV is known to be more active in cold tumors where there are few immune cells to eliminate OV, whereas hot tumors with ample residing TILs could induce premature clearance of OV and attenuate its therapeutic effects (Bell and Ilkow, 2017). Therefore, together with the results of this study, mJX-594 is an optimal combination partner for ICIs, especially for non-inflamed cold tumors with intrinsic resistance to immunotherapy.

GM-CSF is the most commonly used therapeutic genetic payload of OVs. Two OVs in the most advanced phases of clinical trials, T-Vec and Pexa-Vec (JX-594), are both armed with GM-CSF. Although GM-CSF is generally known to induce proliferation of various immune cells such as DCs, there is a concern regarding unwanted proliferation of immunosuppressive cells such as MDSCs (Hou et al., 2016). In the present study, we revealed that mJX-594 did not significantly alter the fraction of intratumoral CD11b+Gr1+ cells. In addition, neutralization of GM-CSF ablated the therapeutic efficacy of mJX-594, which was partly due to the reduction in CD8⁺ TILs, indicating that GM-CSF has an indispensable role in cancer immunity elicited by mJX-594.

Previous studies reported that, although the combination of OV and ICIs elicits impressive immune response, its therapeutic efficacy can be largely affected by administration route and treatment schedule. In particular, when both OV and ICIs are systemically administered simultaneously, the combination could be antagonistic due to the ICI-induced anti-viral immunity that can facilitate premature viral clearance, indicating the importance of adequate time gap in between treatments for OV to induce a successful anticancer immunity. In the present study, local injection of mJX-594 consistently induced anticancer immunity without being significantly affected by administration sequences. We presume that this is because the intratumoral injection provided OV a sufficient time lag to inflame the TME before being eliminated by systemic anti-viral immunity. Therefore, in designing clinical trials of ICI and OV combination, intratumoral OV therapy could be more feasible compared with systemic OV therapy in terms of administration schedule.

In addition to our encouraging results with combination of mJX-594 and ICIs, several clinical trials are already ongoing to investigate the efficacy of JX-594 in combination with αPD-1, αCTLA-4, or αPD-L1 to target various solid cancers, including liver cancer, renal cancer, and colon cancer (ClinicalTrials.gov: NCT03071094, NCT02977156, NCT03294083, and NCT03206073). Thus, we would be able to verify the findings of this study in a clinical setting in the near future.

In conclusion, this study indicated that intratumoral injection of mJX-954 induces a profound remodeling of TME from cold to hot state and elicit robust anti-cancer immunity in combination with ICIs, overcoming immunotherapy resistance.

### EXPERIMENTAL PROCEDURES

### Mice and cell lines

Male BALB/c mice between 6 to 8 weeks of age were purchased from Orient Bio Inc. (Seongnam, Gyeonggi, Korea), and female *MMTV-PyMT* transgenic mice (FVB/N) were purchased from Jackson Laboratory (Bar Harbor, ME, USA, #002374). Mice were housed in a specific-pathogen-free animal facility at CHA University (Seongnam, Geyonggi, Korea). All animal experiments were approved by the Institutional Animal Care and Use Committee (IACUC, #170025) of CHA University and were carried out in accordance with the approved protocols. The Renca murine renal cancer cell line and the CT26 murine colon cancer cell line were obtained from the American Type Culture Collection (Manassas, VA, USA #CRL-2947) and Korean Cell Line Bank (Seoul, Korea, #80009). These cells were maintained in Roswell Park Memorial Institute (RPMI) 1640 medium or Dulbecco's Modified Eagle Medium (DMEM), each supplemented with 10% fetal bovine serum (FBS) and 1% penicillin/streptomycin, and were incubated at 37°C, 5% CO₂ in an incubator.

### Generation and quantification of virus

mJX-594, provided by Sillajen, Inc. (Seoul, Korea), is a Western Reserve (WR) strain of vaccinia virus encoding murine GM-CSF in the vaccinia thymidine kinase gene locus under the control of the p7.5 promoter and was used throughout this study. This virus was amplified in HeLaS3 cells prior to purification. In brief, HeLaS3 cells were infected with recombinant vaccinia virus for 3 days, collected by centrifugation, then homogenized and centrifuged once more. The virus-containing supernatant was layered onto a 36% sucrose cushion and centrifuged at 32,900 g, and the purified viral pellet was resuspended in 1 mM Tris, pH 9.0. To determine the viral titer, serially diluted virus in serum-free DMEM was applied onto a monolayer of U-2 OS cells for 2 hr, and then 1.5% carboxymethylcellulose in DMEM supplemented with 2 % FBS was added. After 72 hr, cells were stained with 0.1% crystal violet and plaques were counted.

### Tumor models and treatment regimens

Tumors were implanted by subcutaneous injection of 2 × 10⁵ Renca cells into the right flank of wild type BALB/c mice. When tumors reached > 50 mm³, mice were treated with either PBS or 1 × 10⁷ plaque forming units (pfu) of mJX-594 by intratumoral injection every 3 days. For the bilateral tumor model, 2 × 10⁵ Renca cells were implanted subcutaneously into the right flank, and 1 × 10⁵ Renca or CT26 cells were implanted subcutaneously into the left flank 4 days later. For the cell depletion study, antibodies against CD4 (200 µg, clone GK1.5, BioXCell), CD8 (200 µg, clone 53-6.72, BioXCell), or GM-CSF (200 µg, clone MP1-22E9, BioXCell) were intraperitoneally injected along with mJX-594. For immune checkpoint blockade, anti-PD-1 (10 mg/kg, clone J43, BioXCell) and/or anti-CTLA-4 (4 mg/kg, clone 9D9, BioXCell) antibodies were injected intraperitoneally with or without mJX-594, every 3 days depending on the dosing schedule. Tumors were measured every 2 or 3 days using a digital caliper, and tumor volumes were calculated using the modified ellipsoid formula (1/2 × (length × width²)). On day 50, the surviving mice with complete tumor regression were re-challenged with 2 × 10⁵ Renca cells in the left flank and monitored for tumor growth and survival. Mice were euthanized when tumors reached 1.5 cm in diameter or when mice became moribund.

Female *MMTV-PyMT* transgenic mice were purchased from Jackson Laboratory. Nine weeks after birth, the volume of every palpable tumor nodule (> 20 mm³) was measured, and the total volume of all tumors combined was used to calculate the tumor burden per mouse. *MMTV-PyMT* mice were randomized according to their initial tumor burden, and were treated with 1 × 10⁷ pfu of mJX-594 in the presence or absence of the immune checkpoint inhibitors PD-1 (10 mg/kg) or CTLA-4 (4 mg/kg) at the indicated time points. After 4 weeks of treatment, mice were anesthetized and tissues were harvested for further analyses. Analyses for MMTV-PyMT was performed as previously described (Kim et al., 2014; Park et al., 2016).

### Histologic analysis

For hematoxylin and eosin (H&E) staining, tumors were fixed overnight in 4% paraformaldehyde (PFA). After tissue processing using standard procedures, samples were embedded in paraffin and cut into 3 µm sections followed by H&E staining. Immunofluorescence was performed on frozen tissue sections. Tumors were fixed in 1% PFA at room temperature and were rinsed several times with PBS, infiltrated with 30% sucrose, and frozen in OCT compound. Frozen sections (50 µm thick) were blocked with 5% normal goat serum in PBS-T (0.1% triton X-100 in PBS) and then incubated overnight with the following primary antibodies: anti-vaccinia virus (rabbit, Abcam), anti-CD31 (hamster, clone 2H8, Millipore; rabbit, Abcam), anti-CD8 (rat, clone 53-6.7, BD Pharmingen), anti-CD11c (hamster, clone HL3, BD Pharmingen), anti-PD-L1 (rabbit, clone 28-8, Abeam), anti-Caspase3 (rabbit, R&D Systems), anti-Pan-Cytokeratin (Mouse, clone AE1/AE3, DAKO), anti-CD11b (rat, clone M1/70, BD Pharmingen) or anti-CD3e (Hamster, clone 145-2C11, BD Pharmingen). After several washes, the samples were incubated for 2 hr at room temperature with the following secondary antibodies: FITC-, Cy3-, or Cy5-conjugated anti-rabbit IgG (Jackson ImmunoResearch), FITC-conjugated anti-rat IgG (Jackson ImmunoResearch), FITC- or Cy3-conjugated anti-hamster IgG (Jackson ImmunoResearch), or FITC-conjugated anti-mouse IgG (Jackson ImmunoResearch). Cell nuclei were counterstained with 4',6-diamidino-2-phenylindole (DAPI, Invitrogen). Finally, samples were mounted with fluorescent mounting medium (DAKO) and images were acquired with a Zeiss LSM 880 microscope (Carl Zeiss).

### Morphometric analysis

Density measurements of vaccinia virus, blood vessels, T lymphocytes, and dendritic cells, as well as measurement of myeloid cell area, was performed using ImageJ software (http://rsb.info.nih.gov/ij). To determine the level of vaccinia virus infection, VV⁺ area per random 0.49 mm² field was calculated in tumor sections. For blood vessel density, CD31⁺ area per random 0.49 mm² field was calculated in peri- and intratumoral regions. The degree of cytotoxic T lymphocyte infiltration was presented as the percentage CD8⁺ area per random 0.49 mm² field in peri- and intratumoral regions. Level of dendritic cells were measured by calculating the percentage CD11c⁺ area in random 0.49 mm² fields. The extent of apoptosis was exhibited as the percentage Caspase3⁺ area per random 0.49 mm² fields. To define PD-L1⁺ cells, co-localization of PD-L1⁺ with Pan-CK+, CD11b⁺, and CD3⁺ was identified in random 0.01 mm² field. Lung metastasis in MMTV-PyMT mice was quantified by measurement of tumor colonies >100 µm in diameter. All measurements were performed in at least 5 fields per mouse.

### Flow cytometric analysis of tumor-infiltrating immune cells

Tumors from each treatment group were minced prior to incubation with shaking for 1 hr at 37°C, in the presence of collagenase D (20 mg/ml, Roche) and DNase I (2 mg/ml, Roche). Cell suspensions were generated by repeated pipetting, and then filtered through a 70 µm cell strainer and lysed to remove red blood cells. After washing with PBS, resuspended cells were filtered through a nylon mesh. Single cell suspensions from tumor tissues were blocked with an antibody against CD16/32 (clone 2.4G2, BD Pharmingen) and stained with a fixable viability dye (eFlouor450, eBioscience) to distinguish the live cells. For analysis of surface markers, cells were stained in PBS containing 1% FBS, with antibodies targeting CD45 (30-F11, BD Pharmingen), CD4 (RM4-5, BD Pharmingen), CD8 (53-6.7, BD Pharmingen), CD3 (17A2 or 145-2C11, eBioscience), ICOS (7E.17G9 or 15F9, eBioscience), CD11b (MI170, BD Pharmingen), F4/80 (BM8, eBioscience), MHC II (M5/114.15.2, eBioscience), Ly6C (HK1.4, eBioscience), Ly6G (1A8-Ly6g or RB6-8C5, eBioscience) or CD206 (MR5D3, eBioscience), for 30 min on ice. Cells were further permeabilized using a FoxP3 fixation and permeabilization kit (eBioscience), and stained for FoxP3 (FJK-16s, eBioscience), CD25 (PC61.5, eBioscience), or Granzyme B (NGZB, eBioscience). Labeled cells were acquired using a CytoFLEX flow cytometer (Beckman Coulter) and analyzed using FlowJo software (Tree Star Inc., Ashland, OR).

### RNA isolation and NanoString gene expression analysis

Total RNA was extracted from whole tumor lysates using TRIzol (Invitrogen) and purified with ethanol; RNA quality was confirmed using a Fragment Analyzer instrument (Advanced Analytical Technologies, IA, USA). Immune profiling was performed with a digital multiplexed NanoString nCounter PanCancer Immune Profiling mouse panel (NanoString Technologies), using 100 ng total RNA isolated from tumor tissues. Hybridizations were carried out at 65 °C for 16-30 hr by combining 5 µl of each RNA sample with 8 µl of nCounter Reporter probe in hybridization buffer and 2 µl of nCounter Capture probes, for a total reaction volume of 15 µl. Excess probe was removed by two-step magnetic bead-based purification using the nCounter Prep Station (NanoString Technologies). Specific target molecule abundance was quantified with the nCounter Digital Analyzer by counting individual fluorescent barcodes and assessing the corresponding target molecules. For each assay, a high-density scan encompassing 280 fields of view was performed. Data were collected using the nCounter Digital Analyzer after acquiring images of the immobilized fluorescent reporters in the sample cartridge with a CCD camera. Data analysis was performed using nSolver software (NanoString Technologies). The mRNA profiling data was normalized to housekeeping genes and analyzed using R software (www.r-project.org).

### Statistical analysis

Statistical analyses were performed using GraphPad Prism 7.0 software (GraphPad Software, La Jolla, CA) and PASW statistics 18 (SPSS). Values are represented as mean +/-standard error of the mean (SEM) unless otherwise indicated. Statistical differences between means were tested using unpaired Student's t-tests. Survival curves were generated using the Kaplan-Meier method, and statistical differences between curves were analyzed using the log-rank test. The level of statistical significance was set at p < 0.05.

### REFERENCES

Abou-Alfa, G. K., Galle, P. R., Chao, Y, Brown, K. T., Heo, J., Borad, M. J., Luca, A., Pelusio, A., Agathon, D., and Lusky, M. (2016). PHOCUS: A phase 3 randomized, open-label study comparing the oncolytic immunotherapy Pexa-Vec followed by sorafenib (SOR) vs SOR in patients with advanced hepatocellular carcinoma (HCC) without prior systemic therapy. In, (2016 ASCO Annual Meeting).
Bell, J. (2014). Oncolytic viruses: immune or cytolytic therapy? Molecular Therapy 22, 1231-1232.
Bell, J. C., and Ilkow, C. S. (2017). A viro-immunotherapy triple play for the treatment of glioblastoma. Cancer cell 32, 133-134.
Breitbach, C. J., Burke, J., Jonker, D., Stephenson, J., Haas, A. R., Chow, L. Q., Nieva, J., Hwang, T. H., Moon, A., Patt, R., et al. (2011a). Intravenous delivery of a multi-mechanistic cancer-targeted oncolytic poxvirus in humans. Nature 477, 99-102.
Breitbach, C. J., De Silva, N. S., Falls, T. J., Aladl, U., Evgin, L., Paterson, J., Sun, Y Y, Roy, D. G., Rintoul, J. L., Daneshmand, M., et al. (2011b). Targeting tumor vasculature with an oncolytic virus. Molecular therapy : the journal of the American Society of Gene Therapy 19, 886-894.
Breitbach, C. J., Parato, K., Burke, J., Hwang, T.-H., Bell, J. C., and Kirn, D. H. (2015a). Pexa-Vec double agent engineered vaccinia: oncolytic and active immunotherapeutic. Current opinion in virology 13, 49-54.
Breitbach, C. J., Parato, K., Burke, J., Hwang, T. H., Bell, J. C., and Kirn, D. H. (2015b). Pexa-Vec double agent engineered vaccinia: oncolytic and active immunotherapeutic. Current opinion in virology 13, 49-54.
Chiocca, E. A., and Rabkin, S. D. (2014). Oncolytic viruses and their application to cancer immunotherapy. Cancer immunology research 2, 295-300.
Cripe, T. P., Ngo, M. C., Geller, J. I., Louis, C. U., Currier, M. A., Racadio, J. M., Towbin, A. J., Rooney, C. M., Pelusio, A., Moon, A., et al. (2015). Phase 1 study of intratumoral Pexa-Vec (mJX-594), an oncolytic and immunotherapeutic vaccinia virus, in pediatric cancer patients. Molecular therapy : the journal of the American Society of Gene Therapy 23, 602-608.
De Palma, M., and Jain, R. K. (2017). CD4+ T cell activation and vascular normalization: Two sides of the same coin? Immunity 46, 773-775.
Gajewski, T. F. (2015). The Next Hurdle in Cancer Immunotherapy: Overcoming the Non-T-Cell-Inflamed Tumor Microenvironment. Seminars in oncology 42, 663-671.
Gajewski, T. F., Schreiber, H., and Fu, Y X. (2013). Innate and adaptive immune cells in the tumor microenvironment. Nature immunology 14, 1014-1022.
Hegde, P. S., Karanikas, V, and Evers, S. (2016). The Where, the When, and the How of Immune Monitoring for Cancer Immunotherapies in the Era of Checkpoint Inhibition. Clinical cancer research : an official journal of the American Association for Cancer Research 22, 1865-1874.
Heo, J., Reid, T., Ruo, L., Breitbach, C. J., Rose, S., Bloomston, M., Cho, M., Lim, H. Y, Chung, H. C., Kim, C. W., et al. (2013). Randomized dose-finding clinical trial of oncolytic immunotherapeutic vaccinia mJX-594 in liver cancer. Nature medicine 19, 329-336.
Hou, W., Sampath, P., Rojas, J. J., and Thorne, S. H. (2016). Oncolytic Virus-Mediated Targeting of PGE2 in the Tumor Alters the Immune Status and Sensitizes Established and Resistant Tumors to Immunotherapy. Cancer cell 30, 108-119.
Kim, C., Yang, H., Fukushima, Y, Saw, P. E., Lee, J., Park, J.-S., Park, I., Jung, J., Kataoka, H., and Lee, D. (2014). Vascular RhoJ is an effective and selective target for tumor angiogenesis and vascular disruption. Cancer cell 25, 102-117.
Kim, M., Nitschk6, M., Sennino, B., Murer, P., Schriver, B. J., Bell, A., Subramanian, A., McDonald, C. E., Wang, J., and Cha, H. (2018). Amplification of oncolytic vaccinia virus widespread tumor cell killing by sunitinib through multiple mechanisms. Cancer research 78, 922-937.
Kirn, D. H., and Thorne, S. H. (2009). Targeted and armed oncolytic poxviruses: a novel multi-mechanistic therapeutic class for cancer. Nature Reviews Cancer 9, 64.
Lichty, B. D., Breitbach, C. J., Stojdl, D. F., and Bell, J. C. (2014). Going viral with cancer immunotherapy. Nature Reviews Cancer 14, 559.
Liu, Z., Ravindranathan, R., Kalinski, P., Guo, Z. S., and Bartlett, D. L. (2017). Rational combination of oncolytic vaccinia virus and PD-L1 blockade works synergistically to enhance therapeutic efficacy. Nature communications 8, 14754.
Park, B. H., Hwang, T., Liu, T. C., Sze, D. Y, Kim, J. S., Kwon, H. C., Oh, S. Y, Han, S. Y, Yoon, J. H., Hong, S. H., et al. (2008). Use of a targeted oncolytic poxvirus, mJX-594, in patients with refractory primary or metastatic liver cancer: a phase I trial. The Lancet Oncology 9, 533-542.
Park, J.-S., Kim, I.-K., Han, S., Park, I., Kim, C., Bae, J., Oh, S. J., Lee, S., Kim, J. H., and Woo, D.-C. (2016). Normalization of tumor vessels by Tie2 activation and Ang2 inhibition enhances drug delivery and produces a favorable tumor microenvironment. Cancer cell 30, 953-967.
Rivera, L. B., and Bergers, G. (2015). Intertwined regulation of angiogenesis and immunity by myeloid cells. Trends in immunology 36, 240-249.
Rojas, J. J., Sampath, P., Hou, W., and Thorne, S. H. (2015). Defining Effective Combinations of Immune Checkpoint Blockade and Oncolytic Virotherapy. Clinical cancer research : an official journal of the American Association for Cancer Research 21, 5543-5551.
Schmittnaegel, M., Rigamonti, N., Kadioglu, E., Cassará, A., Rmili, C. W., Kiialainen, A., Kienast, Y, Mueller, H.-J., Ooi, C.-H., and Laoui, D. (2017). Dual angiopoietin-2 and VEGFA inhibition elicits antitumor immunity that is enhanced by PD-1 checkpoint blockade. Science translational medicine 9, eaak9670.
Sharma, P., Hu-Lieskovan, S., Wargo, J. A., and Ribas, A. (2017). Primary, adaptive, and acquired resistance to cancer immunotherapy. Cell 168, 707-723.
Thorne, S. H. (2014). Immunotherapeutic potential of oncolytic vaccinia virus. Frontiers in oncology 4, 155.
Topalian, S. L., Drake, C. G., and Pardoll, D. M. (2015). Immune checkpoint blockade: a common denominator approach to cancer therapy. Cancer cell 27, 450-461.
Topalian, S. L., Taube, J. M., Anders, R. A., and Pardoll, D. M. (2016). Mechanism-driven biomarkers to guide immune checkpoint blockade in cancer therapy. Nature Reviews Cancer 16, 275.
Wolchok, J. D., and Chan, T. A. (2014). Cancer: Antitumour immunity gets a boost. Nature 515, 496*.*

All of the compositions and/or methods disclosed and claimed herein can be made and executed without undue experimentation in light of the present disclosure. While the compositions and methods of this invention have been described in terms of preferred embodiments, it will be apparent to those of skill in the art that variations may be applied to the compositions and/or methods and in the steps or in the sequence of steps of the method described herein without departing from the concept, spirit and scope of the invention. More specifically, it will be apparent that certain agents which are both chemically and physiologically related may be substituted for the agents described herein while the same or similar results would be achieved. All such similar substitutes and modifications apparent to those skilled in the art are deemed to be within the spirit, scope and concept of the invention as defined by the appended claims.

The examples set forth above are provided to give those of ordinary skill in the art a complete disclosure and description of how to make and use the embodiments of the compositions, systems and methods of the invention, and are not intended to limit the scope of what the inventors regard as their invention. Modifications of the above-described modes for carrying out the invention that are obvious to persons of skill in the art are intended to be within the scope of the following claims. All patents and publications mentioned in the specification are indicative of the levels of skill of those skilled in the art to which the invention pertains. All references cited in this disclosure are incorporated by reference to the same extent as if each reference had been incorporated by reference in its entirety individually.

All headings and section designations are used for clarity and reference purposes only and are not to be considered limiting in any way. For example, those of skilled in the art will appreciate the usefulness of combining various aspects from different headings and sections as appropriate according to the spirit and scope of the invention described herein.

All references cited herein are hereby incorporated by reference herein in their entireties and for all purposes to the same extent as if each individual publication or patent or patent application was specifically and individually indicated to be incorporated by reference in its entirety for all purposes.

Many modifications and variations of this application can be made without departing from its spirit and scope, as will be apparent to those skilled in the art. The specific embodiments and examples described herein are offered by way of example only, and the application is to be limited only by the terms of the appended claims, along with the full scope of equivalents to which the claims are entitled.

### CLAUSES:

1. A method for treating and/or preventing cancer in a subject in need of such treatment comprising concurrently administering to the subject an effective amount of a combination comprising (a) a replicative oncolytic vaccinia virus and (b) one or more immune checkpoint inhibitors.
2. The method of clause 1, wherein the replicative oncolytic vaccinia virus is administered intratumorally, intravenously, intra-arterially, or intraperitoneally.
3. The method of clause 2, wherein the replicative oncolytic vaccinia virus is administered intratumorally.
4. The method of any one of clauses 1-3, wherein the replicative oncolytic vaccinia virus is administered in an amount effective to induce expression of an immune checkpoint protein in the tumor.
5. The method of any one of clauses 1-4, wherein the tumor does not express the immune checkpoint protein or expresses the immune checkpoint protein at a relatively low level prior to administering the replicative oncolytic vaccinia virus.
6. The method of any one of clause 1-5, wherein the immune checkpoint inhibitor is an antibody or fragment thereof that specifically binds to the immune checkpoint protein, preferably a monoclonal antibody, humanized antibody, fully human antibody, fusion protein or combination thereof.
7. The method of any one of clauses 1-6, wherein the checkpoint inhibitor inhibits a checkpoint protein selected from the group consisting of cytotoxic T-lymphocyte antigen-4 (CTLA4), programmed cell death protein 1 (PD-1), PD-L1, PD-L2, B7-H3, B7-H4, herpesvirus entry mediator (HVEM), T-cell membrane protein 3 (TIM3), galectin 9 (GAL9), lymphocyte activation gene 3 (LAG3), V-domain immunoglobulin (Ig)-containing suppressor of T-cell activation (VISTA), Killer-Cell Immunoglobulin-Like Receptor (KIR), B and T lymphocyte attenuator (BTLA), T-cell immunoreceptor with Ig and ITIM domains (TIGIT), indoleamine 2,3-dioxygenase (IDO), and combinations thereof.
8. The method of clause 7, wherein the immune checkpoint inhibitor is a monoclonal antibody that selectively binds to PD-1 or PD-L1, preferably selected from the group consisting of: BMS-936559, atezolizumab, durvalumab, avelumab, nivolumab, pembrolizumab, and lambrolizumab.
9. The method of clause 7, wherein the immune checkpoint inhibitor is a monoclonal antibody that selectively binds to CTLA4, preferably selected from the group consisting of ipilimumab and tremelimumab.
10. The method of any one of clauses 1-9, wherein multiple checkpoint inhibitors are concurrently administered to the subject with the oncolytic vaccinia virus.
11. The method of clause 10, wherein the subject is concurrently administered:
   (a) a CTLA4 inhibitor, a PD-1 inhibitor and a replicative oncolytic vaccinia virus;
   (b) a CTLA4 inhibitor, an IDO inhibitor and a replicative oncolytic vaccinia virus;
   (c) a PD-1 inhibitor, an IDO inhibitor and a replicative oncolytic vaccinia virus; or
   (d) a PD-1 inhibitor, a CTLA4 inhibitor, an IDO inhibitor and a replicative oncolytic vaccinia virus;
   (e) a LAG3 inhibitor, a PD-1 inhibitor and a replicative oncolytic vaccinia virus; or
   (f) a TIGIT inhibitor, a PD-1 inhibitor and a replicative oncolytic vaccinia virus.
12. The method of any one of clauses 1-11, wherein the replicative oncolytic vaccinia virus is a Wyeth Strain, Western Reserve Strain, Lister strain or Copenhagen strain.
13. The method of any one of clauses 1-12, wherein the vaccinia virus comprises one or more genetic modifications to increase selectivity of the virus for cancer cells, preferably the virus is engineered to lack functional thymidine kinase and/or to lack functional vaccinia growth factor.
14. The method of any one of clause 1-13, wherein the vaccinia virus comprises a functional 14L and/or F4L gene.
15. The method of any one of clauses 1-14, wherein the vaccinia virus is engineered to express (i) a cytokine preferably selected from GM-CSF, IL-2, IL-4, IL-5 IL-7, IL-12, IL-15, IL-18, IL-21, IL-24, IFN-γ, TNF-α, and/or (ii) a tumor antigen preferably selected from BAGE, GAGE-1, GAGE-2, CEA, AIM2, CDK4, BMI1, COX-2 , MUM-1, MUC-1, TRP-1 TRP-2, GP100, EGFRvIII, EZH2, LICAM, Livin, Livinβ, MRP-3, Nestin, OLIG2 , SOX2, human papillomavirus-E6, human papillomavirus-E7, ART1, ART4, SART1, SART2, SART3, B-cyclin, β-catenin, Gli1, Cav-1, cathepsin B, CD74, E-cadherin, EphA2/Eck, Fra-1/Fosl 1, Ganglioside/GD2, GnT-V, β1,6-N, Her2/neu, Ki67, Ku70/80, IL-13Ra2, MAGE-1, MAGE-3, NY-ESO-1, MART-1, PROX1, PSCA, SOX10, SOX11, Survivin, caspase-8, UPAR, CA-125, PSA, p185HER2, CD5, IL-2R, Fap-α, tenascin, melanoma-associated antigen p97, and WT-1, regulator of G-protein signaling 5 (RGS5), Surivin (BIRC5=baculoviral inhibitor of apoptosis repeat-containg 5), Insulin-like growth factorbinding protein 3 (IGF-BP3), thymidylate synthetase (TYMS), hypoxia-inducible protein 2, hypoxial inducible lipid droplet associated (HIG2), matrix metallopeptidase 7 (MMP7), prune homolog 2 (PRUNE2), RecQ protein-like (DNA helicase Q1-like) (RECQL), leptin receptor (LEPR), ERBB receptor feedback inhibitor 1 (ERRFI1), lysosomal protein transmembrane 4 alpha (LAPTM4A); RAB1B, RAS oncogene family (RAB1B), CD24, homo sapiens thymosin beta 4, X-linked (TMSB4X), homo sapiens S100 calcium binding protein A6 (S100A6), homo sapiens adenosine A2 receptor (ADORA2B), chromosome 16 open reading frame 61 (C16orf61), ROD1 regulator of differentiation 1 (ROD1), NAD-dependent deacetylase sirtuin-2 (SIR2L), tubulin alpha 1c (TUBA1C), ATPase inhibitory factor 1 (ATPIF1), stromal antigen 2 (STAG2), nuclear casein kinase, and cyclin-dependent substrate 1 (NUCKS1).
16. The method of any one of clauses 1-15, wherein the vaccinia virus is administered in an amount from about 10⁷ to about 10¹¹ pfu, preferably about 10⁸-10¹⁰ pfu, more preferably about 10⁹-10¹⁰ pfu.
17. The method of any one of clauses 1-16, wherein the checkpoint inhibitor is administered in an amount from about 2 mg/kg to 15 mg/kg.
18. The method of any one of clauses 1-17, wherein the subject has a cancer selected from hepatocellular carcinoma, colorectal cancer, renal cell carcinoma, bladder cancer, lung cancer (including non-small cell lung cancer), stomach cancer, esophageal cancer, sarcoma, mesothelioma, melanoma, pancreatic cancer, head and neck cancer, ovarian cancer, cervical and liver cancer.
19. The method of clause 18, wherein the subject has renal cell carcinoma.
20. The method of any one of clauses 1-19, wherein the subject has failed at least one previous chemotherapy or immunotherapy treatment.
21. The method of clause 20, wherein the subject has a cancer that is refractory to an immune checkpoint inhibitor therapy, preferably the cancer is resistant to treatment with anti-PD-1 antibodies and/or anti-CTLA-4 antibodies.
22. The method of clause 21, wherein the subject is identified as a candidate for an immune checkpoint inhibitor therapy.
23. The method of any one of clauses 1-22, comprising administering to the subject an additional therapy selected from chemotherapy (alkylating agents, nucleoside analogs, cytoskeleton modifiers, cytostatic agents) and radiotherapy.
24. The method of any one of clauses 1-23, comprising administering to the subject an additional oncolytic virus therapy (e.g. rhabdovirus, Semliki Forest Virus).
25. The method of any one of clauses 1-24, wherein the subject is a human.
26. The method of any one of clauses 1-25, wherein a first dose of the replicative oncolytic vaccinia virus and a first dose of the immune checkpoint inhibitor are simultaneously administered to the subject followed by at least one subsequent consecutive simultaneous administration of the virus and checkpoint inhibitor to the subject.
27. The method of clause 26, comprising at least a first, second and third consecutive simultaneous administration of the replicative oncolytic vaccinia virus and checkpoint inhibitor to the subject.
28. The method of clause 27, comprising at least a first, second, third and fourth consecutive simultaneous administration of the replicative oncolytic vaccinia virus and checkpoint inhibitor to the subject.
29. The method of any one of clauses 26-28, wherein simultaneous administration of the first dose of the replicative oncolytic vaccinia virus and the first dose of the immune checkpoint inhibitor and at least one subsequent consecutive simultaneous administration of the virus and checkpoint inhibitor to the subject is followed by administration of at least one dose of checkpoint inhibitor alone to the subject.
30. The method of any one of clauses 26-29, comprising an interval of 1-3 weeks between consecutive simultaneous administration of the agents, preferably comprising an interval of about one week, about two weeks or about 3 weeks.
31. A method of treating a tumor in a human comprising concurrently administering to the human a combination comprising (a) a replicative oncolytic vaccinia virus and (b) an inhibitor of an immune checkpoint protein.
32. The method of clause 31, wherein the replicative oncolytic vaccinia virus is administered intratumorally, intravenously, intra-arterially, or intraperitoneally.
33. The method of clause 31, wherein the replicative oncolytic vaccinia virus is administered intratumorally.
34. The method of clause 31, wherein the replicative oncolytic vaccinia virus is administered in an amount effective to induce expression of an immune checkpoint protein in the tumor.
35. The method of clause 31, wherein the tumor does not express the immune checkpoint protein or expresses the immune checkpoint protein at a relatively low level prior to administering the replicative oncolytic vaccinia virus.
36. The method of clause 34 or 35, comprising a step of measuring the expression level of the immune checkpoint protein in the tumor prior to administering the combination.
37. The method of any one of clauses 31-36, wherein the immune checkpoint protein is selected from PD-1, PD-L1, CTLA-4, LAG3, TIM3, and TIGIT.
38. The method of clause 31, wherein the immune checkpoint protein is CTLA-4.
39. The method of clause 31, wherein the immune checkpoint protein is PD-L1
40. The method of clause 31, wherein the immune checkpoint protein is LAG3.
41. The method of clause 31, wherein the immune checkpoint protein is TIGIT.
42. The method of clause 31, wherein the immune checkpoint protein is PD-1.
43. The method of clause 31, wherein the immune checkpoint protein is TIM3.
44. The method of any one of clauses 31-43, wherein the tumor is a solid cancer.
45. The method of any one of clauses 31-43, wherein the tumor is a colorectal cancer.
46. The method of any one of clauses 31-43, wherein the tumor is a renal cell carcinoma.
47. The method of clause 39 or 42, wherein the inhibitor of the immune checkpoint protein is a monoclonal antibody that selectively binds to PD-1 or PD-L1, preferably selected from the group consisting of BMS-936559, atezolizumab, durvalumab, avelumab, nivolumab, pembrolizumab, and lambrolizumab.
48. The method of clause 38, wherein the inhibitor of the immune checkpoint protein is a monoclonal antibody that selectively binds to CTLA-4, preferably selected from the group consisting of ipilimumab and tremelimumab.
49. A method of treating a tumor in a human comprising concurrently administering to the human a combination comprising (a) a replicative oncolytic vaccinia virus, (b) an inhibitor of PD-1 and/or PD-L1, and (c) an inhibitor of an immune checkpoint protein.
50. The method of clause 49, wherein the tumor does not express the immune checkpoint protein or expresses the immune checkpoint protein at a relatively low level prior to administering the replicative oncolytic vaccinia virus.
51. The method of clause 49, wherein the replicative oncolytic vaccinia virus is administered intratumorally, intravenously, intra-arterially, or intraperitoneally.
52. The method of clause 49, wherein the replicative oncolytic vaccinia virus is administered intratumorally.
53. The method of clause 49, wherein the replicative oncolytic vaccinia virus is administered in an amount effective to induce expression of an immune checkpoint protein in the tumor
54. The method of clause 49, wherein the tumor does not express the immune checkpoint protein or expresses the immune checkpoint protein at a relatively low level prior to administering the replicative oncolytic vaccinia virus.
55. The method of clause 53 or 54, comprising a step of measuring the expression level of the checkpoint protein in the tumor prior to administering the combination.
56. The method of any one of clauses 49-55. wherein the immune checkpoint protein is selected from CTLA-4, LAG3, TIM3, and TTGTT.
57. The method of clause 56, wherein the immune checkpoint protein is CTLA-4.
58. The method of clause 56, wherein the immune checkpoint protein is LAG3.
59. The method of clause 56, wherein the immune checkpoint protein is TIGIT.
60. The method of clause 56, wherein the immune checkpoint protein is TIM3.
61. The method of any one of clauses 49-60, wherein the tumor is a solid cancer.
62. The method of any one of clauses 49-61, wherein the tumor is a colorectal cancer.
63. The method of any one of clauses 49-62, wherein the tumor is a renal cell carcinoma.
64. The method of clause 49, wherein the inhibitor of the immune checkpoint protein is a monoclonal antibody that selectively binds to PD-1 or PD-L1 , preferably selected from the group consisting of BMS-936559, atezolizumab, durvalumab, avclumab, nivolumab, pembrolizumab, and lambrolizumab.
65. The method of clause 57, wherein the inhibitor of the immune checkpoint protein is a monoclonal antibody that selectively binds to CTLA-4, preferably selected from the group consisting of ipilimumab and tremelimuniab.

## Claims

1. A combination therapy comprising (a) a replicative oncolytic vaccinia virus engineered to express GM-CSF and (b) an antibody that selectively binds to PD-1 for use in the treatment of renal cell carcinoma in a human subject.

2. The combination for use according to claim 1, wherein the oncolytic vaccinia virus comprises a deactivated thymidine kinase gene.

3. The combination for use according to claim 1 or 2, wherein the replicative oncolytic vaccinia virus is administered intratumorally intravenously, intra-arterially, or intraperitoneally.

4. The combination for use according to any one of claims 1-3, wherein the replicative oncolytic vaccinia virus is a Wyeth Strain.

5. The combination for use according to any one of claims 1-4, wherein the vaccinia virus comprises functional 14L and/or F4L genes.

6. The combination for use according to any one of claims 1-5, wherein the antibody that selectively binds PD-1 is a monoclonal antibody.

7. The combination for use according to any one of claims 1-4, wherein the renal cell carcinoma is metastatic renal cell carcinoma.

8. The combination for use according to any one of claims 1-7, wherein the subject has failed at least one previous chemotherapy or immunotherapy, preferably wherein the subject has a cancer that is refractory to an immune checkpoint inhibitor therapy, preferably wherein the cancer is resistant to treatment with anti-PD-1 antibodies and/or anti-PD-L1 antibodies.

9. The combination for use according to any one of claims 1-8, wherein the replicative oncolytic vaccinia virus is administered multiple times at a dose of 1×10⁸ to 1×10⁹ plaque forming units (pfu), preferably at a dose of 1×10⁹ pfu.

10. The combination for use according to any one of claims 1-9, wherein the replicative oncolytic vaccinia virus is administered weekly.

11. The combination for use according to any one of claims 1-10, wherein the antibody that selectively binds PD-1 is administered multiple times at a dose of 2 mg/kg to 15 mg/kg.

12. The combination for use according to any one of claims 1-11 wherein the antibody that selectively binds PD-1 is administered at least once every month.

13. The combination for use according to any one of claims 1-12, wherein the replicative oncolytic vaccinia virus and the antibody that selectively binds PD-1 are administered concurrently, wherein concurrent administration comprises administering said oncolytic vaccinia virus and said anti-PD-1 antibody within 12, 11, 10, 9, 8, 7, 6, 5, 4, 3, 2 hours or within 1 hour of each other.

14. The combination for use according to any one of claims 1-13, wherein the combination therapy provides improved antitumoral effects relative to single administration of either the replicative oncolytic vaccinia virus or the antibody that selectively binds PD-1.
